(19) Europäisches Patentamt / European Patent Office / Office européen des brevets

(11) EP 2 402 440 A1

(12) **EUROPEAN PATENT APPLICATION**
published in accordance with Art. 153(4) EPC

(43) Date of publication:
**04.01.2012 Bulletin 2012/01**

(21) Application number: **10746257.4**

(22) Date of filing: **25.02.2010**

(51) Int Cl.:
***C12N 15/09*** *(2006.01)* ***A01H 1/00*** *(2006.01)*
***A23K 1/14*** *(2006.01)* ***A23L 1/30*** *(2006.01)*
***C12C 11/00*** *(2006.01)* ***C12G 3/12*** *(2006.01)*
***C12N 1/15*** *(2006.01)* ***C12N 1/19*** *(2006.01)*
***C12N 1/21*** *(2006.01)* ***C12N 5/10*** *(2006.01)*

(86) International application number:
**PCT/JP2010/052939**

(87) International publication number:
**WO 2010/098378 (02.09.2010 Gazette 2010/35)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.02.2009 JP 2009045393**

(71) Applicants:
- **Incorporated Administrative Agency National Agriculture and Food Research Organization Ibaraki 305-8517 (JP)**
- **National University Corporation Okayama University Kita-ku Okayama-shi Okayama 700-8530 (JP)**

(72) Inventors:
- **TONOOKA Takuji Tsukuba-shi Ibaraki 305-8518 (JP)**
- **YOSHIOKA Toji Tsukuba-shi Ibaraki 305-8518 (JP)**
- **AOKI Emiko Tsukuba-shi Ibaraki 305-8518 (JP)**
- **TAKETA Shin Kurashiki-shi Okayama 710-0046 (JP)**

(74) Representative: **Grünecker, Kinkeldey, Stockmair & Schwanhäusser Leopoldstrasse 4 80802 München (DE)**

(54) **-GLUCAN-DEFICIENT GENE IN BARLEY, SYNTHETIC GENE, AND USE OF SAME**

(57) A Genomic DNA and a cDNA of a β-glucan-deficient gene in a barley and of a β-glucan synthesis gene in a barley, a barley having the genomic DNA of a β-glucan-deficient gene and a method for breeding the same, a method for producing an alcohol or a fermented food using barley kernels decreasing or deficient in β-glucan, and an animal feed composition are provided. A Genomic DNA of a β-glucan-deficient gene consisting of a base sequence having 90% or more homology to the base sequence of SEQ ID NO: 1 and producing a protein lacking a β-glucan synthesis activity when transcribed and translated, and a breeding method comprising a step of selecting a barley by determining the barley as having a β-glucan-deficient gene when the base corresponding to position 4,275, or corresponding to position 2,385, of the gene consisting of the base sequence of SEQ ID NO: 4 located near the centromere of chromosome 7H of the barley is mutated from G to A.

*Fig.2*

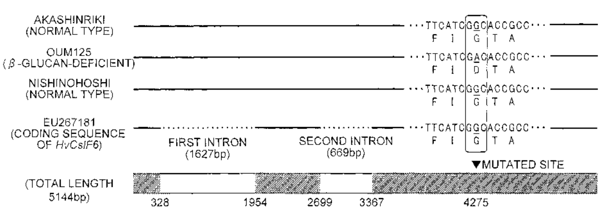




Printed by Jouve, 75001 PARIS (FR)

## Description

### Technical Field

The present invention relates to a gene deficient in (1-3, 1-4)-β-D-glucan (hereinafter referred to as "β-glucan") (hereinafter referred to as a "β-glucan-deficient gene") and a β-glucan synthesis gene in barley kernels and the use thereof. Specifically, the invention relates to: genomic DNA and cDNA of a β-glucan-deficient gene in a barley; genomic DNA and cDNA of a β-glucan synthesis gene in a barley; a barley having the genomic DNA of a β-glucan-deficient gene and a method for breeding the same; a method for decreasing or abolishing β-glucan in barley kernels by suppressing the expression of the β-glucan synthesis gene and a barley having a decrease or deficiency in β-glucan in kernels thereof, obtainable by the method; a method for producing an alcohol or a fermented food, comprising a step of fermenting kernels of the barley decreasing or deficient in β-glucan; and an animal feed composition containing the barley kernels. Here, the β-glucan synthesis gene means a gene encoding a protein having the function of participating in the pathway for the synthesis of β-glucan and promoting the synthesis of β-glucan.

### Background Art

Unlike cereals such as wheat and rice, barley kernels are rich in β-glucan as one of polysaccharides. β-Glucan is the major component constituting the endosperm cell wall thereof. It has been shown that a high content of β-glucan decreases the saccharification of starch and the efficiency of fermentation in the brewing of beer and shochu (a Japanese distilled spirit) and has negative effects such as poor digestion, poor absorption and a low feeding effect. Thus, in the barley used for these applications, there is a need for the lower content of β-glucan; a gene deficient in β-glucan is useful.
β-Glucan has also been shown to have physiological effects such as the suppression of the increased blood glucose level and the reduction of blood cholesterol and be effective in preventing and improving lifestyle-related diseases. It has been reported to be also effective in improving the immune function in recent years (Non Patent Literature 1). Thus, when barley is used as food, that having a higher content of β-glucan has a higher use value.

### Citation List

### Non Patent Literature

Non Patent Literature 1
Brennan, C.S. and L.J. Cleary (2005) J. Cereal Sci. 42: 1-13.

### Summary of Invention

### Technical Problem

In order to genetically control the β-glucan content for breeding, it is essential to elucidate the gene involved in the synthesis and control of β-glucan. The gene involved in β-glucan synthesis remains to be elucidated despite that β-glucan is an important component constituting the cell wall of a plant while being a highly functional polysaccharide attracting world-wide attention. The elucidation of the gene becomes severely competitive on a worldwide scale.
An object of the present invention is to provide genomic DNA and cDNA of a β-glucan-deficient gene in a barley. Another object of the present invention is to provide genomic DNA and cDNA of a β-glucan synthesis gene in a barley. A further object of the present invention is to provide a barley having the genomic DNA of a β-glucan-deficient gene and a method for breeding the same, a method for producing an alcohol, comprising a step of fermenting kernels of the barley, and a an animal feed composition containing the barley kernels.

### Solution to Problem

In one aspect, the present invention provides a genomic DNA of a β-glucan-deficient gene which is a DNA consisting of a base sequence having 90% or more homology to the base sequence of SEQ ID NO: 1 and producing a protein lacking a β-glucan synthesis activity when transcribed and translated.
In another aspect, the present invention provides a genomic DNA of a β-glucan-deficient gene which is a DNA consisting of a base sequence having 90% or more homology to the base sequence of SEQ ID NO: 18 and producing a protein lacking a β-glucan synthesis activity when transcribed and translated.
The genomic DNA of a β-glucan-deficient gene can be used for producing a barley in whose kernels the aleurone layer and the endosperm cell wall are completely deficient in β-glucan and the endosperm cell wall is noticeably thin.

The use of barley kernels deficient in β-glucan as a raw material can enhance the efficiency of saccharification of starch and fermentation in the production of beer and shochu. An animal feed composition for pigs, fowls, or the like, comprising barley kernels deficient in β-glucan is also excellent in digestion absorption and has a high feeding effect. In addition, the barley deficient in β-glucan contains slightly more arabinoxylan important as a functional polysaccharide; thus, it can be used for the development of a functional food specialized in arabinoxylan. Although it is generally difficult to separate β-glucan and arabinoxylan, pure arabinoxylan can be obtained by extracting arabinoxylan from the barley deficient in β-glucan.

The protein lacking a β-glucan synthesis activity produced when the genomic DNA of a β-glucan-deficient gene of the present invention is transcribed and translated is more preferably a protein lacking a β-glucan synthesis activity consisting of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is an amino acid other than glycine. The amino acid corresponding to position 660 is more preferably aspartic acid. In addition, the genomic DNA of a β-glucan-deficient gene of the present invention particularly preferably consists of the base sequence of SEQ ID NO: 1.

Alternatively, the protein lacking a β-glucan synthesis activity produced when the genomic DNA of a β-glucan-deficient gene of the present invention is transcribed and translated is more preferably a protein lacking a β-glucan synthesis activity consisting of an amino acid sequence in which one or several amino acids are deleted, substituted, or added in the amino acid sequence of SEQ ID NO: 20, wherein the amino acid corresponding to position 253 of the amino acid sequence of SEQ ID NO: 20 is an amino acid other than cysteine. The amino acid corresponding to position 253 is more preferably tyrosine. In addition, the genomic DNA of a β-glucan-deficient gene of the present invention particularly preferably consists of the base sequence of SEQ ID NO: 18.

In another aspect, the present invention provides a cDNA of a β-glucan-deficient gene encoding a protein lacking a β-glucan synthesis activity consisting of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is an amino acid other than glycine.

In another aspect, the present invention provides a cDNA of a β-glucan-deficient gene encoding a protein lacking a β-glucan synthesis activity consisting of an amino acid sequence in which one or several amino acids are deleted, substituted, or added in the amino acid sequence of SEQ ID NO: 20, wherein the amino acid corresponding to position 253 of the amino acid sequence of SEQ ID NO: 20 is an amino acid other than cysteine.

The cDNA of a β-glucan-deficient gene is involved in the synthesis of β-glucan; thus, it can be used for the elucidation of a plant gene involved in the synthesis of β-glucan in the field of life science. If the gene involved in the synthesis of β-glucan is elucidated, the results can be used to genetically control the content of β-glucan in breeding.

In the amino acid sequence encoded by the cDNA of a β-glucan-deficient gene according to the present invention, the amino acid corresponding to position 660 of the amino acid sequence of SEQ ID NO: 2 is more preferably aspartic acid. The cDNA of a β-glucan-deficient gene according to the present invention also particularly preferably consists of the base sequence of SEQ ID NO: 3.

Alternatively, in the amino acid sequence encoded by the cDNA of a β-glucan-deficient gene according to the present invention, the amino acid corresponding to position 253 of the amino acid sequence of SEQ ID NO: 20 is more preferably tyrosine. The cDNA of a β-glucan-deficient gene according to the present invention also particularly preferably consists of the base sequence of SEQ ID NO: 19.

In another aspect, the present invention provides a genomic DNA of a β-glucan synthesis gene which is DNA consisting of a base sequence having 90% or more homology to the base sequence of SEQ ID NO: 4 and producing a protein having a β-glucan synthesis activity when transcribed and translated. The genomic DNA of a β-glucan synthesis gene particularly preferably consists of the base sequence of SEQ ID NO: 4.

In another aspect, the present invention provides a genomic DNA of a β-glucan synthesis gene which is DNA consisting of a base sequence having 90% or more homology to the base sequence of SEQ ID NO: 15 and producing a protein having a β-glucan synthesis activity when transcribed and translated. The genomic DNA of a β-glucan synthesis gene particularly preferably consists of the base sequence of SEQ ID NO: 15.

Although the gene involved in the synthesis of β-glucan has not been elucidated, the present inventors have determined that the above DNAs are involved in β-glucan synthesis. It is possible to develop a plant variety showing a high production level of β-glucan by a method such as increasing the number of copies of the genomic DNA of a β-glucan synthesis gene in the plant. β-Glucan has a higher use value because it has physiological effects such as the suppression of the increased blood glucose level and the reduction of blood cholesterol and effects such as the promotion of immune function.

In another aspect, the present invention provides a cDNA of a β-glucan synthesis gene which is DNA consisting of a base sequence having 90% or more homology to the base sequence of SEQ ID NO: 6 and encoding a protein having a β-glucan synthesis activity. The cDNA of a β-glucan synthesis gene particularly preferably consists of the base sequence of SEQ ID NO: 6.

Although the gene involved in the synthesis of β-glucan has not been elucidated, it has been determined according to the present invention that the above cDNA is involved in β-glucan synthesis. The cDNA can be introduced into a bacterium, a yeast, a plant, or the like by linking downstream of a suitable promoter to produce β-glucan on a large scale. β-Glucan has a high use value because it has physiological effects such as the suppression of the increased blood glucose level and the reduction of blood cholesterol and effects such as the promotion of immune function.

In another aspect, the present invention provides a method for breeding a barley having a genomic DNA of a β-glucan-deficient gene, comprising a selection step of selecting the barley by determining the barley as having a genomic DNA of a β-glucan-deficient gene when the base corresponding to position 4,275 of the genomic DNA consisting of the base sequence of SEQ ID NO: 4 located near the centromere of chromosome 7H of the barley is mutated from G (guanine) to A (adenine).

In another aspect, the present invention provides a method for breeding a barley having a genomic DNA of a β-glucan-deficient gene, comprising a selection step of selecting the barley by determining the barley as having a genomic DNA of a β-glucan-deficient gene when the base corresponding to position 2,385 of the genomic DNA consisting of the base sequence of SEQ ID NO: 4 located near the centromere of chromosome 7H of the barley is mutated from G to A.

These breeding methods can be used to breed a barley having a genomic DNA of a β-glucan-deficient gene. Conventional methods for determining the presence of β-glucan have been methods which use a kernel as a specimen to detect a fluorochrome specifically adsorbing to, or a product resulting from an enzymatic treatment of β-glucan. Thus, in order to perform the above determination, it is required to grow a plant until the stage in which seeds are formed. In contrast, the use of the present invention enables the determination to be carried out even in an infant plant before the formation of seeds and also enables the determination of a homozygous or heterozygous genotype. Therefore, the period necessary for breeding can be remarkably shortened.

In one aspect of the above breeding method, the present invention provides a breeding method comprising: an amplification step of amplifying a DNA fragment containing a base corresponding to position 4,275 of the base sequence of SEQ ID NO: 4 using a genomic DNA extracted from a barley as a template; a detection step of cleaving the DNA fragment amplified in the amplification step with a restriction enzyme selected from the group consisting of TaqI, BanI, and NlaIV and detecting the cleaved DNA fragment; and a selection step of selecting the barley by determining the barley as having a genomic DNA of a β-glucan-deficient gene when the DNA fragment is cleaved with the restriction enzyme TaqI or not cleaved with the restriction enzyme BanI or NlaIV in the detection step.

The breeding method enables rapid determination when the base is mutated which corresponds to position 4,275 of the genomic DNA consisting of the base sequence of SEQ ID NO: 4. The use of the restriction enzyme BanI or NlaIV enables determination when the base corresponding to the position 4,275 is mutated from G to A, C (cytosine), or T (thymine). The use of the restriction enzyme TaqI enables determination when the base corresponding to the position 4,275 is mutated from G to A.

In one aspect of the above breeding method, the present invention provides a breeding method comprising: an amplification step of amplifying a DNA fragment containing a base corresponding to position 2,385 of the base sequence of SEQ ID NO: 4 using a genomic DNA extracted from a barley as a template; a detection step of cleaving the DNA fragment amplified in the amplification step with a restriction enzyme, Fnu4HI, and detecting the cleaved DNA fragment; and a selection step of selecting the barley by determining the barley as having a genomic DNA of a β-glucan-deficient gene when the DNA fragment is not cleaved between the bases corresponding to positions 2,386 and 2,387 of SEQ ID NO: 4 in the detection step.

The breeding method enables rapid determination when the base is mutated which corresponds to position 2,385 of the genomic DNA consisting of the base sequence of SEQ ID NO: 4. Thus, when the base is G, the amplified DNA fragment is cleaved between the bases corresponding to positions 2,386 and 2,387 of SEQ ID NO: 4 by Fnu4HI, indicating that the barley is wild type. When the base corresponding to position 2,385 of the genomic DNA consisting of the base sequence of SEQ ID NO: 4 is mutated from G to A, C, or T, the above amplified DNA fragment is not cleaved between the bases corresponding to positions 2,386 and 2,387 of SEQ ID NO: 4 by Fnu4HI, indicating that the barley has a genomic DNA of a β-glucan-deficient gene.

In another aspect, the present invention provides a barley bred by the above method, the barley having any of the DNAs:

(a) a genomic DNA of a β-glucan-deficient gene consisting of a base sequence having 90% or more homology to the base sequence of SEQ ID NO: 1 and producing a protein lacking a β-glucan synthesis activity when transcribed and translated;

(b) the genomic DNA of a β-glucan-deficient gene according to (a) above, wherein the protein produced in the transcription and translation consists of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is an amino acid other than glycine;

(c) the genomic DNA of a β-glucan-deficient gene according to (b) above, wherein the amino acid other than glycine is aspartic acid;

(d) a genomic DNA of a β-glucan-deficient gene, consisting of the base sequence of SEQ ID NO: 1;

(e) a genomic DNA of a β-glucan-deficient gene consisting of a base sequence having 90% or more homology to the base sequence of SEQ ID NO: 18 and producing a protein lacking a β-glucan synthesis activity when transcribed and translated;

(f) the genomic DNA of a β-glucan-deficient gene according to (e) above, wherein the protein produced in the transcription and translation consists of an amino acid sequence in which one or several amino acids are deleted, substituted, or added in the amino acid sequence of SEQ ID NO: 20, wherein the amino acid corresponding to position 253 of the amino acid sequence of SEQ ID NO: 20 is an amino acid other than cysteine;

(g) the genomic DNA of a β-glucan-deficient gene according to (f) above, wherein the amino acid other than cysteine is tyrosine; and

(h) a genomic DNA of a β-glucan-deficient gene, consisting of the base sequence of SEQ ID NO: 18. The barley of the present invention more preferably has any genomic DNA of a β-glucan-deficient gene of (a) to (h) above in homozygous form.

A barley having any genomic DNA of a β-glucan-deficient gene of (a) to (h) above in heterozygous form has decreased β-glucan in a kernel as compared to a wild-type barley; a barley having any genomic DNA of a β-glucan-deficient gene of (a) to (h) above in homozygous form is completely deficient in kernel β-glucan.

In another aspect, the present invention provides a method for decreasing or abolishing β-glucan in barley kernels, comprising a step of suppressing the expression of a genomic DNA of a β-glucan synthesis gene consisting of a base sequence having 90% or more homology to the base sequence of SEQ ID NO: 4 and producing a protein having a β-glucan synthesis activity when transcribed and translated or a genomic DNA of a β-glucan synthesis gene consisting of the base sequence of SEQ ID NO: 4 and a barley decreasing or deficient in β-glucan obtainable by the method. This barley decreases or is completely deficient in kernel β-glucan.

In another aspect, the present invention provides a method for decreasing or abolishing β-glucan in barley kernels, comprising a step of suppressing the expression of a genomic DNA of a β-glucan synthesis gene consisting of a base sequence having 90% or more homology to the base sequence of SEQ ID NO: 15 and producing a protein having a β-glucan synthesis activity when transcribed and translated or a genomic DNA of a β-glucan synthesis gene consisting of the base sequence of SEQ ID NO: 15 and a barley decreasing or deficient in β-glucan obtainable by the method. This barley decreases or is completely deficient in kernel β-glucan.

In another aspect, the present invention provides a method for producing an alcohol, comprising a step of fermenting kernels derived from a barley bred by the above breeding method, which is a barley having any genomic DNA of a β-glucan-deficient gene of (a) to (h) above or a barley decreasing or deficient in kernel β-glucan obtainable by suppressing the expression of the above genomic DNA of a β-glucan synthesis gene. A barley having genomic DNA of a β-glucan-deficient gene in heterozygous form has decreased β-glucan in a kernel as compared to a wild-type barley; a barley having that in homozygous form is completely deficient in kernel β-glucan. Barley kernels decreasing or deficient in β-glucan can be used as a raw material to provide effects such as the shortening of the saccharification time of starch, the shortening of filtration time of wort, the increase of the alcohol formation and the decrease of fermentation residues in the production of beer or shochu.

In another aspect, the present invention provides a method for producing a fermented food, comprising a step of fermenting kernels derived from a barley bred by the above breeding method, which is a barley having any genomic DNA of a β-glucan-deficient gene of (a) to (h) above or a barley decreasing or deficient in kernel β-glucan obtainable by suppressing the expression of the above genomic DNA of a β-glucan synthesis gene. The fermented food is a food comprising one obtained by fermenting barley kernels and, for example, a soybean paste or a soy sauce. Barley kernels decreasing or deficient in β-glucan can be used as a raw material to provide effects such as the shortening of the time necessary for fermentation because the efficiency of fermentation can be increased.

In another aspect, the present invention provides an animal feed composition comprising kernels derived from a barley bred by the above breeding method, which is a barley having any genomic DNA of a β-glucan-deficient gene of (a) to (h) above or a barley decreasing or deficient in kernel β-glucan obtainable by suppressing the expression of the above genomic DNA of a β-glucan synthesis gene. The animal feed composition comprising barley kernels decreasing or completely deficient in β-glucan is excellent for digestion and absorption and has a high feeding effect. The improved digestibility results in the obtaining of effects such as the decrease of the amount of feces. The kernel can be more easily ground in producing the feed composition because the endosperm cell wall is thin and the kernel is soft.

In another aspect, the present invention provides a transformant holding, so as to be expressible, a vector containing any of the DNAs:

(a)a genomic DNA of a β-glucan synthesis gene consisting of a base sequence having 90% or more homology to the base sequence of SEQ ID NO: 4 and producing a protein having a β-glucan synthesis activity when transcribed and translated;
(b) a genomic DNA of a β-glucan synthesis gene, consisting of the base sequence of SEQ ID NO: 4;
(c) a cDNA of a β-glucan synthesis gene consisting of a base sequence having 90% or more homology to the base sequence of SEQ ID NO: 6 and encoding a protein having a β-glucan synthesis activity;
(d) a cDNA of a β-glucan synthesis gene consisting of the base sequence of SEQ ID NO: 6;
(e) a genomic DNA of a β-glucan synthesis gene consisting of a base sequence having 90% or more homology to the base sequence of SEQ ID NO: 15 and producing a protein having a β-glucan synthesis activity when transcribed and translated; and
(f) a genomic DNA of a β-glucan synthesis gene consisting of the base sequence of SEQ ID NO: 15. The transformant may be a barley or a plant containing no β-glucan or containing β-glucan only in an extremely small quantity. Alternatively, the transformant may be a microorganism exemplified by a prokaryote such as Escherichia coli or a eukaryote such as yeast.

Such a transformant plant has high added value since it contains β-glucan in more abundance. Alternatively, such a transformant microorganism can also be used to industrially produce β-glucan in volume.

The transformant more preferably overexpresses the genomic DNA of the β-glucan synthesis gene or the cDNA of the β-glucan synthesis gene. The overexpression means expressing high amounts of the β-glucan synthesis gene at the mRNA level or the protein level compared to the host before transformation.

## Advantageous Effects of Invention

According to the present invention, a genomic DNA and a cDNA of a β-glucan-deficient gene in a barley are provided. A genomic DNA and a cDNA of a β-glucan synthesis gene in a barley are also provided. Further provided are: a barley having the genomic DNA of a β-glucan-deficient gene and a method for breeding the same; a method for decreasing or abolishing β-glucan in barley kernels by suppressing the expression of a β-glucan synthesis gene, and a barley decreasing or deficient in β-glucan in kernels thereof, obtainable by the method; a method for producing an alcohol or a fermented food, comprising a step of fermenting these barley kernels decreasing or deficient in β-glucan; and an animal feed composition comprising the barley kernels.

The modification of a gene involved in the synthesis of β-glucan is useful in the elucidation of a gene involved in the synthesis of β-glucan in a plant in the field of life science and the breeding of a barley variety containing no β-glucan in the field of agriculture. The elucidation of a β-glucan-deficient gene and a β-glucan deficiency mechanism contribute to the identification of a gene involved in the synthesis of β-glucan. A barley deficient in β-glucan is an important resource for the development of a functional food specialized in arabinoxylan because of containing a slightly large amount of arabinoxylan important as a functional polysaccharide together with β-glucan. Conventional methods for determining the presence of β-glucan have been methods which use a kernel as a specimen to detect a fluorochrome specifically adsorbing to, or a product resulting from an enzymatic treatment of β-glucan. Thus, in order to perform the above determination, it is required to grow a plant until the stage in which seeds are formed. In contrast, the use of the present invention enables the determination to be carried out even in an infant plant before the formation of seeds and also enables the determination of a homozygous or heterozygous genotype. The use of a β-glucan-deficient gene according to the present invention enables the production of a barley in which the aleurone layer and the endosperm cell wall are completely deficient in β-glucan and the endosperm cell wall is noticeably thin.

Brief Description of Drawings

Figure 1 is a diagram showing a position on which a β-glucan-deficient gene is located on a chromosome.
Figure 2 is a diagram showing a base sequence ofHvCslF6 gene and a deduced amino acid sequence therefrom in a β-glucan-deficient barley variety and a wild-type variety thereof.
Figure 3 is a set of photographs showing an example of the determination of a HvCslF6 genotype using a CAPS marker.
Figure 4 is a set of photographs showing observations of kernel fragments of a Nishinohoshi (bgl) near-isogenic line and Nishinohoshi under a light microscope.
Figure 5 is a set of photographs showing observations of kernel fragments of a Nishinohoshi (bgl) near-isogenic line and Nishinohoshi under a light microscope and a scanning electron microscope.
Figure 6 is a diagram showing promoter sequences of HvCslF6 genes in TR251 and CDC-Bold.
Figure 7 is a diagram showing promoter sequences of HvCslF6 genes in TR251 and CDC-Bold.
Figure 8 is a diagram showing base sequences of HvCslF6 genes in Sachiho Golden (TN5) and KM27. TN5-DNA indicates a portion of a base sequence of genomic DNA of a HvCslF6 gene (SEQ ID NO: 15) in Sachiho Golden. TN5-cDNA indicates a portion of a base sequence of cDNA of a HvCslF6 gene (SEQ ID NO: 16) in Sachiho Golden. TN5-a.a. indicates a portion of an amino acid sequence of HvCslF6 (SEQ ID NO: 17) in Sachiho Golden. KM27-DNA indicates a portion of a base sequence of genomic DNA of a HvCslF6 gene (bgl gene) (SEQ ID NO: 18) in KM27. KM27-cDNA indicates a portion of a base sequence of cDNA of bgl gene (SEQ ID NO: 19) in KM27. KM27-a.a. indicates a portion of an amino acid sequence (SEQ ID NO: 20) encoded by bgl gene in KM27.

Figure 9 is a diagram showing a predicted three-dimensional structure of HvCslF6. (i) indicates a site at which cysteine is mutated to tyrosine in KM27. (ii) indicates a site at which glycine is mutated to aspartic acid in OUM125.

Figure 10 is a photograph showing an example of the determination of a HvCslF6 genotype using a CAPS marker.

## Description of Embodiments

As used herein, a "base sequence having 90% or more homology" means a base sequence whose bases are the same as 90% or more, preferably 95% or more, more preferably 98% or more, still more preferably 99% or more of the bases of another base sequence when these two base sequences are compared by alignment such that as many of their bases as possible coincide with each other. Here, in aligning the base sequences, gaps may be contained so as to provide the maximum homology.

As used herein, an "amino acid sequence in which one or several amino acids are deleted, substituted, or added" means an amino acid sequence in which 1 to 10, more preferably 1 to 5 amino acids are deleted, substituted, or added in comparison to a reference amino acid sequence.

As used herein, "genomic DNA" means DNA containing introns and "cDNA" means DNA containing no introns. For example, the base sequence of SEQ ID NO: 1 shows the base sequence of genomic DNA of a β-glucan-deficient gene; positions 328 to 1,954 and positions 2,699 to 3,367 of this base sequence are introns. Introns are removed by splicing in the process that the genomic DNA is transcribed into mRNA and translated into a protein in a host. Here, the host is preferably a plant and more preferably a barley. For example, SEQ ID NO: 3 shows a base sequence of cDNA of a β-glucan-deficient gene in which an intron portion is removed from the base sequence of SEQ ID NO: 1. As used herein, a "β-glucan-deficient gene" may mean both of the genomic DNA and the cDNA.

As used herein, a "β-glucan synthesis activity" means an activity participating in the pathway of β-glucan synthesis and promoting the synthesis of β-glucan. In addition, "β-glucan synthesis gene" means a gene encoding a protein having the function of participating in the pathway of β-glucan synthesis and promoting the synthesis of β-glucan.

In one aspect, the present invention provides a method for breeding a barley having a genomic DNA of a β-glucan-deficient gene, comprising a selection step of selecting the barley by determining the barley as having a genomic DNA of a β-glucan-deficient gene when the base corresponding to position 4,275 of the genomic DNA consisting of the base sequence of SEQ ID NO: 4 located near the centromere of chromosome 7H of the barley is mutated from G to A. In the selection step according to the breeding method, the method for detecting the mutation of the genomic DNA is not particularly limited. For example, the region on genomic DNA to be detected may be subjected to PCR amplification, followed by determining the base sequence of the PCR fragment by the direct sequencing thereof or by sequencing after inserting the PCR fragment into a sequencing vector to determine whether the fragment has the intended mutation. However, because of simpler determination operation, it is preferable to detect the presence of the intended mutation using the cleavability by a restriction enzyme as an indicator.

Specifically, using a genomic DNA extracted from a barley as a template, for example, the DNA fragment consisting of the base sequence corresponding to positions 3,893 to 4,361 of the base sequence of SEQ ID NO: 4 is PCR amplified; the amplified 469 bp DNA fragment is cleaved with a restriction enzyme, BanI; the cleaved DNA fragment is detected by agarose electrophoresis or the like; and the barley can be determined as having a genomic DNA of a β-glucan-deficient gene when the DNA fragment is not cleaved with the restriction enzyme BanI. This method enables rapid determination when the base corresponding to position 4,275 of the genomic DNA consisting of the base sequence of SEQ ID NO: 4 is mutated to A. In addition, it similarly enables determination when the base corresponding to the position 4,275 is mutated to C or T.

TaqI, NlaIV, or the like as well as BanI can be used as a restriction enzyme. The recognition sequence of TaqI is 5'-TCGA-3', which is cleaved into the form of 5-T/CGA-3'by digestion with TaqI. Thus, the base corresponding to the position 4,275 of the genomic DNA consisting of the base sequence of SEQ ID NO: 4 is cleaved when it has been mutated from G to A. The recognition sequence of NlaIV is 5'-GGNNCC-3', which is cleaved into the form of 5'-GGN/NCC-3' by digestion with NlaIV. Here, N means A, T, G, or C. Thus, the base corresponding to the position 4,275 of the genomic DNA consisting of the base sequence of SEQ ID NO: 4 is cleaved when it has been mutated from G to A, C, or T.

A plurality of the recognition sequences cleaved by restriction enzymes such as BanI, TaqI or NlaIV are present around the base corresponding to the position 4,275 of the genomic DNA consisting of the base sequence of SEQ ID NO: 4. In determining the mutation of the base corresponding to the 4,275 position by cleavage using each of these restriction enzymes, those skilled in the art can easily select which region of the base sequence of SEQ ID NO: 4 can be amplified to prepare a DNA fragment for determination. Usable restriction enzymes other than these restriction enzymes will be apparent to those skilled in the art.

The method for extracting a genomic DNA from a barley is not particularly limited; for example, the DNA can be extracted by collecting about 0.1 g of young leaves and milling them on a mortar after adding liquid nitrogen, followed by extraction using DNeasy Plant Mini Kit (Qiagen Inc.) according to an instruction.

The breeding method of the present embodiment can be carried out as follows, for example. A wild-type barley is first treated with a mutation-inducing agent such as ethyl methanesulfonate (EMS) to provide mutants. Subsequently, a genomic DNA is extracted from each of the resultant mutants, and the mutation of the base corresponding to the position 4,275 of the genomic DNA consisting of the base sequence of SEQ ID NO: 4 is detected by the above method to determine and select a barley individual having genomic DNA of a β-glucan-deficient gene. In this way, a barley having a genomic DNA of a β-glucan-deficient gene can be bred. Alternatively, a barley having a genomic DNA of a β-glucan-deficient gene can be bred by crossing a wild-type barley variety with a barley variety already shown to have a genomic DNA of a β-glucan-deficient gene and, from among the progenies thereof, determining and selecting a barley having the genomic DNA of a β-glucan-deficient gene by the same method as that described above. Alternatively, a barley having a genomic DNA of a β-glucan-deficient gene can be bred by artificially mutating the base corresponding to the position 4,275 of the genomic DNA consisting of the base sequence of SEQ ID NO: 4 in a wild type barley from G to A, C, or T using a molecular biological technique such as a gene-targeting approach by homologous recombination. The artificially introduced mutation is preferably mutation from G to A.

In another aspect, the present invention provides a method for breeding a barley having a genomic DNA of a β-glucan-deficient gene, comprising a selection step of selecting the barley by determining the barley as having a genomic DNA of a β-glucan-deficient gene when the base corresponding to position 2,385 of the genomic DNA consisting of the base sequence of SEQ ID NO: 4 located near the centromere of chromosome 7H of the barley is mutated from G to A. In the selection step according to the breeding method, the method for detecting the mutation of the genomic DNA is not particularly limited. For example, the region on a genomic DNA to be detected may be subjected to PCR amplification, followed by determining the base sequence of the PCR fragment by the direct sequencing thereof or by sequencing after inserting the PCR fragment into a sequencing vector to determine whether the fragment has the intended mutation. However, because of simpler determination operation, it is preferable to detect the presence of the intended mutation using the cleavability by a restriction enzyme as an indicator.

Specifically, using a genomic DNA extracted from a barley as a template, for example, the DNA fragment consisting of the base sequence corresponding to positions 2,219 to 2,529 of the base sequence of SEQ ID NO: 4 is PCR amplified, and the amplified 311 bp DNA fragment is cleaved with a restriction enzyme, Fnu4HI; the cleaved DNA fragment is detected by agarose electrophoresis or the like. In a wild-type variety, digestion with the restriction enzyme Fnu4HI cleaves the amplified fragment into 6 fragments with 3, 41, 50, 56, 62 and 99 bp. In contrast, when the base corresponding to position 2,385 of the genomic DNA consisting of the base sequence of SEQ ID NO: 4 is mutated from G to A or mutated from G to C or T, digestion with Fnu4HI cleaves the amplified fragment into 5 fragments with 3, 41, 50, 56 and 161 bp because one of the recognition sites of Fnu4HI is eliminated by single base substitution. The difference in the fragments produced by Fnu4HI digestion can be easily distinguished by electrophoresis or the like as shown in Figure 10. This method enables rapid determination of the presence of mutation of the base corresponding to position 2,385 of the genomic DNA consisting of the base sequence of SEQ ID NO: 4.

The recognition sequence of Fnu4HI is 5'-GCNGC-3', which is cleaved into the form of 5'-GC/NGC-3' by Fnu4HI digestion. Here, N means A, T, G, or C. BisI, BsoFI, Fsp4HI, ItaI, SatI, and the like are known as restriction enzymes recognizing the same base sequence as that for Fnu4HI and cleaving the sequence into the same form as that for Fnu4HI. These restriction enzymes can be used in the same way as that for Fnu4HI.

A plurality of the recognition sequences cleaved by Fnu4HI are present around the base corresponding to the position 2,385 of the genomic DNA consisting of the base sequence of SEQ ID NO: 4. In determining the mutation of the base corresponding to the 2,385 position by cleavage using Fnu4HI, those skilled in the art can easily select which region of the base sequence of SEQ ID NO: 4 can be amplified to prepare a DNA fragment for determination. Usable restriction enzymes other than Fnu4HI will be apparent to those skilled in the art.

The breeding method of the present embodiment can be carried out as follows, for example. A wild-type barley is first treated with a mutation-inducing agent such as ethyl methanesulfonate (EMS) to provide mutants. Subsequently, a genomic DNA is extracted from each of the resultant mutants, and the mutation of the base corresponding to the position 2,385 of the genomic DNA consisting of the base sequence of SEQ ID NO: 4 by the above method is detected to determine and select a barley individual having a genomic DNA of a β-glucan-deficient gene. In this way, a barley having a genomic DNA of a β-glucan-deficient gene can be bred. Alternatively, a barley having genomic DNA of a β-glucan-deficient gene can be bred by crossing a wild-type barley variety with a barley variety already shown to have a genomic DNA of a β-glucan-deficient gene and, from among the progenies thereof, determining and selecting a barley having the genomic DNA of a β-glucan-deficient gene by the same method as that described above. Alternatively, a barley having a genomic DNA of a β-glucan-deficient gene can be bred by artificially mutating the base corresponding to the position 2,385 of the genomic DNA consisting of the base sequence of SEQ ID NO: 4 in a wild type barley from G to A, C, or T using a molecular biological technique such as a gene-targeting approach by homologous recombination. The artificially introduced mutation is preferably mutation from G to A.

In one aspect, the present invention provides a genomic DNA and a cDNA of a β-glucan synthesis gene in a barley. It is possible to develop a plant variety showing a high production level of β-glucan by a method such as a method involving introducing the genomic DNA or cDNA of a β-glucan synthesis gene of the present invention into the genome of a plant such as a barley and increasing the number of copies thereof. Alternatively, it is also possible to industrially produce β-glucan on a large scale by introducing the genomic DNA or cDNA of a β-glucan synthesis gene of the present invention into a microorganism including a prokaryote such as *Escherichia coli* or a eukaryote such as yeast. β-Glucan has a high use value because it has physiological effects such as the suppression of the increased blood glucose level and the reduction of blood cholesterol and effects such as the promotion of immune function.

When a β-glucan synthesis gene is introduced into a plant body, a β-glucan synthesis gene expression vector is first constructed. The expression vector comprises a promoter expressible in a plant body, a β-glucan synthesis gene, and a translational termination sequence. The β-glucan synthesis gene may be a genomic DNA or a cDNA. Here, the plant body providing a host is not particularly limited; however, it is preferably a barley. The expression vector may also comprise a replication origin in *E. coli,* a selection marker in *E. coli* (an ampicillin-resistant gene, a tetracycline resistance gene, or the like), a selection marker in a plant body (a kanamycin resistance gene, a hygromycin resistance gene, a bialaphos (bar) resistance gene, or the like), a polyadenylation sequence, an enhancer sequence, and the like.

The promoter expressible in a plant body may be one inducing seed-specific expression or one performing constitutive expression independent of tissue. Examples thereof can include an ubiquitin promoter, an actin promoter, an Em promoter, and the 35S promoter of cauliflower mosaic virus.

The introduction of a gene into a barley can be performed using a common particle-bombardment method or an *Agrobacterium* method. For example, a gene is introduced by delivering gold particles coated with a β-glucan synthesis gene expression vector DNA into an immature embryo taken out of an immature barley seed using the particle-bombardment method. Subsequently, the gene-introduced immature embryo can be cultured in a medium containing auxin or the like as a phytohormone, followed by decreasing the concentration of auxin to redifferentiate the individual to provide a transformant plant body. For example, when a β-glucan synthesis gene expression vector containing a biapholas resistance gene has been introduced into a barley, the transformant plant body having incorporated the transgene can be selected using a medium containing biapholas because it becomes resistant to biapholas. Alternatively, a β-glucan synthesis gene can be introduced into a barley by immersing a barley-derived callus in a solution of *Agrobacterium* having a β-glucan synthesis gene expression vector introduced thereinto for infection with *Agrobacterium.* For example, when a β-glucan synthesis gene expression vector containing a hygromycin resistance gene has been introduced into a barley, the transformant having incorporated the gene can be selected in a medium containing hygromycin and a bacteria-eliminating agent such as carbenicillin for eliminating *Agrobacterium.* Thereafter, the resultant transformant can be cultured in an auxin-removed redifferentiation medium for redifferentiation and then turned into a rooting medium to provide a transformant plant body holding the β-glucan synthesis gene so as to be expressible.

A microorganism exemplified by a prokaryote such as *E. coli* or a eukaryote such as yeast can also be used as a host for introducing the β-glucan synthesis gene expression vector. By way of non-limited example, the K-12 strain is preferably used as *E. coli* and a pBR322 or pUC plasmid is typically used as a vector. A tryptophan (trp) promoter, a lactose (lac) promoter, or the like can be used as a promoter for *E. coli.* As yeast, for example, a Saccharomyces yeast, e.g., Saccharomyces cerevisiae, a bakery yeast, or Pichia pastoris, a petroleum yeast, can be used. As a promoter for yeast, for example, a promoter for alcohol dehydrogenase gene, a promoter for acid phosphatase gene, or the like can be used.

In one aspect, the present invention provides a method for decreasing or abolishing β-glucan in barley kernels, comprising a step of suppressing the expression of genomic DNA of a β-glucan synthesis gene as DNA consisting of a base sequence having 90% or more homology to the base sequence of SEQ ID NO: 4 and producing a protein having a β-glucan synthesis activity when transcribed and translated or genomic DNA of a β-glucan synthesis gene consisting of the base sequence of SEQ ID NO: 4.

In one aspect, the present invention provides a method for decreasing or abolishing β-glucan in barley kernels, comprising a step of suppressing the expression of genomic DNA of a β-glucan synthesis gene as DNA consisting of a base sequence having 90% or more homology to the base sequence of SEQ ID NO: 15 and producing a protein having a β-glucan synthesis activity when transcribed and translated or genomic DNA of a β-glucan synthesis gene consisting of the base sequence of SEQ ID NO: 15.

A known method involving introducing an siRNA or antisense nucleic acid into cells can be used as a method for suppressing the expression of DNA. Specifically, for example, a vector for expressing an siRNA targeting the genomic DNA of a β-glucan synthesis gene or a vector for expressing an antisense RNA targeting the genomic DNA of a β-glucan synthesis gene may be introduced into a barley using a particle-bombardment method or an *Agrobacterium* method as described above. According to this method, a barley decreasing or completely deficient in β-glucan in kernels can be obtained by suppressing the expression of the genomic DNA of a β-glucan synthesis gene.

Examples

The present invention will be described more specifically below with reference to Examples of the present invention. However, the present invention is not intended to be limited to these Examples, and various changes can be made without departing from the technical idea of the present invention.

(Production of Barley Cultivar "Nishinohoshi" Near-Isogenic Line)

The present inventors have found that a barley cultivar (line), OUM125, is deficient in β-glucan, and identified a gene deficient in β-glucan based thereon, thereby accomplishing the present invention. OUM125 is a semidwarf mutant produced by treating a six-rowed naked barley cultivar, Akashinriki, with ethyl methanesulfonate (EMS) in Okayama University. The seeds of OUM125 used in this Example were provided by Dr. Kazuhiro Sato in the Barley and Wild Plant Resource Center, Institute of Plant Science and Resources, Okayama University.

Nishinohoshi is a two-rowed barley variety having superior traits. Izumi-kei A41 is a line produced by crossing a progeny between Saikaikawa 55 and OUM125 with Saikaikawa 54 (development name of Nishinohoshi) in the Kyushu Agricultural Experiment Station in 1997, and has β-glucan deficiency and a naked caryopsis. Inventors used Izumi-kei A41 as a nonrecurrent parent and Nishinohoshi as a recurrent parent for crossing to produce a β-glucan-deficient and hulled Nishinohoshi near-isogenic line.

(Analysis of β-Glucan Deficiency)

Individual kernels are each cut in the transverse direction and a portion away from the embryo was crushed using a pair of pinchers. The β-glucan deficiency was determined by treating the crushed kernels with lichenase and β-glucosidase and color developing glucose using a glucose assay kit (Glucose C-II Test, Wako Pure Chemical Industries, Ltd.). The reaction solution became colorless or light pink for β-glucan-deficient kernels, whereas the reaction solution became deep red for wild-type kernels.

(Linkage Analysis of β-Glucan Deficiency and Hulled and Naked Caryopsis)

The linkage analysis of β-glucan deficiency and naked caryopsis was performed in 228 individuals obtained by crossing Nishinohoshi with its near-isogenic line. The results of the linkage analysis are shown in Table 1. The segregation ratio of β-glucan-deficient type versus wild type was 1:3. Thus, β-glucan deficiency was probably due to a monofactorial recessive gene. This gene was designated a β-glucan-deficient gene ((1-3,1-4)-β-D-glucanless, bgl). A significant linkage was detected between a β-glucan-deficient gene and a naked caryopsis (nud) gene. The recombination value between these genes was calculated to be 14.4% ± 2.5% by the maximum-likelihood method of Allard (Hilgardia 24:235-278, 1956). The nud gene is known to be present on the long arm of chromosome 7H; thus, the β-glucan-deficient gene was also considered to be located on the chromosome 7H.

[Table 1]

Table 1: Joint Segregation of β-Glucan Deficiency and Naked Caryopsis in $F_2$ of Nishinohoshi (β-Glucan Deficient + Naked) Near-Isogenic Line x Nishinohoshi

| | Presence of β-Glucan | | Total |
|---|---|---|---|
| | Normal | Deficient | |
| Hulled | 153 | 15 | 168 |
| Naked | 16 | 44 | 60 |
| Total | 169 | 59 | 228 |

Test of Segregation Ratio for Normal Type and Deficient Type of β-Glucan

$$X^2(3:1)=0.0936,\ 0.90>p>0.75$$

Test of Independence between β-Glucan Deficiency and Naked Caryopsis

$$X^2L=101.3,\ p<0.01$$

Recombination Value between β-Glucan Deficiency and Naked Caryopsis = 14.4±2.5%

(Mapping)

Genomic DNAs were extracted from Nishinohoshi, its near-isogenic line, and 228 individuals obtained by their crossing by the CTAB method. Using the SSR marker reported by Ramsay et al. (Genetics 156:1997-2005, 2000), the genomic DNAs were analyzed and the β-glucan-deficient gene was mapped. The SSR markers on chromosome 7H showing polymorphism between the parents were selected and PCR analyzed. The position of each marker on the chromosome was determined by analysis using a wheat line having a barley chromosome added (Islam, Sakamoto, S. ed., Proc. 6th Int. Wheat Genet. Symp. Maruzen Kyoto. pp. 233-238, 1983). The recombination value was calculated using MAPMAKER version 2.0 (Lander et al., Genomics 1: 174-181, 1987). The genetic distance was calculated using Kosambi function (Kosambi, Ann. Eugen. 12: 172-175, 1944).

The results of mapping are shown in Figure 1. The β-glucan-deficient gene was mapped at a position 3.4 cM distant from Bmac0162, indicating that it is co-localized with Bmag0321, Bmag0359, Bmac0167 and HvCslF6 genes. The HvCslF6 gene is one of the cellulose synthase-like gene subfamilies. The β-glucan-deficient gene was demonstrated to be located near the centromere of chromosome 7H.

It was further demonstrated that a single nucleotide polymorphism (SNP) capable of being detected with the restriction enzyme BanI is present in HvCslF6 gene and the phenotype of β-glucan deficiency completely agrees with the genotype of HvCslF6 gene locus. That is, as shown in Figure 2, HvCslF6 gene whose base of position 4,275 is substituted from G to A is a β-glucan-deficient gene; when the β-glucan-deficient gene is held in homozygous form, kernels are completely deficient in β-glucan. As shown in Figure 2, the deduced amino acid is glycine for the wild-type variety and aspartic acid for the variety having the β-glucan-deficient gene.

(CAPS Analysis)

The presence of a β-glucan-deficient gene can be clearly determined by a co-dominant CAPS (cleaved amplified polymorphic sequence) marker. Here, the co-dominant marker means a marker by which a heterotype pattern can be distinguished from both homotypes of parents. To perform the CAPS analysis of a β-glucan-deficient gene, the primers (CAPS marker): 5'-GCCAAGACCAAGTACGAGAAGC-3' (forward, SEQ ID NO: 11) and 5'-TGTTCTTGGAGAAGAAGATCTCG-3' (reverse, SEQ ID NO: 12) were prepared.

Using these primers, a genomic DNA of a barley is subjected to PCR to provide an amplified fragment with 469 bp. For a wild type variety, the amplified fragment is cleaved into 382 bp and 87 bp by digestion with the restriction enzyme BanI. In contrast, for a variety having a β-glucan-deficient gene, the amplified fragment is not cleaved by digestion with the restriction enzyme BanI because of the absence of a BanI recognition site due to single base substitution. The recognition sequence of BanI is 5'-GGYRCC-3', which is cleaved into the form of 5'-G/GYRCC-3'by digestion with BanI. Here, Y means C or T, and R means A or G.

Figure 3 shows the results of determining the presence of a β-glucan-deficient gene by the CAPS analysis. After the PCR amplification of the genomic DNA using the above primers, the amplified fragment was cleaved with the restriction enzyme BanI and subjected to agarose gel electrophoresis. Lanes A to F are the results of analyzing the genomic DNA of OUM125 (β-glucan deficient), Akashinriki (wild type), F1 (hetero type) between OUM125 and Akashinriki, a Nishinohoshi near-isogenic line (β-glucan-deficient and naked), Nishinohoshi (wild type), and F1 (hetero type) between the Nishinohoshi near-isogenic line (β-glucan-deficient and naked) and Nishinohoshi.

(Measurement of Content of Starch, β-Glucan and Arabinoxylan)

Nishinohoshi (bgl) and Nishinohoshi were grown and harvested. Here, Nishinohoshi (bgl) means a Nishinohoshi near-isogenic line which is β-glucan-deficient and hulled. After the harvest, they were each ground and passed through a 0.5 mm sieve, followed by measuring the contents of β-glucan and arabinoxylan in a mature whole-kernel sample.

The content of β-glucan was measured by an enzymatic method (McCleary and Codd, 1991) using Mixed Linkage β-Glucan Assay Kit (Megazyme International, Ireland). The content of arabinoxylan was determined by, according to the method of Sekiwa et al. (2003), hydrolyzing the sample with sulfuric acid and then separately measuring the contents of arabinose and xylose. The content of arabinose was measured by an enzymatic method as described in "Arabinan assay procedure (Megazyme International Ireland 2002)". The content of xylose was measured using D-Xylose Assay Kit (Megazyme International, Ireland). Before measuring the contents of β-glucan and arabinoxylan, free low molecular saccharides in the ground sample was removed by treatment with 80% ethanol. The results are shown in Table 2.

β-Glucan was not detected in a Nishinohoshi (bgl) kernel, while 3.2% by mass of β-glucan was found to be contained in a Nishinohoshi kernel. The content of arabinoxylan was 6.3% by mass in the Nishinohoshi kernel, while being 7.2% by mass in the Nishinohoshi (bgl) kernel.

[Table 2]

Table 2: Content of β-Glucan and Arabinoxylan in Kernel of Nishinohoshi (bgl) Near-Isogenic Line

| Variety/Line | β-Glucan (%) | Arabinoxylan (%) |
|---|---|---|
| Nishinohoshi (bgl) Near-Isogenic Line | 0.0a | 7.2b |
| Nishinohoshi | 3.2b | 6.3a |

Significant difference are present at a 5% level between the numerical values to which the different alphabets are attached (Fisher's PLSD test).

(Observation under Light Microscope)

Nishinohoshi (bgl) and Nishinohoshi kernels were each sliced, fixed in glutaraldehyde, dehydrated by a series of ethanol treatments, and embedded in a low temperature polymerization resin, Technovit 7100 (Heraeus Kulzer, Germany). A 7 μm-thick section was double-stained using 0.5% Fast Green FCF (Sigma-Aldrich Corporation, USA) and 0.01% Calcofluor (Wako Pure Chemical Industries, Ltd.). The section was observed by a factor of 200 times under a light microscope equipped with a UV filter (Axiophot, Carl Zeiss, Germany). The cell wall, cytoplasm and nucleus are stained by Fast Green FCF, and β-glucan is stained by Calcofluor. The results are shown in Figure 4. The observation magnification was 200 times. In Figure 4, A indicates bright-field observation images; B indicates ultraviolet radiation images. In Figure 4, a means a husk; b, a seed coat and a seed vessel; c, an aleurone layer; and d, an endosperm. Fluorescence was observed in the aleurone and endosperm cell wall for the sample of Nishinohoshi, while their fluorescence was not observed for Nishinohoshi (bgl). This result shows that the line having a β-glucan-deficient gene is completely deficient in β-glucan in both of the aleurone layer and the endosperm.

(Observation under Electron Microscope)

Horizontal sections of Nishinohoshi (bgl) and Nishinohoshi kernels were each observed by a factor of 5,000 times under a scanning electron microscope (N-3400, Hitachi). The results are shown in Figure 5. In Figure 5, A indicates bright-field observation images (observation magnification: 200 times) under a light microscope; B indicates electron microscope observation images (observation magnification: 5,000 times). It was demonstrated that whereas Nishinohoshi had a thick endosperm cell wall, Nishinohoshi (bgl) had a markedly thin endosperm cell wall. The cell wall of aleurone cells was not found to be different in thickness therebetween.

(Measurement of β-Glucan Content in Seedling Leaf)

Before the extension of the stem of Nishinohoshi (bgl) and Nishinohoshi, their infant plants in a rosette state were each collected from a field and freeze dried. Subsequently, the contents of β-glucan in the leaf blade and sheath thereof were each measured using Mixed Linkage β-Glucan Assay Kit (Megazyme International, Ireland). Before the measurement of the β-glucan content, free low molecular saccharides were removed by 80% hot ethanol treatment. The results are shown in Table 3.

Little or no β-glucan was detected in leaves of Nishinohoshi (bgl), while 13.5 mg/g of β-glucan was contained in leaves of Nishinohoshi. The above result shows that Nishinohoshi (bgl) is deficient in β-glucan not only in kernels but also in the vegetative organ.

[Table 3]

| Table 3: Content of β-Glucan in Seedling Leaf of Nishinohoshi (bgl) Near-Isogenic Line | |
|---|---|
| Variety/Line | β-Glucan (mg/g) |
| Nishinohoshi (bgl) Near-Isogenic Line | 0.1a |
| Nishinohoshi | 13.5b |
| Significant difference are present at a 5% level between the numerical values to which the different alphabets are attached (Fisher's PLSD test). | |

(Measurement of Kernel Hardness)

The kernel hardness of Nishinohoshi (bgl) and Nishinohoshi kernels were each measured. The kernel hardness was measured in 300 grains each of the kernels using a single-kernel hardness tester (SKCS-4100, Perten Inc., Sweden). The milling time is set at the time necessary for milling 180 g of kernels (raw barley) to a yield of 56% using a test mill (TM-05, Satake Corporation, Higashi-Hiroshima City), and the kernel breakage rate was determined as the mass ratio of broken kernels and defective kernels in 10 g of the milled barley. The results are shown in Table 4.

Nishinohoshi (bgl) kernels were shown to become markedly soft and brittle compared to Nishinohoshi kernels. This result probably stems from the thinning of the endosperm cell wall due to β-glucan deficiency. The characteristic that the kernels are soft and brittle has the usefulness that it makes the crushing thereof easy in the production of an animal feed and facilitates the processing thereof into grits.

[Table 4]

| Table 4: Characteristics of Kernel of Nishinohoshi (bgl) Near-Isogenic Line | | | |
|---|---|---|---|
| Variety/Line | Kernel Hardness (Hardness Index) | Milling Time (second) | Kernel Breakage Rate (%) |
| Nishinohoshi (bgl) Near-Isogenic Line | 30.8a | 265a | 35.9b |
| Nishinohoshi | 74.9b | 497b | 3.8a |
| Significant difference are present at a 5% level between the numerical values to which the different alphabets are attached (Fisher's PLSD test). | | | |

(Polymorphism in Promoter Region of HvCslF6 Gene)

Barley lines, TR251 and CDC-Bold, are known to be different in the production amount of β-glucan (Journal of Cereal Science 48:647-655, 2008). TR251 is a high β-glucan production line, and CDC-Bold is a low β-glucan production line. The present inventors have believed that the difference in the production amount of β-glucan between these lines may be due to the mutation of the base sequence of HvCslF6 gene, and determined the base sequences of the genomes of HvCslF6 gene regions of these barley lines. TR251 was distributed by Dr. W.G Legge (Brandon Research Centre, Agriculture and Agri-Food Canada, Canada). CDC-Bold was distributed by Dr. B.G. Rossnagel (Crop Development Centre, University of Saskatchewan, Canada).

Genomic DNAs were extracted from these barley lines by the CTAB method, and the base sequences of the genomes were determined which lay about 2 kb upstream of the initiation codons of HvCslF6 genes, containing the promoter regions of HvCslF6 genes (hereinafter referred to as "promoter sequences"). The promoter sequences of the HvCslF6 genes of TR251 and CDC-Bold are shown in SEQ ID NOS: 13 and 14.

As shown in Figures 6 and 7, the comparison of these base sequences demonstrated that the bases corresponding to positions 10, 45, 614 and 2,002 of the base sequence of SEQ ID NO: 14 (the promoter sequence of CDC-Bold) in the promoter sequence of TR251 (SEQ ID NO: 13) were mutated from A to T, from A to G, from G to A, and from C to A, respectively. In addition, the 8 base pairs of 5'-TCTCTCAA-3' were observed to be inserted between the bases corresponding to positions 678 and 679 of the base sequence of SEQ ID NO: 14. In the figure, each mutation site is indicated by an open character. It was shown that TR251 displayed the phenotype of highly producing β-glucan because of the differences in the base sequence.

A barley variety highly producing or lowly producing β-glucan can be efficiently bred using polymorphism in these promoter regions as an indicator.

Typical TATA boxes were found at positions 1,755 to 1,758 (about 350 bp upstream of the initiation codon of the HvCslF6 gene) of the base sequence of SEQ ID NO: 14 (the promoter sequence of CDC-Bold) and the positions corresponding thereto in the promoter sequence of TR251 (SEQ ID NO: 13). In Figure 7, the TATA boxes are each indicated by open characters. The initiation codons of HvCslF6 genes were each boxed, and the coding regions were underlined.

(Polymorphism in Coding Region of HvCslF6 Gene)

The base sequence of the genomic DNA of the coding region of the HvCslF6 gene of TR251 is shown in SEQ ID NO: 23; the base sequence of the cDNA of the region, in SEQ ID NO: 24; and the deduced amino acid sequence therefrom, in SEQ ID NO: 25. The base sequence of the genomic DNA of the coding region of the HvCslF6 gene of CDC-Bold is shown in SEQ ID NO: 26; the base sequence of the cDNA of the region, in SEQ ID NO: 27; and the deduced amino acid sequence therefrom, in SEQ ID NO: 28. It was demonstrated that the bases corresponding to positions 174, 1,768 and 2,505 of the sequence of the cDNA of the HvCslF6 gene of Akashinriki (wild type) (SEQ ID NO: 6) in the base sequence of the cDNA of the HvCslF6 gene of TR251 (SEQ ID NO: 24) were mutated from G to A, from G to A, and from T to G, respectively. Of these mutations, the mutation in the base of the position 1,768 was demonstrated to result in the mutation of the amino acid corresponding to position 590 of the amino acid sequence of the HvCslF6 protein of Akashinriki (wild type) (SEQ ID NO: 5) in the amino acid sequence of the HvCslF6 protein of TR251 (SEQ ID NO: 25) from alanine to threonine. These mutations also probably have the possibility of having to do with the fact that TR251 is a high β-glucan production line.

(Obtaining New β-Glucan-Deficient Mutant)

The inventors have discovered a new β-glucan-deficient mutant of a barley variety, Sachiho Golden (TN5) (wild type), and designated it as KM27. Then, the base sequences of the HvCslF6 genes of Sachiho Golden and KM27 were determined. The HvCslF6 gene of KM27 is hereinafter referred to as a β-glucan-deficient gene. The base sequence of the genome of the HvCslF6 gene of Sachiho Golden is shown in SEQ ID NO: 15; the base sequence of the cDNA of the gene, in SEQ ID NO: 16; and the deduced amino acid sequence therefrom, in SEQ ID NO: 17. The base sequence of the genome of the β-glucan-deficient gene of KM27 is shown in SEQ ID NO: 18; the base sequence of the cDNA of the gene, in SEQ ID NO: 19; and the deduced amino acid sequence therefrom, in SEQ ID NO: 20.

As shown in Figure 8, it was demonstrated that the base of position 2,385 of the base sequence of the genome of the β-glucan-deficient gene of KM27 shown in SEQ ID NO: 18 is mutated from G as the base of the HvCslF6 gene of Sachiho Golden corresponding to the former base to A. The base of position 2,385 of SEQ ID NO: 18 corresponds to that of position 758 of SEQ ID NO: 19 (the base sequence of the cDNA of the β-glucan-deficient gene of KM27). This mutation was demonstrated to result in the mutation of the amino acid of position 253 of the amino acid sequence encoded by the β-glucan-deficient gene of KM27 from cysteine as the amino acid residue on HvCslF6 protein of Sachiho Golden, corresponding to the former amino acid to tyrosine. This mutation is different from the mutation identified in the β-glucan-deficient gene of each of OUM125 and Nishinohoshi (bgl).

The fact that two different mutants having mutations in HvCslF6 genes, having β-glucan deficiency were obtained is more strong evidence that the HvCslF6 gene is a β-glucan synthesis gene.

(Prediction of Three-Dimensional Structure of HvCslF6)

The three-dimensional structure of HvCslF6 protein was predicted using a software for predicting the three-dimensional structure of protein, SOSUI (freeware, http://bp.nuap.nagoya-u.ac.jp/sosui/). The results are shown in Figure 9. In Figure 9, the site in which cysteine is mutated to tyrosine in KM27 is indicated by (i), and the site in which glycine is mutated to aspartic acid in OUM125 is indicated by (ii). The positions of motif D and motif QxxRW are indicated by D and QxxRW, respectively.

(CAPS Analysis)

To perform the CAPS analysis of the same β-glucan-deficient gene as that of KM27, the following primers (CAPS marker) were prepared: 5'-ATCAAGGAGCCCATCCTCTC-3' (HvCSLF6_MPP2-1F, SEQ ID NO: 21) and 5'-TTGATCCTGGCCTTGAACTC-3' (HvCSLF6_MPP2-1R, SEQ ID NO: 22).

A Genomic DNA of a barley is subjected to PCR using these primers to provide an amplified fragment with 311 bp. For a wild-type variety, this fragment is cleaved into 6 fragments with 3, 41, 50, 56, 62 and 99 bp by digestion with the restriction enzyme Fnu4HI. In contrast, for a variety having the same β-glucan-deficient gene as that of KM27, digestion with Fnu4HI cleaves the above amplified fragment into 5 fragments with 3, 41, 50, 56 and 161 bp because the single base substitution eliminates one recognition site of Fnu4HI. The difference in these fragments produced by Fnu4HI digestion can be easily distinguished by electrophoresis or the like. The recognition sequence of Fnu4HI is 5'-GCNGC-3', which is cleaved into the form of 5'-GC/NGC-3' by Fnu4HI digestion. Here, N means A, T, G, or C. BisI, BsoFI, Fsp4HI, ItaI, SatI, and the like are known as restriction enzymes recognizing the same base sequence as that for Fnu4HI and cleaving the sequence into the same form as that for Fnu4HI. These restriction enzymes can be used in the same way as that for Fnu4HI.

Figure 10 shows the results of determining the presence of the same β-glucan-deficient gene as that of KM27 by the CAPS analysis. Genomic DNAs were each PCR amplified using the above primers, and the amplified fragment was cleaved with the restriction enzyme Fnu4HI and subjected to agarose gel electrophoresis. Lanes A to F are the results of analyzing the genomic DNA of Sachiho Golden (wild type), KM27 (β-glucan-deficient), F1 (hetero type) between Sachiho Golden and KM27, KM27 (β-glucan-deficient), Nishinohoshi (bgl) (β-glucan-deficient), and F 1 (hetero type) between Sachiho Golden and KM27.

Industrial Applicability

The use of the present invention enables the elucidation of a gene involved in the synthesis of β-glucan in a plant in the field of life science. If the gene involved in the synthesis of β-glucan is elucidated, it is expected that the results can be used to genetically control the content of β-glucan in breeding. The use thereof also enables the breeding of a barley variety for brewing/animal consumption containing no β-glucan in the agricultural field. Its application to a food using the functionality of arabinoxylan can also be carried out.

## Claims

**1.** A Genomic DNA of a β-glucan-deficient gene selected from the group consisting of the DNAs of (a) to (h):

(a) a DNA consisting of a base sequence having 90% or more homology to the base sequence of SEQ ID NO: 1 and producing a protein lacking a β-glucan synthesis activity when transcribed and translated;
(b) a DNA consisting of a base sequence having 90% or more homology to the base sequence of SEQ ID NO: 1 and producing a protein lacking a β-glucan synthesis activity when transcribed and translated, wherein the protein consists of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is an amino acid other than glycine;
(c) a DNA consisting of a base sequence having 90% or more homology to the base sequence of SEQ ID NO: 1 and producing a protein lacking a β-glucan synthesis activity when transcribed and translated, wherein the protein consists of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is aspartic acid; and
(d) a DNA consisting of the base sequence of SEQ ID NO: 1;
(e) a DNA consisting of a base sequence having 90% or more homology to the base sequence of SEQ ID NO: 18 and producing a protein lacking a β-glucan synthesis activity when transcribed and translated;
(f) a DNA consisting of a base sequence having 90% or more homology to the base sequence of SEQ ID NO: 18 and producing a protein lacking a β-glucan synthesis activity when transcribed and translated, wherein the protein consists of an amino acid sequence in which one or several amino acids are deleted, substituted, or added in the amino acid sequence of SEQ ID NO: 20, wherein the amino acid corresponding to position 253 of the amino acid sequence of SEQ ID NO: 20 is an amino acid other than cysteine;
(g) a DNA consisting of a base sequence having 90% or more homology to the base sequence of SEQ ID NO: 18 and producing a protein lacking a β-glucan synthesis activity when transcribed and translated, wherein the protein consists of an amino acid sequence in which one or several amino acids are deleted, substituted, or added in the amino acid sequence of SEQ ID NO: 20, wherein the amino acid corresponding to position 253 of the amino acid sequence of SEQ ID NO: 20 is tyrosine; and
(h) a DNA consisting of the base sequence of SEQ ID NO: 18.

**2.** A cDNA of a β-glucan-deficient gene selected from the group consisting of the DNAs of (a) to (f):

(a) a DNA encoding a protein lacking a β-glucan synthesis activity, wherein the protein consists of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is an amino acid other than glycine;
(b) a DNA encoding a protein lacking a β-glucan synthesis activity, wherein the protein consists of an amino acid sequence in which one or several amino acids are deleted, substituted, or added in the amino acid sequence of SEQ ID NO: 2, wherein the amino acid corresponding to position 660 of the amino acid sequence of SEQ ID NO: 2 is aspartic acid; and
(c) a DNA consisting of the base sequence of SEQ ID NO: 3;
(d) a DNA encoding a protein lacking a β-glucan synthesis activity, wherein the protein consists of an amino acid sequence in which one or several amino acids are deleted, substituted, or added in the amino acid sequence of SEQ ID NO: 20, wherein the amino acid corresponding to position 253 of the amino acid sequence of SEQ ID NO: 20 is an amino acid other than cysteine;
(e) a DNA encoding a protein lacking a β-glucan synthesis activity, wherein the protein consists of an amino acid sequence in which one or several amino acids are deleted, substituted, or added in the amino acid sequence of SEQ ID NO: 20, wherein the amino acid corresponding to position 253 of the amino acid sequence of SEQ ID NO: 2 is tyrosine; and
(f) a DNA consisting of the base sequence of SEQ ID NO: 19.

**3.** A Genomic DNA of a β-glucan synthesis gene selected from the group consisting of the DNAs of (a) to (d):

(a) a DNA consisting of a base sequence having 90% or more homology to the base sequence of SEQ ID NO: 4 and producing a protein having a β-glucan synthesis activity when transcribed and translated;
(b) a DNA consisting of the base sequence of SEQ ID NO: 4;
(c) a DNA consisting of a base sequence having 90% or more homology to the base sequence of SEQ ID NO: 15 and producing a protein having a β-glucan synthesis activity when transcribed and translated; and
(d) a DNA consisting of the base sequence of SEQ ID NO: 15.

**4.** A cDNA of a β-glucan synthesis gene consisting of (a) a DNA consisting of a base sequence having 90% or more homology to the base sequence of SEQ ID NO: 6 and encoding a protein having a β-glucan synthesis activity or (b) a DNA consisting of the base sequence of SEQ ID NO: 6.

**5.** A method for breeding a barley having a genomic DNA of a β-glucan-deficient gene, comprising:

a selection step of selecting the barley by determining the barley as having a genomic DNA of a β-glucan-deficient gene when the base corresponding to position 4,275 of the genomic DNA consisting of the base sequence of SEQ ID NO: 4 located near the centromere of chromosome 7H of the barley is mutated from G to A; or
a selection step of selecting the barley by determining the barley as having a genomic DNA of a β-glucan-deficient gene when the base corresponding to position 2,385 of the genomic DNA consisting of the base sequence of SEQ ID NO: 4 located near the centromere of chromosome 7H of the barley is mutated from G to A.

**6.** A method for breeding a barley having a genomic DNA of a β-glucan-deficient gene, comprising:

an amplification step of amplifying a DNA fragment containing a base corresponding to position 4,275 of the base sequence of SEQ ID NO: 4, using a genomic DNA extracted from a barley as a template;
a detection step of cleaving the DNA fragment amplified in the amplification step with a restriction enzyme selected from the group consisting of TaqI, BanI, and NlaIV and detecting the cleaved DNA fragment; and
a selection step of selecting the barley by determining the barley as having a genomic DNA of a β-glucan-deficient gene when the DNA fragment is cleaved with the restriction enzyme TaqI or not cleaved with the restriction enzyme BanI or NlaIV in the detection step.

**7.** A method for breeding a barley having a genomic DNA of a β-glucan-deficient gene, comprising:

an amplification step of amplifying a DNA fragment containing a base corresponding to position 2,385 of the base sequence of SEQ ID NO: 4, using a genomic DNA extracted from a barley as a template;
a detection step of cleaving the DNA fragment amplified in the amplification step with a restriction enzyme Fnu4HI and detecting the cleaved DNA fragment; and
a selection step of selecting the barley by determining the barley as having a genomic DNA of a β-glucan-deficient gene when the DNA fragment is not cleaved between the bases corresponding to positions 2,386 and 2,387 of SEQ ID NO: 4 in the detection step.

**8.** A method for decreasing or abolishing β-glucan in barley kernels, comprising a step of suppressing the expression of the genomic DNA of a β-glucan synthesis gene according to claim 3.

**9.** A barley decreasing or deficient in β-glucan in kernels obtainable by the method according to claim 8.

**10.** A barley having the genomic DNA of a β-glucan-deficient gene according to claim 1 and bred by the breeding method according to any one of claims 5 to 7.

**11.** A barley having the genomic DNA of a β-glucan-deficient gene according to claim 1 in a homozygous form and bred by the breeding method according to any one of claims 5 to 7.

**12.** A transformant comprising a vector containing the genomic DNA of a β-glucan synthesis gene according to claim 3 so as to be expressible.

**13.** A transformant comprising a vector containing the cDNA of a β-glucan synthesis gene according to claim 4 so as to be expressible.

**14.** A method for producing an alcohol, comprising a step of fermenting kernels derived from the barley according to any one of claims 9 to 11.

**15.** A method for producing a fermented food, comprising a step of fermenting kernels derived from the barley according to any one of claims 9 to 11.

**16.** An animal feed composition comprising kernels derived from the barley according to any one of claims 9 to 11.

Fig.1

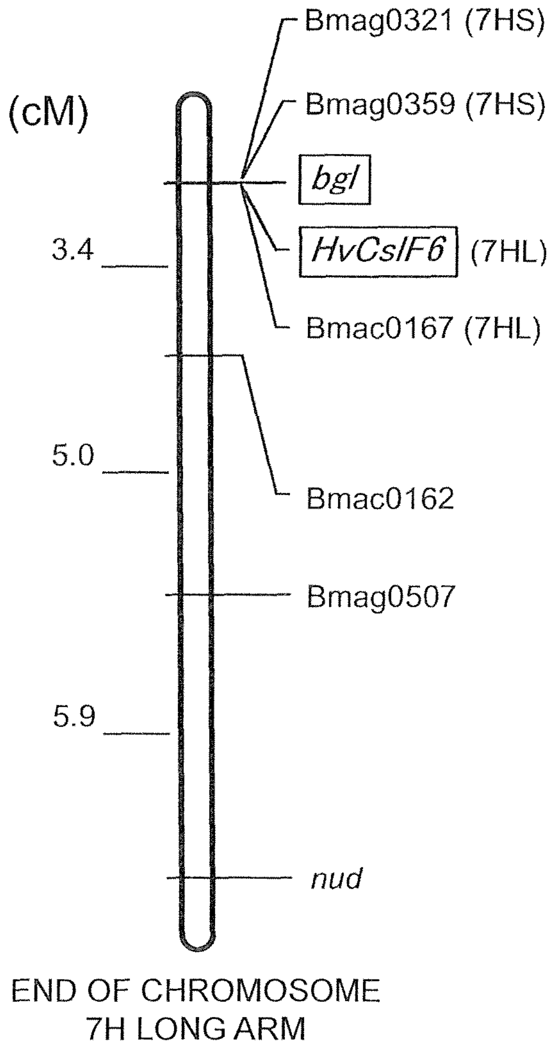
(cM)
3.4
5.0
5.9
Bmag0321 (7HS)
Bmag0359 (7HS)
bgl
HvCslF6 (7HL)
Bmac0167 (7HL)
Bmac0162
Bmag0507
nud
END OF CHROMOSOME
7H LONG ARM

*Fig.2*

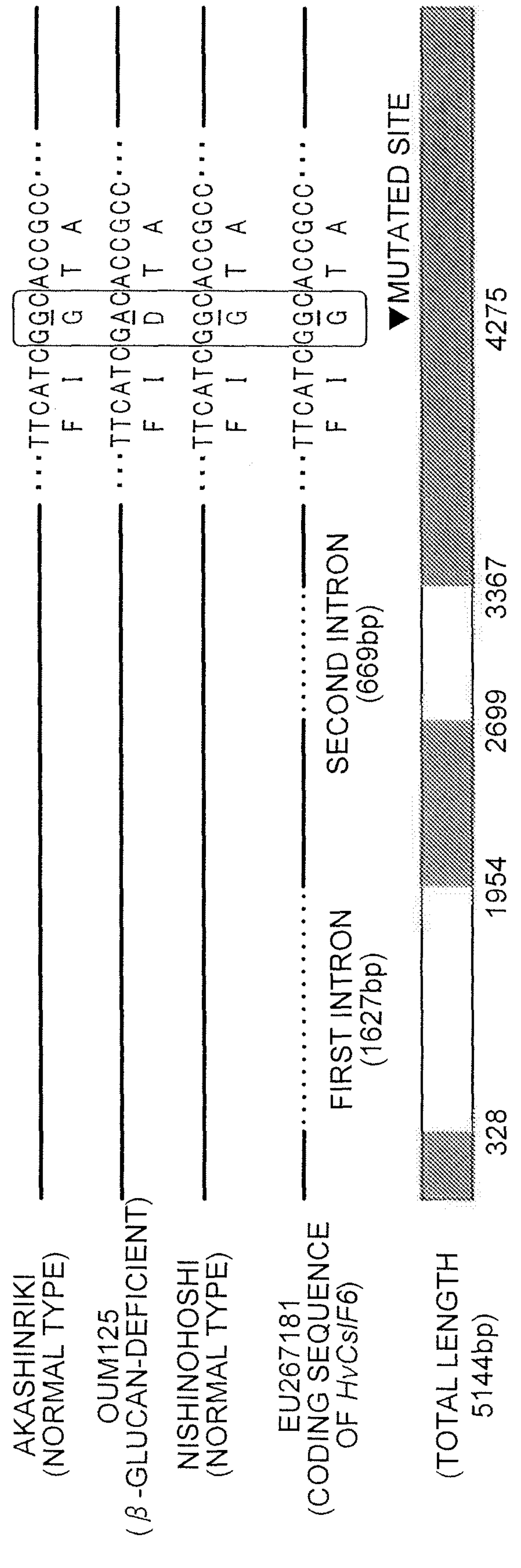
AKASHINRIKI (NORMAL TYPE)
OUM125 (β-GLUCAN-DEFICIENT)
NISHINOHOSHI (NORMAL TYPE)
EU267181 (CODING SEQUENCE OF *HvCslF6*)
(TOTAL LENGTH 5144bp)
FIRST INTRON (1627bp)
SECOND INTRON (669bp)
...TTCATCGGCACCGCC...
F I G T A
...TTCATCGACACCGCC...
F I D T A
...TTCATCGGCACCGCC...
F I G T A
...TTCATCGGCACCGCC...
F I G T A
▼MUTATED SITE
328
1954
2699
3367
4275

*Fig.3*

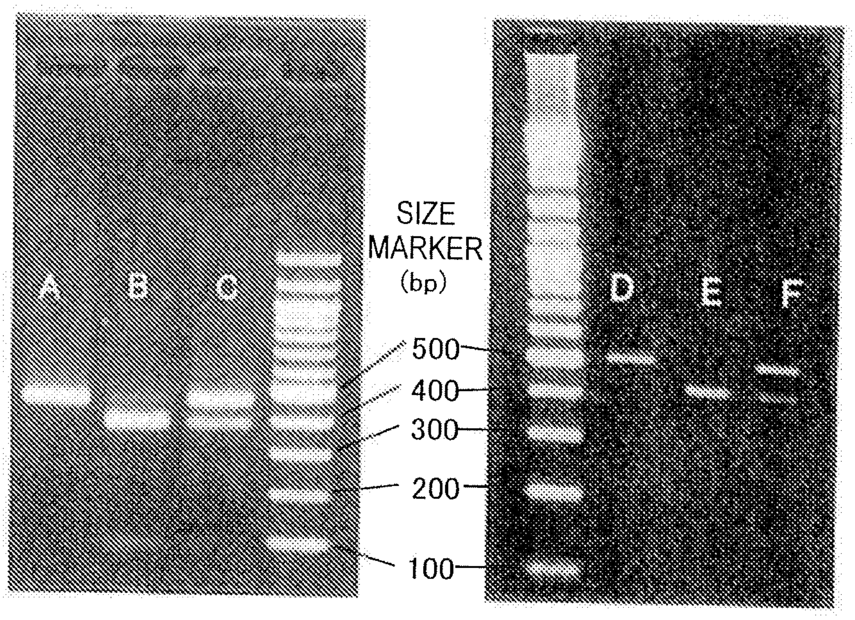

# Fig.4

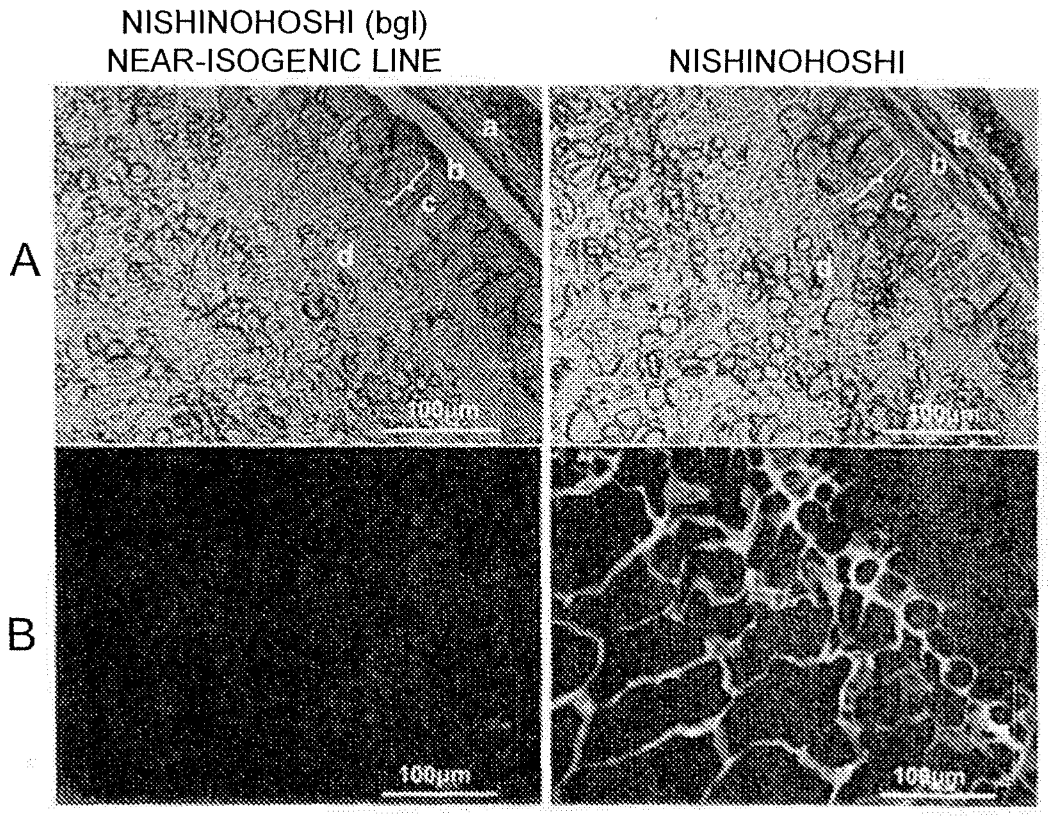
NISHINOHOSHI (bgl)
NEAR-ISOGENIC LINE
NISHINOHOSHI
A
B
a
b
c
d
100μm

***Fig.5***

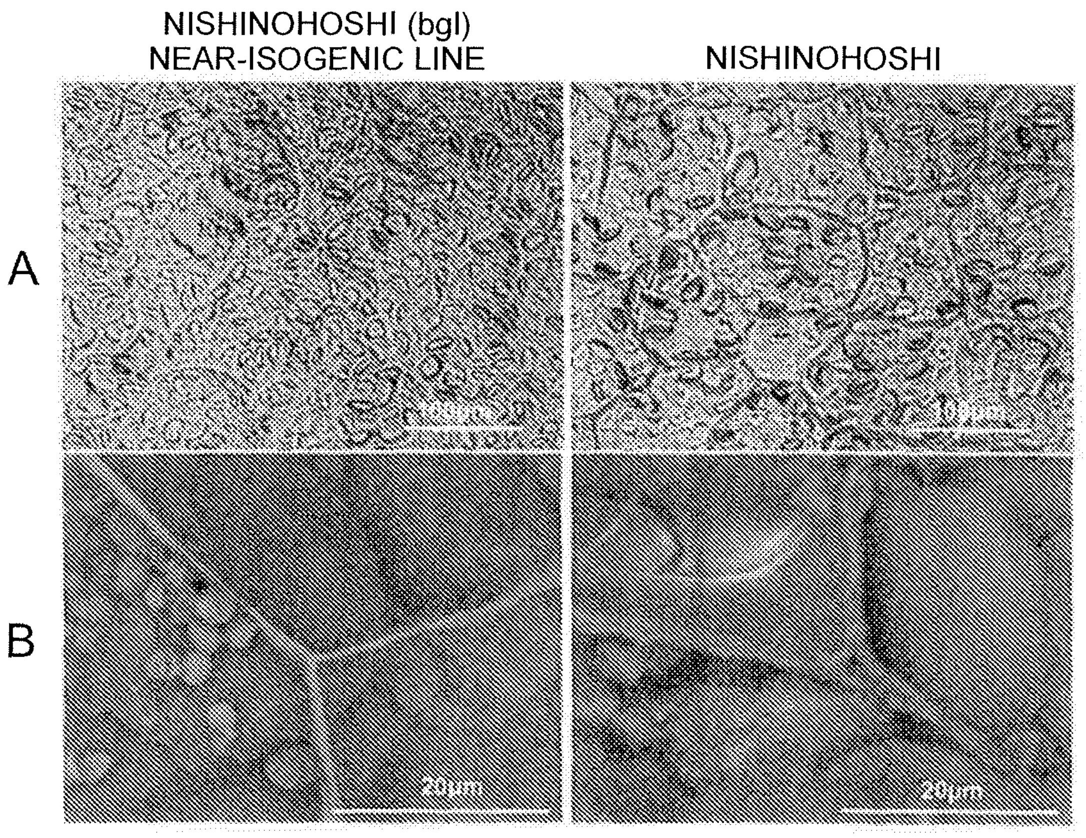
NISHINOHOSHI (bgl)
NEAR-ISOGENIC LINE
NISHINOHOSHI
A
B
100μm
100μm
20μm
20μm

# Fig.6

```
TR251     (SEQ ID NO:13)     1:ATCTTGTTTTTGCGGGACAGCTTACGAATTACTTTTTATTTTGCGGTACAGCTTACGAATTACTTAACTTGATGGAGCAGTTCAGCAAAGAAATTGAGCA 100
CDC-Bold  (SEQ ID NO:14)     1:ATCTTGTTTATGCGGGACAGCTTACGAATTACTTTTTATTTTGCAGTACAGCTTACGAATTACTTAACTTGATGGAGCAGTTCAGCAAAGAAATTGAGCA 100

TR251     (SEQ ID NO:13)   101:AAAATCATTTGCAGAGTCAAGGGGTGAGCTTGTGGGCGTCAATCAGGTGATTCGTCTCCTGTCCACACATAGACGCGCGCGCACACATAAGCAAGTGAGT 200
CDC-Bold  (SEQ ID NO:14)   101:AAAATCATTTGCAGAGTCAAGGGGTGAGCTTGTGGGCGTCAATCAGGTGATTCGTCTCCTGTCCACACATAGACGCGCGCGCACACATAAGCAAGTGAGT 200

TR251     (SEQ ID NO:13)   201:AGTAGGTGATTACATTACCGTCGGCGTGAAGCGTTAAAATTGCGTCCGTTCTCTCCCGCCTCCTCGTAAATGCTTTGGGACTCGTTGATTGTAGCAGTGG 300
CDC-Bold  (SEQ ID NO:14)   201:AGTAGGTGATTACATTACCGTCGGCGTGAAGCGTTAAAATTGCGTCCGTTCTCTCCCGCCTCCTCGTAAATGCTTTGGGACTCGTTGATTGTAGCAGTGG 300

TR251     (SEQ ID NO:13)   301:TAGTTTATCATGAATGCTGGGGCTAGCGTGCCCGCATATGTAGTTGGATCGTCGATGCAACAGCAAGGCCGGTTAATTACTCCTCCGGCCTCTCCATACC 400
CDC-Bold  (SEQ ID NO:14)   301:TAGTTTATCATGAATGCTGGGGCTAGCGTGCCCGCATATGTAGTTGGATCGTCGATGCAACAGCAAGGCCGGTTAATTACTCCTCCGGCCTCTCCATACC 400

TR251     (SEQ ID NO:13)   401:AGTGCACGGCTCGCCACGATCGTTTTCTTGTGGTATGTTGTCCTTTTGTGTGTTGTGTGGCGGTGTGGTTATAGTTGGAGGTCGTGACAGAACCCATTAG 500
CDC-Bold  (SEQ ID NO:14)   401:AGTGCACGGCTCGCCACGATCGTTTTCTTGTGGTATGTTGTCCTTTTGTGTGTTGTGTGGCGGTGTGGTTATAGTTGGAGGTCGTGACAGAACCCATTAG 500

TR251     (SEQ ID NO:13)   501:TTGTCCGGCGCGCGCGCGTCATTGTCCTAACAACTGGCCTAATTTGCAAATTGCTCTAGACCTCATGCTTGATTCCTCCCGTTAGTTCAGGGAGCTCCAT 600
CDC-Bold  (SEQ ID NO:14)   501:TTGTCCGGCGCGCGCGCGTCATTGTCCTAACAACTGGCCTAATTTGCAAATTGCTCTAGACCTCATGCTTGATTCCTCCCGTTAGTTCAGGGAGCTCCAT 600

TR251     (SEQ ID NO:13)   601:CAGCACGGATTACATGCTCCTCCCCGTCCCCGCCGTGAATCTCTCATCGGATACGCGCGCGACGTTCCCCGCCGTGACTCTCTCAATCTCTCATCGGATA 700
CDC-Bold  (SEQ ID NO:14)   601:CAGCACGGATTACGTGCTCCTCCCCGTCCCCGCCGTGAATCTCTCATCGGATACGCGCGCGACGTTCCCCGCCGTGAC--------TCTCTCATCGGATA 692

TR251     (SEQ ID NO:13)   701:CGCGCGGGACGTTCCTATAGGGACGTCGTAACGGGCAGAAGGGCAAGCCTCCTTCCGGCCAGGGGACCAATGCATTCCTTCTCGTGAAGCATTTGCTCGC 800
CDC-Bold  (SEQ ID NO:14)   693:CGCGCGGGACGTTCCTATAGGGACGTCGTAACGGGCAGAAGGGCAAGCCTCCTTCCGGCCAGGGGACCAATGCATTCCTTCTCGTGAAGCATTTGCTCGC 792

TR251     (SEQ ID NO:13)   801:CAAAGCCAAAGCCAAAGCCAAAGCCACAAGGGGAAAAAGAGAGAGAAGGAGGCGGTAGAACCGGAAAGGGCTCTGCATGTCTACCTTTCGCTGTGACCAA 900
CDC-Bold  (SEQ ID NO:14)   793:CAAAGCCAAAGCCAAAGCCAAAGCCACAAGGGGAAAAAGAGAGAGAAGGAGGCGGTAGAACCGGAAAGGGCTCTGCATGTCTACCTTTCGCTGTGACCAA 892

TR251     (SEQ ID NO:13)   901:AATTACTCTTCCCTTCCGTGGCCCTGCTGCATGAGCATGGCCCTCCGCTTTTCAACCAGCAAGACAAGAATATGTCGCAATTGCTCCTACTAGTGTTAAA 1000
CDC-Bold  (SEQ ID NO:14)   893:AATTACTCTTCCCTTCCGTGGCCCTGCTGCATGAGCATGGCCCTCCGCTTTTCAACCAGCAAGACAAGAATATGTCGCAATTGCTCCTACTAGTGTTAAA 992

TR251     (SEQ ID NO:13)  1001:CCGATGGATCAATCCTCCAAAATGCATCCCCCAACCAGACCCAACTTGCGCATAAACATCAATGTGCTAAAAATTCCATGGACAGAAGAGAGCGCCGCAT 1100
CDC-Bold  (SEQ ID NO:14)   993:CCGATGGATCAATCCTCCAAAATGCATCCCCCAACCAGACCCAACTTGCGCATAAACATCAATGTGCTAAAAATTCCATGGACAGAAGAGAGCGCCGCAT 1092
```

# Fig.7

```
TR251     (SEQ ID NO:13)  1101:CGCATTTGCCGGTGGGCTGCAAGGCCACAGCGGAGCAAAGAGGCTTGTACGGTACTCAAACCAGCTCACTGATGCATGGCGATGCTACAGGGACTCACGG 1200
CDC-Bold  (SEQ ID NO:14)  1093:CGCATTTGCCGGTGGGCTGCAAGGCCACAGCGGAGCAAAGAGGCTTGTACGGTACTCAAACCAGCTCACTGATGCATGGCGATGCTACAGGGACTCACGG 1192

TR251     (SEQ ID NO:13)  1201:GCCACGCTGGCTGGGAGAGAGCAGCAGAAAAGGCGCAAGAAAAGAATCTTTCTCTCAATCAAGGGCAACAACCAAGCACAAGCTCACAAACCCCCCACCA 1300
CDC-Bold  (SEQ ID NO:14)  1193:GCCACGCTGGCTGGGAGAGAGCAGCAGAAAAGGCGCAAGAAAAGAATCTTTCTCTCAATCAAGGGCAACAACCAAGCACAAGCTCACAAACCCCCCACCA 1292

TR251     (SEQ ID NO:13)  1301:AGTTAAGAAAAAGAAAAAAAAATACAGGCACTTCGCATAACAAACAAACTAATCATCATCACCACTCATCGCCCCCGGCACCTTTATTCTACGCCCAGCT 1400
CDC-Bold  (SEQ ID NO:14)  1293:AGTTAAGAAAAAGAAAAAAAAATACAGGCACTTCGCATAACAAACAAACTAATCATCATCACCACTCATCGCCCCCGGCACCTTTATTCTACGCCCAGCT 1392

TR251     (SEQ ID NO:13)  1401:CCTCTCTCATTACACTTCTAACATATATAACAATCAATCGGTGGAGGAGGAGGGACCAATTAATTAATGGATTAGTAATAACAATTCTTATGCGAGCAAA 1500
CDC-Bold  (SEQ ID NO:14)  1393:CCTCTCTCATTACACTTCTAACATATATAACAATCAATCGGTGGAGGAGGAGGGACCAATTAATTAATGGATTAGTAATAACAATTCTTATGCGAGCAAA 1492

TR251     (SEQ ID NO:13)  1501:AATATACGTACTCCTGTACTAATAAGAATCGGAAATGCAAGCTAATCAGAGCTACTGCGTACGTACTAGTTTTCTTGCGCGGGGAGGGCATCACAAATCA 1600
CDC-Bold  (SEQ ID NO:14)  1493:AATATACGTACTCCTGTACTAATAAGAATCGGAAATGCAAGCTAATCAGAGCTACTGCGTACGTACTAGTTTTCTTGCGCGGGGAGGGCATCACAAATCA 1592

TR251     (SEQ ID NO:13)  1601:CATGGGACACGCAGGGGCAGGAATATGAGTGGAAGCAACCTGGTAGGTAGGTGAGGTGCGGTGCGGTGCGGGCATATGATAAGCATATGGGCTGGGCCGA 1700
CDC-Bold  (SEQ ID NO:14)  1593:CATGGGACACGCAGGGGCAGGAATATGAGTGGAAGCAACCTGGTAGGTAGGTGAGGTGCGGTGCGGTGCGGGCATATGATAAGCATATGGGCTGGGCCGA 1692

TR251     (SEQ ID NO:13)  1701:GCATACACGCGTACATGCATTGCATTTGCATACACCGAAGGAAGTGCTTGGCACCACCTACATATAAACGCACCCGCACACCGCTCAAACACAATTTACA 1800
CDC-Bold  (SEQ ID NO:14)  1693:GCATACACGCGTACATGCATTGCATTTGCATACACCGAAGGAAGTGCTTGGCACCACCTACATATAAACGCACCCGCACACCGCTCAAACACAATTTACA 1792

TR251     (SEQ ID NO:13)  1801:ACCCGCACACAGCTCCAACCCGTTGGTTGGATAGCTCAGCAAGACAAGCGAGCTGAGCAGAGCTTTGCTTTGCTTGAACCCCCTACCGACACTTCTTGTG 1900
CDC-Bold  (SEQ ID NO:14)  1793:ACCCGCACACAGCTCCAACCCGTTGGTTGGATAGCTCAGCAAGACAAGCGAGCTGAGCAGAGCTTTGCTTTGCTTGAACCCCCTACCGACACTTCTTGTG 1892

TR251     (SEQ ID NO:13)  1901:CATCCCAGCCCATAAATCCCCCACGTACTTTACTCGTCATTTCTCGCACCTCCTCCTCCCCCTCCCCCTCGCCTACATTCATTCATTGCTTCCTTTTCTC 2000
CDC-Bold  (SEQ ID NO:14)  1893:CATCCCAGCCCATAAATCCCCCACGTACTTTACTCGTCATTTCTCGCACCTCCTCCTCCCCCTCCCCCTCGCCTACATTCATTCATTGCTTCCTTTTCTC 1992

TR251     (SEQ ID NO:13)  2001:TCTCCCTGCACATGCTAAAGCCTGCCTCGGCCATTGCCTGAGCCTGCCATTGTTGGACCTTGGACCTGTTCTCCTTGTCGTAAAGGCAAAGGAGAGCGCG 2100
CDC-Bold  (SEQ ID NO:14)  1993:TCTCCCTGCCCATGCTAAAGCCTGCCTCGGCCATTGCCTGAGCCTGCCATTGTTGGACCTTGGACCTGTTCTCCTTGTCGTAAAGGCAAAGGAGAGCGCG 2092

TR251     (SEQ ID NO:13)  2101:TGCATTGAGGACGACGGCCATGGCGCCAGCGGTGGCCGGAGGGGGCCGCGTGCGGAGCAATGAGCCGGTTGCTGCTGCTGCCGCCGCGCCGGCGGCCAGC 2200
CDC-Bold  (SEQ ID NO:14)  2093:TGCATTGAGGACGACGGCCATGGCGCCAGCGGTGGCCGGAGGGGGCCGCGTGCGGAGCAATGAGCCGGTTGCTGCTGCTGCCGCCGCGCCGGCGGCCAGC 2192

TR251     (SEQ ID NO:13)  2201:GGCAAGCCCTGCGTGTGCGGCTTCCAGGTTTGCGCCTGCACGGGGTCGGCCGCGGTGGCCTCCGCCGCCTCGTCGCTGG---------------------- 2279
CDC-Bold  (SEQ ID NO:14)  2193:GGCAAGCCCTGCGTGTGCGGCTTCCAGGTTTGCGCCTGCACGGGGTCGGCCGCGGTGGCCTCCGCCGCCTCGTCGCTGG---------------------- 2271
```

Fig.8

2385 (758)

| | | | |
|---|---|---|---|
| TN5-DNA | 2372: | TGGGTGCCCTTCTGCCGCAAGCACGGG | 2398 |
| TN5-cDNA | 745: | TGGGTGCCCTTCTGCCGCAAGCACGGG | 771 |
| TN5-a.a. | 249: | W V P F Cys R K H G | 257 |
| KM27-DNA | 2372: | TGGGTGCCCTTCTACCGCAAGCACGGG | 2398 |
| KM27-cDNA | 745: | TGGGTGCCCTTCTACCGCAAGCACGGG | 771 |
| KM27-a.a. | 249: | W V P F Tyr R K H G | 257 |

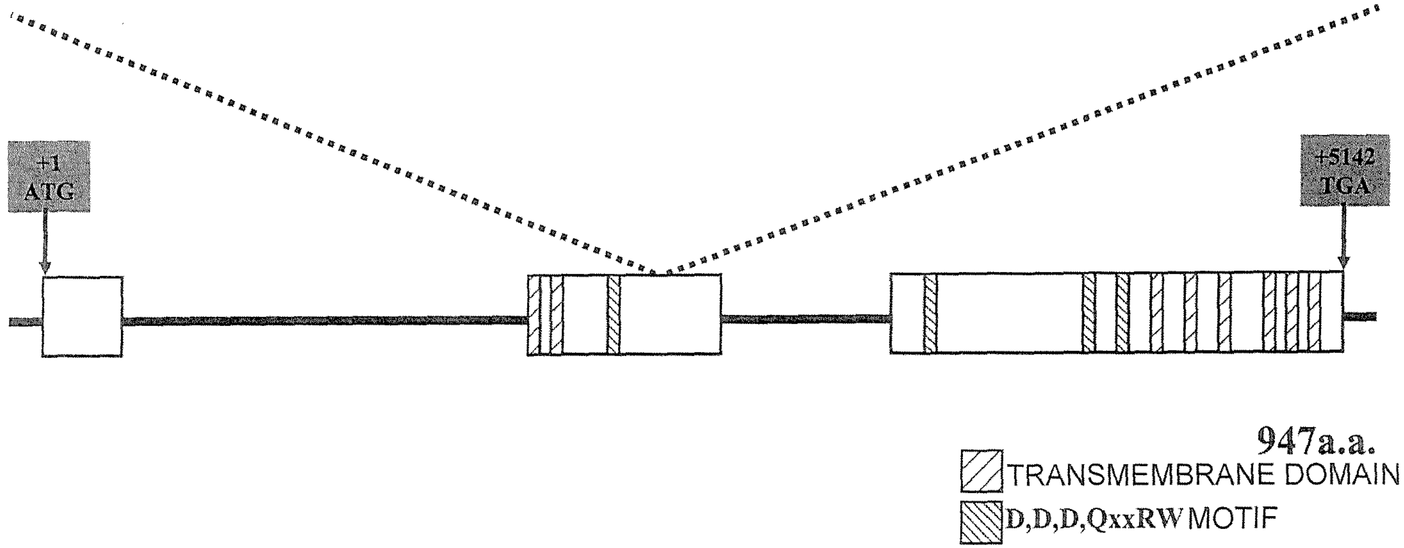

Fig.9

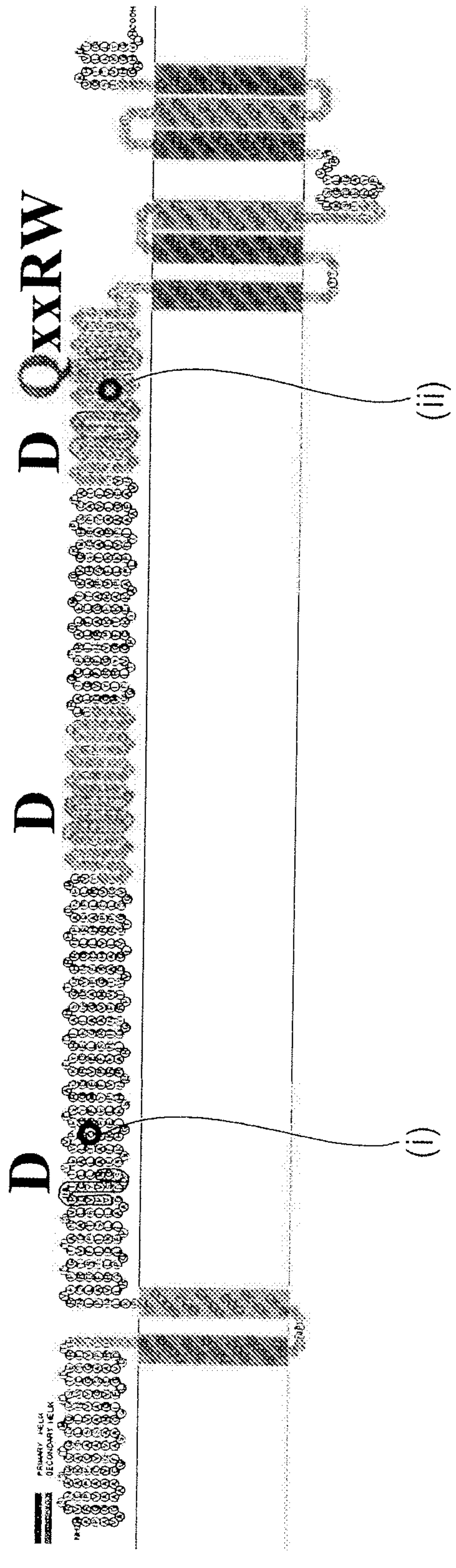

*Fig.10*

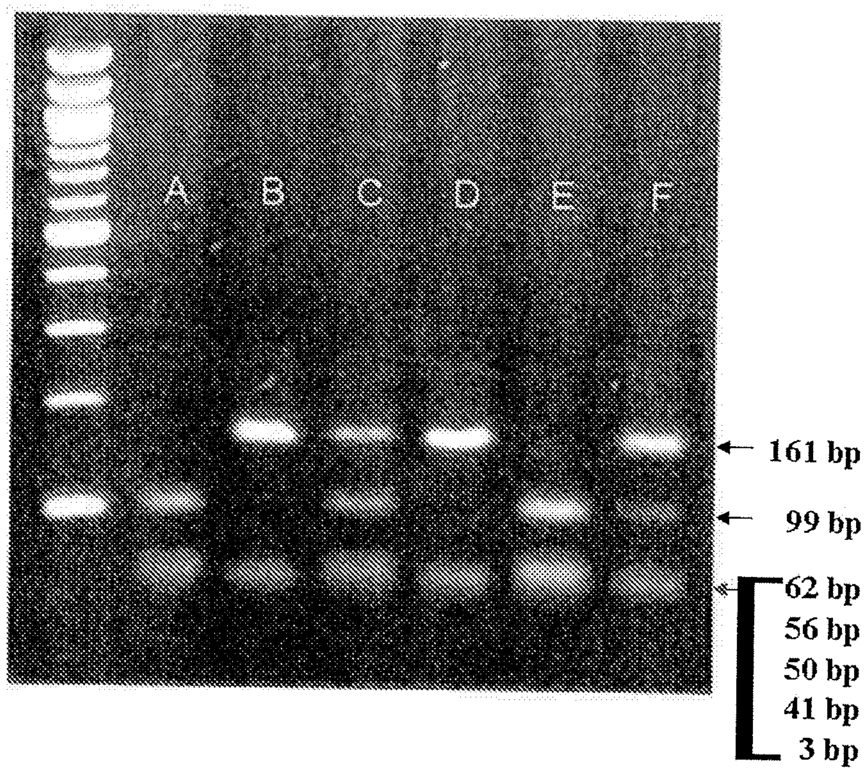

**INTERNATIONAL SEARCH REPORT**

International application No. PCT/JP2010/052939

A. CLASSIFICATION OF SUBJECT MATTER
*C12N15/09*(2006.01)i, *A01H1/00*(2006.01)i, *A23K1/14*(2006.01)i, *A23L1/30*(2006.01)i, *C12C11/00*(2006.01)i, *C12G3/12*(2006.01)i, *C12N1/15*(2006.01)i, *C12N1/19*(2006.01)i, *C12N1/21*(2006.01)i, *C12N5/10*(2006.01)i

According to International Patent Classification (IPC) or to both national classification and IPC

B. FIELDS SEARCHED

Minimum documentation searched (classification system followed by classification symbols)
C12N15/09, A01H1/00, A23K1/14, A23L1/30, C12C11/00, C12G3/12, C12N1/15, C12N1/19, C12N1/21, C12N5/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/BIOSIS/MEDLINE/WPIDS(STN), JSTPlus/JMEDPlus/JST7580(JDreamII), GenBank/EMBL/DDBJ/GeneSeq, UniProt/GeneSeq, PubMed, CiNii

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2007/014433 A1 (GRAINS RESEARCH & DEVELOPMENT CORP.), 08 February 2007 (08.02.2007), SUMMARY OF THE INVENTION; table 1; claims & JP 2009-502180 A & US 2008/0307549 A1 & EP 1920057 A | 3,4,8,9,12,13<br>1,2,10,11,14-16<br>5-7 |
| X<br>Y<br>A | Takuji TONOOKA et al., "Omugi no β-glucan Kesshitsu Hen'i no Iden Yoshiki to Hainyu Saiboheki Kozo", [online] Heisei 19 Nendo 'Kanto Tokai Hokuriku Nogyo' Kenkyu Seika Joho, 28 August 2008 (28.08.2008), <URL> http://ss.inada.affrc.go.jp/chousei/shiryou/kankou/seika/kanto19/10/19_10_40.html, [retrieval date 20 April 2010 (20.04.2010)], entire text | 10,11,14-16<br>1,2,10,11,14-16<br>3-9,12,13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 April, 2010 (21.04.10) | 11 May, 2010 (11.05.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/052939

C (Continuation). DOCUMENTS CONSIDERED TO BE RELEVANT

| Category* | Citation of document, with indication, where appropriate, of the relevant passages | Relevant to claim No. |
|---|---|---|
| P,X | TONOOKA, T., et al., A novel mutant gene for (1-3,1-4)-β-D-glucanless grain on barley (Hordeum vulgare L.) chromosome 7H, Breeding Science, 2009.03, Vol. 59, No. 1, pp.47-54 | 1-16 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Brennan, C.S. ; L.J. Cleary.** *J. Cereal Sci.,* 2005, vol. 42, 1-13
- **Dr. Kazuhiro Sato.** *Barley and Wild Plant Resource Center*
- **Allard.** *Hilgardia,* 1956, vol. 24, 235-278
- **Ramsay et al.** *Genetics,* 2000, vol. 156, 1997-2005
- Proc. 6th Int. Wheat Genet. Symp. Maruzen Kyoto, 1983, 233-238
- **Lander et al.** *Genomics,* 1987, vol. 1, 174-181
- **Kosambi.** *Ann. Eugen.,* 1944, vol. 12, 172-175
- *Journal of Cereal Science,* 2008, vol. 48, 647-655

SEQUENCE LISTING

<110> National Agriculture and Food Research Organization
Okayama University

<120> Barley beta-glucanless gene, beta-glucan synthase gene and use thereof

<130> 10-0056-00

<150> JP2009-045393
<151> 2009-02-27

<160> 28

<170> PatentIn version 3.5

<210> 1
<211> 5140
<212> DNA
<213> Hordeum vulgare

<220>
<221> misc_feature
<223> OUM125 bgl gene genomic DNA

<400> 1
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccggtac gtcctgctcc cacaactaaa cagaaactcc 360

ctatatctgc gtcacactca acaattaatc caactaagtc tctctactac tctagtattt 420

atttttactc tctatctgca caacaagcgc tactacaatt aacccaacaa gcaccacgcc 480

aggttgacag tcaggataat ttgatcttga ccggagtaag tactagtact aggtcggtgt 540

taatcagagt aattattgca ctagttaatt aaaatttgag taatccgaga caggtgcacg 600

ttagggccgg gccaatgatg gctcgaatcc acccaaaata gcgcgtcccg gtgtgggctg 660

tcggctcggt gcttcttcct tccattttac tagtcgcagt cactgcagct tgggcccacg 720

ggaggggacg ttagccgttg ggcctgcctg gcaggtgggc cccggtggcc accctggcgg 780

ctcataaatc cttgctactt tggagctgta atggacgctc tgcaatagca ataggaatcc 840

gaggtgaaac gacgacagtg ggcatggcat ggcttgcatg tgaatccaag ccacatcatt 900

aaaagcatcc tccctgggca cgtcgcggtg agaaagttgg ataaactttt gggggttcgg 960

acaagatgag aaaaagcaag taacatgtcc ctttttggc accgaagaaa tcctatgtac 1020

ggcgagtttt ttctgcatct agttatgggt agatgtacgt tagtttttgt tgagcgtttt 1080

atgtgctaca tatatggaga aaaagagaaa aatattatca tgtcatgtca tgccatgcca 1140

tgagaaggag gagaagaaaa agaagtgttg ccaccgctcg aatgcctttt ttttctttcg 1200
gaaggatgcg tgagtcatgt tggcaccgag aaaagccata ttaaagtggc agagttacaa 1260
ctcagaataa atgcgggtgt tacaaaaaca ctaggatatg tgaagggcac tcggcacaac 1320
actttaagac tacacaattg aaaaagtgta ctctctgtat ctaaataatt ataattgaaa 1380
agaattaatc tatatatgaa agtagtaatg attagcggta gaaggttcca acgacttttt 1440
tggcgccaat agcaagaaga aagaaaaaga aaaaaatctt tactgtacag tataacgaga 1500
aaagaggccc attaatagag caacgaatcg agcggcacca cctctggcgg tcacgtccat 1560
gccctcgcac gacggatgag gcccgggggg tcctactgac agccgaagca tgtcggtgct 1620
caaacacggc gccgtttgct gccaagtgtg ccagctcgca ctcattgact tgccagctct 1680
ctccttggtt gtcaatgaga acatgatgcc ttttggcatt tgcaaactta ttaaaactag 1740
ctgtcgtccg atagggaaaa gaaaagaaaa gaaaagaata agaaaaaaag gacaaagaga 1800
aaagatgaac atggcgcatg ttccctccaa taattgcagg caccaacact gggtcgatta 1860
atccaacaac aatattttac tataccagac gagagtacag tagtcgggtg atgatggact 1920
gtaactgact gagtatgaat gactgtaatg cagggtgctg attttcgttc gtctgatcgc 1980
cttcacgctg ttcgtgatct ggcgtatctc ccacaagaac ccagacgcga tgtggctgtg 2040
ggtgacatcc atctgcggcg agttctggtt cggtttctcg tggctgctgg atcagctgcc 2100
caagctgaac ccccatcaacc gcgtgccgga cctggcggtg ctgcggcagc gcttcgaccg 2160
ccccgacggc acctccacgc tcccggggct ggacatcttc gtcaccacgg ccgaccccat 2220
caaggagccc atcctctcca ccgccaactc ggtgctctcc atcctggccg ccgactaccc 2280
cgtggaccgc aacacatgct acgtctccga cgacagtggc atgctgctca cctacgaggc 2340
cctggcagag tcctccaagt tcgccacgct ctgggtgccc ttctgccgca agcacgggat 2400
cgagcccagg ggtccggaga gctacttcga gctcaagtca caccccttaca tggggagagc 2460
ccaggacgag ttcgtcaacg accgccgccg cgttcgcaag gagtacgacg agttcaaggc 2520
caggatcaac agcctggagc atgacatcaa gcagcgcaac gacgggtaca acgccgccat 2580
tgcccacagc caaggcgtgc ccccggcccac ctggatggcg gacggcaccc agtgggaggg 2640
cacatgggtc gacgcctccg agaaccaccg caggggcgac cacgccggca tcgtactggt 2700
cagtatccat ccatctttct gctgcttata ttactcttag gttactctta tcgtctcttt 2760
cctatactgt acatgcatgc atgctgctat tcttggaatc gtggttggtt actactccac 2820
catgcaaaaa taacaagaag aggaatcttg gttagttagg gcctcgttgt tatattagtg 2880
gccatctgat gtgatgcctg ccggctgtgc ccatccatat ccatggaaga tttcgacaga 2940
atcgacgtgg tgatagtcga gagtgcaacc accacccaga gccagccaag cacatgcatg 3000
cttctcttct cgtctcgtcg tgtggccagc agcgcattca tgctattgct gtgacgaggg 3060

aggaatggta gttggggtgg tcctttcccc ccgacagcac tacagcctcc actttatgac 3120
ccatttaatt caccggccct gctttgttgt aaccgccttc tcatctcaat caatcattca 3180
ttcattcata agtttactca ctctttgtta ctactcgaac cactaatcag gaaggagtag 3240
gagtaatgca gatttactat tgacagttaa aggagtaaaa agaaggaagc acaattacag 3300
aaccttgttt ttttttacta ctgtacgtaa ggtgtaagaa tggagtgctg acagagaatg 3360
gatgcaggtg ctgctgaacc acccgagcca ccgccggcag acgggcccgc cggcgagcgc 3420
tgacaaccca ctggacttga gcggcgtgga tgtgcgtctc cccatgctgg tgtacgtgtc 3480
ccgtgagaag cgccccgggc acgaccacca gaagaaggcc ggtgccatga acgcgcttac 3540
ccgcgcctcg gcgctgctct ccaactcccc cttcatcctc aacctcgact gcgatcatta 3600
catcaacaac tcccaggccc ttcgcgccgg catctgcttc atggtgggac gggacagcga 3660
cacggttgcc ttcgtccagt tcccgcagcg cttcgagggc gtcgacccca ccgacctcta 3720
cgccaaccac aaccgcatct tcttcgacgg caccctccgt gccctggacg gcatgcaggg 3780
ccccatctac gtcggcactg ggtgtctctt ccgccgcatc accgtctacg gcttcgaccc 3840
gccgaggatc aacgtcggcg gtccctgctt ccccaggctc gccgggctct tcgccaagac 3900
caagtacgag aagcccgggc tcgagatgac cacggccaag gccaaggccg cgccccgtgcc 3960
cgccaagggt aagcacggct tcttgccact gcccaagaag acgtacggca agtcggacgc 4020
cttcgtggac accatcccgc gcgcgtcgca cccgtcgccc tacgccgcgg cggctgaggg 4080
gatcgtggcc gacgaggcga ccatcgtcga ggcggtgaac gtgacggccg ccgcgttcga 4140
gaagaagacc ggctggggca aagagatcgg ctgggtgtac gacaccgtca cggaggacgt 4200
ggtcaccggc taccggatgc atatcaaggg gtggcggtca cgctactgct ccatctaccc 4260
acacgccttc atcgacaccg cccccatcaa cctcacggag aggctcttcc aggtgctccg 4320
ctggtccacg ggatccctcg agatcttctt ctccaagaac aacccgctct tcggcagcac 4380
atacctccac ccgctgcagc gcgtcgccta catcaacatc accacttacc ccttcaccgc 4440
catcttcctc atcttctaca ccaccgtgcc ggcgctatcc ttcgtcaccg gccacttcat 4500
cgtgcagcgc ccgaccacca tgttctacgt ctacctgggc atcgtgctat ccacgctgct 4560
cgtcatcgcc gtgctggagg tcaagtgggc cggggtcaca gtcttcgagt ggttcaggaa 4620
cggccagttc tggatgacag caagttgctc cgcctacctc gccgccgtct gccaggtgct 4680
gaccaaggtg atattccggc gggacatctc cttcaagctc acatccaagc taccctcggg 4740
agacgagaag aaggacccct acgccgacct ctacgtggtg cgctggacgc cgctcatgat 4800
tacacccatc atcatcatct tcgtcaacat catcggatcc gccgtggcct tcgccaaggt 4860
tctcgacggc gagtggacgc actggctcaa ggtcgccggc ggcgtcttct tcaacttctg 4920
ggtgctcttc cacctctacc ccttcgccaa gggcatcctg ggaagcacg gaaagacgcc 4980
agtcgtggtg ctcgtctggt gggcattcac cttcgtcatc accgccgtgc tctacatcaa 5040

```
catccccac atgcatacct cgggaggcaa gcacacaacg gtgcatggtc accatggcaa     5100

gaagttggtc gacacagggc tctatggctg gctccattga                          5140


<210>  2
<211>  947
<212>  PRT
<213>  Hordeum vulgare


<220>
<221>  misc_feature
<223>  OUM125 bgl aa

<400>  2

Met Ala Pro Ala Val Ala Gly Gly Gly Arg Val Arg Ser Asn Glu Pro
1               5                   10                  15


Val Ala Ala Ala Ala Ala Ala Pro Ala Ala Ser Gly Lys Pro Cys Val
            20                  25                  30


Cys Gly Phe Gln Val Cys Ala Cys Thr Gly Ser Ala Ala Val Ala Ser
        35                  40                  45


Ala Ala Ser Ser Leu Asp Met Asp Ile Val Ala Met Gly Gln Ile Gly
    50                  55                  60


Ala Val Asn Asp Glu Ser Trp Val Gly Val Glu Leu Gly Glu Asp Gly
65                  70                  75                  80


Glu Thr Asp Glu Ser Gly Ala Ala Val Asp Asp Arg Pro Val Phe Arg
                85                  90                  95


Thr Glu Lys Ile Lys Gly Val Leu Leu His Pro Tyr Arg Val Leu Ile
            100                 105                 110


Phe Val Arg Leu Ile Ala Phe Thr Leu Phe Val Ile Trp Arg Ile Ser
        115                 120                 125


His Lys Asn Pro Asp Ala Met Trp Leu Trp Val Thr Ser Ile Cys Gly
    130                 135                 140


Glu Phe Trp Phe Gly Phe Ser Trp Leu Leu Asp Gln Leu Pro Lys Leu
145                 150                 155                 160


Asn Pro Ile Asn Arg Val Pro Asp Leu Ala Val Leu Arg Gln Arg Phe
                165                 170                 175


Asp Arg Pro Asp Gly Thr Ser Thr Leu Pro Gly Leu Asp Ile Phe Val
            180                 185                 190
```

```
Thr Thr Ala Asp Pro Ile Lys Glu Pro Ile Leu Ser Thr Ala Asn Ser
          195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240

Glu Ser Ser Lys Phe Ala Thr Leu Trp Val Pro Phe Cys Arg Lys His
                245                 250                 255

Gly Ile Glu Pro Arg Gly Pro Glu Ser Tyr Phe Glu Leu Lys Ser His
            260                 265                 270

Pro Tyr Met Gly Arg Ala Gln Asp Glu Phe Val Asn Asp Arg Arg Arg
        275                 280                 285

Val Arg Lys Glu Tyr Asp Glu Phe Lys Ala Arg Ile Asn Ser Leu Glu
    290                 295                 300

His Asp Ile Lys Gln Arg Asn Asp Gly Tyr Asn Ala Ala Ile Ala His
305                 310                 315                 320

Ser Gln Gly Val Pro Arg Pro Thr Trp Met Ala Asp Gly Thr Gln Trp
                325                 330                 335

Glu Gly Thr Trp Val Asp Ala Ser Glu Asn His Arg Arg Gly Asp His
            340                 345                 350

Ala Gly Ile Val Leu Val Leu Leu Asn His Pro Ser His Arg Arg Gln
        355                 360                 365

Thr Gly Pro Pro Ala Ser Ala Asp Asn Pro Leu Asp Leu Ser Gly Val
    370                 375                 380

Asp Val Arg Leu Pro Met Leu Val Tyr Val Ser Arg Glu Lys Arg Pro
385                 390                 395                 400

Gly His Asp His Gln Lys Lys Ala Gly Ala Met Asn Ala Leu Thr Arg
                405                 410                 415

Ala Ser Ala Leu Leu Ser Asn Ser Pro Phe Ile Leu Asn Leu Asp Cys
            420                 425                 430

Asp His Tyr Ile Asn Asn Ser Gln Ala Leu Arg Ala Gly Ile Cys Phe
        435                 440                 445
```

```
Met Val Gly Arg Asp Ser Asp Thr Val Ala Phe Val Gln Phe Pro Gln
    450                 455                 460

Arg Phe Glu Gly Val Asp Pro Thr Asp Leu Tyr Ala Asn His Asn Arg
465                 470                 475                 480

Ile Phe Phe Asp Gly Thr Leu Arg Ala Leu Asp Gly Met Gln Gly Pro
                485                 490                 495

Ile Tyr Val Gly Thr Gly Cys Leu Phe Arg Arg Ile Thr Val Tyr Gly
            500                 505                 510

Phe Asp Pro Pro Arg Ile Asn Val Gly Gly Pro Cys Phe Pro Arg Leu
        515                 520                 525

Ala Gly Leu Phe Ala Lys Thr Lys Tyr Glu Lys Pro Gly Leu Glu Met
    530                 535                 540

Thr Thr Ala Lys Ala Lys Ala Ala Pro Val Pro Ala Lys Gly Lys His
545                 550                 555                 560

Gly Phe Leu Pro Leu Pro Lys Lys Thr Tyr Gly Lys Ser Asp Ala Phe
                565                 570                 575

Val Asp Thr Ile Pro Arg Ala Ser His Pro Ser Pro Tyr Ala Ala Ala
            580                 585                 590

Ala Glu Gly Ile Val Ala Asp Glu Ala Thr Ile Val Glu Ala Val Asn
        595                 600                 605

Val Thr Ala Ala Ala Phe Glu Lys Lys Thr Gly Trp Gly Lys Glu Ile
    610                 615                 620

Gly Trp Val Tyr Asp Thr Val Thr Glu Asp Val Val Thr Gly Tyr Arg
625                 630                 635                 640

Met His Ile Lys Gly Trp Arg Ser Arg Tyr Cys Ser Ile Tyr Pro His
                645                 650                 655

Ala Phe Ile Asp Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685

Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
```

705 710 715 720

Tyr Thr Thr Val Pro Ala Leu Ser Phe Val Thr Gly His Phe Ile Val
725 730 735

Gln Arg Pro Thr Thr Met Phe Tyr Val Tyr Leu Gly Ile Val Leu Ser
740 745 750

Thr Leu Leu Val Ile Ala Val Leu Glu Val Lys Trp Ala Gly Val Thr
755 760 765

Val Phe Glu Trp Phe Arg Asn Gly Gln Phe Trp Met Thr Ala Ser Cys
770 775 780

Ser Ala Tyr Leu Ala Ala Val Cys Gln Val Leu Thr Lys Val Ile Phe
785 790 795 800

Arg Arg Asp Ile Ser Phe Lys Leu Thr Ser Lys Leu Pro Ser Gly Asp
805 810 815

Glu Lys Lys Asp Pro Tyr Ala Asp Leu Tyr Val Val Arg Trp Thr Pro
820 825 830

Leu Met Ile Thr Pro Ile Ile Ile Ile Phe Val Asn Ile Ile Gly Ser
835 840 845

Ala Val Ala Phe Ala Lys Val Leu Asp Gly Glu Trp Thr His Trp Leu
850 855 860

Lys Val Ala Gly Gly Val Phe Phe Asn Phe Trp Val Leu Phe His Leu
865 870 875 880

Tyr Pro Phe Ala Lys Gly Ile Leu Gly Lys His Gly Lys Thr Pro Val
885 890 895

Val Val Leu Val Trp Trp Ala Phe Thr Phe Val Ile Thr Ala Val Leu
900 905 910

Tyr Ile Asn Ile Pro His Met His Thr Ser Gly Gly Lys His Thr Thr
915 920 925

Val His Gly His His Gly Lys Lys Leu Val Asp Thr Gly Leu Tyr Gly
930 935 940

Trp Leu His
945

<210> 3
<211> 2844

<212> DNA
<213> Hordeum vulgare

<220>
<221> misc_feature
<223> OUM125 bgl gene cDNA

<400> 3
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccgggtg ctgattttcg ttcgtctgat cgccttcacg 360

ctgttcgtga tctggcgtat ctcccacaag aacccagacg cgatgtggct gtgggtgaca 420

tccatctgcg gcgagttctg gttcggtttc tcgtggctgc tggatcagct gcccaagctg 480

aaccccatca accgcgtgcc ggacctggcg gtgctgcggc agcgcttcga ccgccccgac 540

ggcacctcca cgctcccggg gctggacatc ttcgtcacca cggccgaccc catcaaggag 600

cccatcctct ccaccgccaa ctcggtgctc tccatcctgg ccgccgacta ccccgtggac 660

cgcaacacat gctacgtctc cgacgacagt ggcatgctgc tcacctacga ggccctggca 720

gagtcctcca agttcgccac gctctgggtg cccttctgcc gcaagcacgg gatcgagccc 780

aggggtccgg agagctactt cgagctcaag tcacaccctt acatggggag agcccaggac 840

gagttcgtca acgaccgccg ccgcgttcgc aaggagtacg acgagttcaa ggccaggatc 900

aacagcctgg agcatgacat caagcagcgc aacgacgggt acaacgccgc cattgcccac 960

agccaaggcg tgccccggcc cacctggatg gcggacggca cccagtggga gggcacatgg 1020

gtcgacgcct ccgagaacca ccgcaggggc gaccacgccg gcatcgtact ggtgctgctg 1080

aaccacccga gccaccgccg gcagacgggc ccgccggcga gcgctgacaa cccactggac 1140

ttgagcggcg tggatgtgcg tctccccatg ctggtgtacg tgtcccgtga gaagcgcccc 1200

gggcacgacc accagaagaa ggccggtgcc atgaacgcgc ttacccgcgc ctcggcgctg 1260

ctctccaact ccccccttcat cctcaacctc gactgcgatc attacatcaa caactcccag 1320

gcccttcgcg ccggcatctg cttcatggtg ggacgggaca gcgacacggt tgccttcgtc 1380

cagttcccgc agcgcttcga gggcgtcgac cccaccgacc tctacgccaa ccacaaccgc 1440

atcttcttcg acggcaccct ccgtgccctg gacggcatgc agggccccat ctacgtcggc 1500

actgggtgtc tcttccgccg catcaccgtc tacggcttcg acccgccgag gatcaacgtc 1560

ggcggtccct gcttccccag gctcgccggg ctcttcgcca agaccaagta cgagaagccc 1620

gggctcgaga tgaccacggc caaggccaag gccgcgcccg tgcccgccaa gggtaagcac 1680

ggcttcttgc cactgcccaa gaagacgtac ggcaagtcgg acgccttcgt ggacaccatc 1740

ccgcgcgcgt cgcacccgtc gccctacgcc gcggcggctg aggggatcgt ggccgacgag 1800

gcgaccatcg tcgaggcggt gaacgtgacg gccgccgcgt tcgagaagaa gaccggctgg 1860

ggcaaagaga tcggctgggt gtacgacacc gtcacggagg acgtggtcac cggctaccgg 1920

atgcatatca aggggtggcg gtcacgctac tgctccatct acccacacgc cttcatcgac 1980

accgccccca tcaacctcac ggagaggctc ttccaggtgc tccgctggtc cacgggatcc 2040

ctcgagatct tcttctccaa gaacaacccg ctcttcggca gcacatacct ccacccgctg 2100

cagcgcgtcg cctacatcaa catcaccact taccccttca ccgccatctt cctcatcttc 2160

tacaccaccg tgccggcgct atccttcgtc accggccact tcatcgtgca gcgcccgacc 2220

accatgttct acgtctacct gggcatcgtg ctatccacgc tgctcgtcat cgccgtgctg 2280

gaggtcaagt gggccggggt cacagtcttc gagtggttca ggaacggcca gttctggatg 2340

acagcaagtt gctccgccta cctcgccgcc gtctgccagg tgctgaccaa ggtgatattc 2400

cggcgggaca tctccttcaa gctcacatcc aagctaccct cgggagacga gaagaaggac 2460

ccctacgccg acctctacgt ggtgcgctgg acgccgctca tgattacacc catcatcatc 2520

atcttcgtca acatcatcgg atccgccgtg gccttcgcca aggttctcga cggcgagtgg 2580

acgcactggc tcaaggtcgc cggcggcgtc ttcttcaact tctgggtgct cttccacctc 2640

taccccttcg ccaagggcat cctggggaag cacggaaaga cgccagtcgt ggtgctcgtc 2700

tggtgggcat tcaccttcgt catcaccgcc gtgctctaca tcaacatccc ccacatgcat 2760

acctcgggag gcaagcacac aacggtgcat ggtcaccatg gcaagaagtt ggtcgacaca 2820

gggctctatg gctggctcca ttga 2844

<210> 4
<211> 5140
<212> DNA
<213> Hordeum vulgare

<220>
<221> misc_feature
<223> Akashinriki HvCslF6 gene genomic DNA

<400> 4
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccggtac gtcctgctcc cacaactaaa cagaaactcc 360

ctatatctgc gtcacactca acaattaatc caactaagtc tctctactac tctagtattt 420

atttttactc tctatctgca caacaagcgc tactacaatt aacccaacaa gcaccacgcc 480
aggttgacag tcaggataat ttgatcttga ccggagtaag tactagtact aggtcggtgt 540
taatcagagt aattattgca ctagttaatt aaaatttgag taatccgaga caggtgcacg 600
ttagggccgg gccaatgatg gctcgaatcc acccaaaata gcgcgtcccg gtgtgggctg 660
tcggctcggt gcttcttcct tccattttac tagtcgcagt cactgcagct tgggcccacg 720
ggaggggacg ttagccgttg ggcctgcctg gcaggtgggc cccggtggcc accctggcgg 780
ctcataaatc cttgctactt tggagctgta atggacgctc tgcaatagca ataggaatcc 840
gaggtgaaac gacgacagtg ggcatggcat ggcttgcatg tgaatccaag ccacatcatt 900
aaaagcatcc tccctgggca cgtcgcggtg agaaagttgg ataaactttt gggggttcgg 960
acaagatgag aaaaagcaag taacatgtcc ctttttggc accgaagaaa tcctatgtac 1020
ggcgagtttt ttctgcatct agttatgggt agatgtacgt tagtttttgt tgagcgtttt 1080
atgtgctaca tatatggaga aaaagagaaa aatattatca tgtcatgtca tgccatgcca 1140
tgagaaggag gagaagaaaa agaagtgttg ccaccgctcg aatgcctttt ttttctttcg 1200
gaaggatgcg tgagtcatgt tggcaccgag aaaagccata ttaaagtggc agagttacaa 1260
ctcagaataa atgcgggtgt tacaaaaaca ctaggatatg tgaagggcac tcggcacaac 1320
actttaagac tacacaattg aaaaagtgta ctctctgtat ctaaataatt ataattgaaa 1380
agaattaatc tatatatgaa agtagtaatg attagcggta gaaggttcca acgacttttt 1440
tggcgccaat agcaagaaga aagaaaaaga aaaaaatctt tactgtacag tataacgaga 1500
aaagaggccc attaatagag caacgaatcg agcggcacca cctctggcgg tcacgtccat 1560
gccctcgcac gacggatgag gcccgggggg tcctactgac agccgaagca tgtcggtgct 1620
caaacacggc gccgtttgct gccaagtgtg ccagctcgca ctcattgact tgccagctct 1680
ctccttggtt gtcaatgaga acatgatgcc ttttggcatt tgcaaactta ttaaaactag 1740
ctgtcgtccg atagggaaaa gaaaagaaaa gaaaagaata agaaaaaaag gacaaagaga 1800
aaagatgaac atggcgcatg ttccctccaa taattgcagg caccaacact gggtcgatta 1860
atccaacaac aatattttac tataccagac gagagtacag tagtcgggtg atgatggact 1920
gtaactgact gagtatgaat gactgtaatg cagggtgctg attttcgttc gtctgatcgc 1980
cttcacgctg ttcgtgatct ggcgtatctc ccacaagaac ccagacgcga tgtggctgtg 2040
ggtgacatcc atctgcggcg agttctggtt cggtttctcg tggctgctgg atcagctgcc 2100
caagctgaac ccccatcaacc gcgtgccgga cctggcggtg ctgcggcagc gcttcgaccg 2160
ccccgacggc acctccacgc tcccggggct ggacatcttc gtcaccacgg ccgaccccat 2220
caaggagccc atcctctcca ccgccaactc ggtgctctcc atcctggccg ccgactaccc 2280
cgtggaccgc aacacatgct acgtctccga cgacagtggc atgctgctca cctacgaggc 2340

cctggcagag tcctccaagt tcgccacgct ctgggtgccc ttctgccgca agcacgggat 2400
cgagcccagg ggtccggaga gctacttcga gctcaagtca cacccttaca tggggagagc 2460
ccaggacgag ttcgtcaacg accgccgccg cgttcgcaag gagtacgacg agttcaaggc 2520
caggatcaac agcctggagc atgacatcaa gcagcgcaac gacgggtaca acgccgccat 2580
tgcccacagc caaggcgtgc cccggcccac ctggatggcg gacggcaccc agtgggaggg 2640
cacatgggtc gacgcctccg agaaccaccg caggggcgac cacgccggca tcgtactggt 2700
cagtatccat ccatctttct gctgcttata ttactcttag gttactctta tcgtctcttt 2760
cctatactgt acatgcatgc atgctgctat tcttggaatc gtggttggtt actactccac 2820
catgcaaaaa taacaagaag aggaatcttg gttagttagg gcctcgttgt tatattagtg 2880
gccatctgat gtgatgcctg ccggctgtgc ccatccatat ccatggaaga tttcgacaga 2940
atcgacgtgg tgatagtcga gagtgcaacc accacccaga gccagccaag cacatgcatg 3000
cttctcttct cgtctcgtcg tgtggccagc agcgcattca tgctattgct gtgacgaggg 3060
aggaatggta gttggggtgg tcctttcccc ccgacagcac tacagcctcc actttatgac 3120
ccatttaatt caccggccct gctttgttgt aaccgccttc tcatctcaat caatcattca 3180
ttcattcata agtttactca ctctttgtta ctactcgaac cactaatcag gaaggagtag 3240
gagtaatgca gatttactat tgacagttaa aggagtaaaa agaaggaagc acaattacag 3300
aaccttgttt ttttttacta ctgtacgtaa ggtgtaagaa tggagtgctg acagagaatg 3360
gatgcaggtg ctgctgaacc acccgagcca ccgccggcag acgggcccgc cggcgagcgc 3420
tgacaaccca ctggacttga gcggcgtgga tgtgcgtctc cccatgctgg tgtacgtgtc 3480
ccgtgagaag cgccccgggc acgaccacca gaagaaggcc ggtgccatga acgcgcttac 3540
ccgcgcctcg gcgctgctct ccaactcccc cttcatcctc aacctcgact gcgatcatta 3600
catcaacaac tcccaggccc ttcgcgccgg catctgcttc atggtgggac gggacagcga 3660
cacggttgcc ttcgtccagt tcccgcagcg cttcgagggc gtcgacccca ccgacctcta 3720
cgccaaccac aaccgcatct tcttcgacgg caccctccgt gccctggacg gcatgcaggg 3780
ccccatctac gtcggcactg ggtgtctctt ccgccgcatc accgtctacg gcttcgaccc 3840
gccgaggatc aacgtcggcg gtccctgctt ccccaggctc gccgggctct tcgccaagac 3900
caagtacgag aagcccgggc tcgagatgac cacggccaag gccaaggccg cgcccgtgcc 3960
cgccaagggt aagcacggct tcttgccact gcccaagaag acgtacggca agtcggacgc 4020
cttcgtggac accatcccgc gcgcgtcgca cccgtcgccc tacgccgcgg cggctgaggg 4080
gatcgtggcc gacgaggcga ccatcgtcga ggcggtgaac gtgacggccg ccgcgttcga 4140
gaagaagacc ggctggggca aagagatcgg ctgggtgtac gacaccgtca cggaggacgt 4200
ggtcaccggc taccggatgc atatcaaggg gtggcggtca cgctactgct ccatctaccc 4260
acacgccttc atcggcaccg cccccatcaa cctcacggag aggctcttcc aggtgctccg 4320

ctggtccacg ggatccctcg agatcttctt ctccaagaac aacccgctct tcggcagcac 4380

atacctccac ccgctgcagc gcgtcgccta catcaacatc accacttacc ccttcaccgc 4440

catcttcctc atcttctaca ccaccgtgcc ggcgctatcc ttcgtcaccg gccacttcat 4500

cgtgcagcgc ccgaccacca tgttctacgt ctacctgggc atcgtgctat ccacgctgct 4560

cgtcatcgcc gtgctggagg tcaagtgggc cggggtcaca gtcttcgagt ggttcaggaa 4620

cggccagttc tggatgacag caagttgctc cgcctacctc gccgccgtct gccaggtgct 4680

gaccaaggtg atattccggc gggacatctc cttcaagctc acatccaagc taccctcggg 4740

agacgagaag aaggacccct acgccgacct ctacgtggtg cgctggacgc cgctcatgat 4800

tacacccatc atcatcatct tcgtcaacat catcggatcc gccgtggcct tcgccaaggt 4860

tctcgacggc gagtggacgc actggctcaa ggtcgccggc ggcgtcttct tcaacttctg 4920

ggtgctcttc cacctctacc ccttcgccaa gggcatcctg gggaagcacg gaaagacgcc 4980

agtcgtggtg ctcgtctggt gggcattcac cttcgtcatc accgccgtgc tctacatcaa 5040

catcccccac atgcatacct cgggaggcaa gcacacaacg gtgcatggtc accatggcaa 5100

gaagttggtc gacacagggc tctatggctg gctccattga 5140

<210> 5
<211> 947
<212> PRT
<213> Hordeum vulgare

<220>
<221> misc_feature
<223> Akashinriki HvCslF6 aa

<400> 5

Met Ala Pro Ala Val Ala Gly Gly Gly Arg Val Arg Ser Asn Glu Pro
1 5 10 15

Val Ala Ala Ala Ala Ala Ala Pro Ala Ala Ser Gly Lys Pro Cys Val
20 25 30

Cys Gly Phe Gln Val Cys Ala Cys Thr Gly Ser Ala Ala Val Ala Ser
35 40 45

Ala Ala Ser Ser Leu Asp Met Asp Ile Val Ala Met Gly Gln Ile Gly
50 55 60

Ala Val Asn Asp Glu Ser Trp Val Gly Val Glu Leu Gly Glu Asp Gly
65 70 75 80

Glu Thr Asp Glu Ser Gly Ala Ala Val Asp Asp Arg Pro Val Phe Arg
85 90 95

```
Thr Glu Lys Ile Lys Gly Val Leu Leu His Pro Tyr Arg Val Leu Ile
            100                 105                 110

Phe Val Arg Leu Ile Ala Phe Thr Leu Phe Val Ile Trp Arg Ile Ser
        115                 120                 125

His Lys Asn Pro Asp Ala Met Trp Leu Trp Val Thr Ser Ile Cys Gly
    130                 135                 140

Glu Phe Trp Phe Gly Phe Ser Trp Leu Leu Asp Gln Leu Pro Lys Leu
145                 150                 155                 160

Asn Pro Ile Asn Arg Val Pro Asp Leu Ala Val Leu Arg Gln Arg Phe
                165                 170                 175

Asp Arg Pro Asp Gly Thr Ser Thr Leu Pro Gly Leu Asp Ile Phe Val
            180                 185                 190

Thr Thr Ala Asp Pro Ile Lys Glu Pro Ile Leu Ser Thr Ala Asn Ser
        195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240

Glu Ser Ser Lys Phe Ala Thr Leu Trp Val Pro Phe Cys Arg Lys His
                245                 250                 255

Gly Ile Glu Pro Arg Gly Pro Glu Ser Tyr Phe Glu Leu Lys Ser His
            260                 265                 270

Pro Tyr Met Gly Arg Ala Gln Asp Glu Phe Val Asn Asp Arg Arg Arg
        275                 280                 285

Val Arg Lys Glu Tyr Asp Glu Phe Lys Ala Arg Ile Asn Ser Leu Glu
    290                 295                 300

His Asp Ile Lys Gln Arg Asn Asp Gly Tyr Asn Ala Ala Ile Ala His
305                 310                 315                 320

Ser Gln Gly Val Pro Arg Pro Thr Trp Met Ala Asp Gly Thr Gln Trp
                325                 330                 335

Glu Gly Thr Trp Val Asp Ala Ser Glu Asn His Arg Arg Gly Asp His
            340                 345                 350
```

Ala Gly Ile Val Leu Val Leu Leu Asn His Pro Ser His Arg Arg Gln
355 360 365

Thr Gly Pro Pro Ala Ser Ala Asp Asn Pro Leu Asp Leu Ser Gly Val
370 375 380

Asp Val Arg Leu Pro Met Leu Val Tyr Val Ser Arg Glu Lys Arg Pro
385 390 395 400

Gly His Asp His Gln Lys Lys Ala Gly Ala Met Asn Ala Leu Thr Arg
405 410 415

Ala Ser Ala Leu Leu Ser Asn Ser Pro Phe Ile Leu Asn Leu Asp Cys
420 425 430

Asp His Tyr Ile Asn Asn Ser Gln Ala Leu Arg Ala Gly Ile Cys Phe
435 440 445

Met Val Gly Arg Asp Ser Asp Thr Val Ala Phe Val Gln Phe Pro Gln
450 455 460

Arg Phe Glu Gly Val Asp Pro Thr Asp Leu Tyr Ala Asn His Asn Arg
465 470 475 480

Ile Phe Phe Asp Gly Thr Leu Arg Ala Leu Asp Gly Met Gln Gly Pro
485 490 495

Ile Tyr Val Gly Thr Gly Cys Leu Phe Arg Arg Ile Thr Val Tyr Gly
500 505 510

Phe Asp Pro Pro Arg Ile Asn Val Gly Gly Pro Cys Phe Pro Arg Leu
515 520 525

Ala Gly Leu Phe Ala Lys Thr Lys Tyr Glu Lys Pro Gly Leu Glu Met
530 535 540

Thr Thr Ala Lys Ala Lys Ala Ala Pro Val Pro Ala Lys Gly Lys His
545 550 555 560

Gly Phe Leu Pro Leu Pro Lys Lys Thr Tyr Gly Lys Ser Asp Ala Phe
565 570 575

Val Asp Thr Ile Pro Arg Ala Ser His Pro Ser Pro Tyr Ala Ala Ala
580 585 590

Ala Glu Gly Ile Val Ala Asp Glu Ala Thr Ile Val Glu Ala Val Asn
595 600 605

Val Thr Ala Ala Ala Phe Glu Lys Lys Thr Gly Trp Gly Lys Glu Ile

```
        610                 615                 620

Gly Trp Val Tyr Asp Thr Val Thr Glu Asp Val Val Thr Gly Tyr Arg
625                 630                 635                 640

Met His Ile Lys Gly Trp Arg Ser Arg Tyr Cys Ser Ile Tyr Pro His
                645                 650                 655

Ala Phe Ile Gly Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685

Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
705                 710                 715                 720

Tyr Thr Thr Val Pro Ala Leu Ser Phe Val Thr Gly His Phe Ile Val
                725                 730                 735

Gln Arg Pro Thr Thr Met Phe Tyr Val Tyr Leu Gly Ile Val Leu Ser
            740                 745                 750

Thr Leu Leu Val Ile Ala Val Leu Glu Val Lys Trp Ala Gly Val Thr
        755                 760                 765

Val Phe Glu Trp Phe Arg Asn Gly Gln Phe Trp Met Thr Ala Ser Cys
    770                 775                 780

Ser Ala Tyr Leu Ala Ala Val Cys Gln Val Leu Thr Lys Val Ile Phe
785                 790                 795                 800

Arg Arg Asp Ile Ser Phe Lys Leu Thr Ser Lys Leu Pro Ser Gly Asp
                805                 810                 815

Glu Lys Lys Asp Pro Tyr Ala Asp Leu Tyr Val Val Arg Trp Thr Pro
            820                 825                 830

Leu Met Ile Thr Pro Ile Ile Ile Ile Phe Val Asn Ile Ile Gly Ser
        835                 840                 845

Ala Val Ala Phe Ala Lys Val Leu Asp Gly Glu Trp Thr His Trp Leu
    850                 855                 860

Lys Val Ala Gly Gly Val Phe Phe Asn Phe Trp Val Leu Phe His Leu
865                 870                 875                 880
```

```
Tyr Pro Phe Ala Lys Gly Ile Leu Gly Lys His Gly Lys Thr Pro Val
                885                 890                 895

Val Val Leu Val Trp Trp Ala Phe Thr Phe Val Ile Thr Ala Val Leu
            900                 905                 910

Tyr Ile Asn Ile Pro His Met His Thr Ser Gly Gly Lys His Thr Thr
        915                 920                 925

Val His Gly His His Gly Lys Lys Leu Val Asp Thr Gly Leu Tyr Gly
    930                 935                 940

Trp Leu His
945


<210>  6
<211>  2844
<212>  DNA
<213>  Hordeum vulgare


<220>
<221>  misc_feature
<223>  Akashinriki HvCslF6 gene cDNA

<400>  6
atggcgccag cggtggccgg aggggcgcgc gtgcggagca atgagccggt tgctgctgct      60

gccgccgcgc cggcggccag cggcaagccc tgcgtgtgcg gcttccaggt ttgcgcctgc     120

acggggtcgg ccgcggtggc ctccgccgcc tcgtcgctgg acatggacat cgtggccatg     180

gggcagatcg gcgccgtcaa cgacgagagc tgggtgggcg tggagctcgg cgaagatggc     240

gagaccgacg aaagcggtgc cgccgttgac gaccgccccg tattccgcac cgagaagatc     300

aagggtgtcc tcctccaccc ctaccgggtg ctgattttcg ttcgtctgat cgccttcacg     360

ctgttcgtga tctggcgtat ctcccacaag aacccagacg cgatgtggct gtgggtgaca     420

tccatctgcg gcgagttctg gttcggtttc tcgtggctgc tggatcagct gcccaagctg     480

aaccccatca accgcgtgcc ggacctggcg gtgctgcggc agcgcttcga ccgccccgac     540

ggcacctcca cgctcccggg gctggacatc ttcgtcacca cggccgaccc catcaaggag     600

cccatcctct ccaccgccaa ctcggtgctc tccatcctgg ccgccgacta ccccgtggac     660

cgcaacacat gctacgtctc cgacgacagt ggcatgctgc tcacctacga ggccctggca     720

gagtcctcca agttcgccac gctctgggtg cccttctgcc gcaagcacgg gatcgagccc     780

aggggtccgg agagctactt cgagctcaag tcacaccctt acatggggag agcccaggac     840

gagttcgtca acgaccgccg ccgcgttcgc aaggagtacg acgagttcaa ggccaggatc     900

aacagcctgg agcatgacat caagcagcgc aacgacgggt acaacgccgc cattgcccac     960
```

agccaaggcg tgccccggcc cacctggatg gcggacggca cccagtggga gggcacatgg 1020
gtcgacgcct ccgagaacca ccgcaggggc gaccacgccg gcatcgtact ggtgctgctg 1080
aaccacccga gccaccgccg gcagacgggc ccgccggcga gcgctgacaa cccactggac 1140
ttgagcggcg tggatgtgcg tctccccatg ctggtgtacg tgtcccgtga gaagcgcccc 1200
gggcacgacc accagaagaa ggccggtgcc atgaacgcgc ttacccgcgc ctcggcgctg 1260
ctctccaact cccccttcat cctcaacctc gactgcgatc attacatcaa caactcccag 1320
gcccttcgcg ccggcatctg cttcatggtg ggacgggaca gcgacacggt tgccttcgtc 1380
cagttcccgc agcgcttcga gggcgtcgac ccccaccgacc tctacgccaa ccacaaccgc 1440
atcttcttcg acggcaccct ccgtgccctg gacggcatgc agggccccat ctacgtcggc 1500
actgggtgtc tcttccgccg catcaccgtc tacggcttcg acccgccgag gatcaacgtc 1560
ggcggtccct gcttccccag gctcgccggg ctcttcgcca agaccaagta cgagaagccc 1620
gggctcgaga tgaccacggc caaggccaag gccgcgcccg tgcccgccaa gggtaagcac 1680
ggcttcttgc cactgcccaa gaagacgtac ggcaagtcgg acgccttcgt ggacaccatc 1740
ccgcgcgcgt cgcacccgtc gccctacgcc gcggcggctg aggggatcgt ggccgacgag 1800
gcgaccatcg tcgaggcggt gaacgtgacg gccgccgcgt tcgagaagaa gaccggctgg 1860
ggcaaagaga tcggctgggt gtacgacacc gtcacggagg acgtggtcac cggctaccgg 1920
atgcatatca aggggtggcg gtcacgctac tgctccatct acccacacgc cttcatcggc 1980
accgccccca tcaacctcac ggagaggctc ttccaggtgc tccgctggtc cacgggatcc 2040
ctcgagatct tcttctccaa gaacaacccg ctcttcggca gcacatacct ccacccgctg 2100
cagcgcgtcg cctacatcaa catcaccact taccccttca ccgccatctt cctcatcttc 2160
tacaccaccg tgccggcgct atccttcgtc accggccact tcatcgtgca gcgcccgacc 2220
accatgttct acgtctacct gggcatcgtg ctatccacgc tgctcgtcat cgccgtgctg 2280
gaggtcaagt gggccggggt cacagtcttc gagtggttca ggaacggcca gttctggatg 2340
acagcaagtt gctccgccta cctcgccgcc gtctgccagg tgctgaccaa ggtgatattc 2400
cggcgggaca tctccttcaa gctcacatcc aagctaccct cgggagacga gaagaaggac 2460
ccctacgccg acctctacgt ggtgcgctgg acgccgctca tgattacacc catcatcatc 2520
atcttcgtca acatcatcgg atccgccgtg gccttcgcca aggttctcga cggcgagtgg 2580
acgcactggc tcaaggtcgc cggcggcgtc ttcttcaact tctgggtgct cttccacctc 2640
taccccttcg ccaagggcat cctggggaag cacggaaaga cgccagtcgt ggtgctcgtc 2700
tggtgggcat tcaccttcgt catcaccgcc gtgctctaca tcaacatccc ccacatgcat 2760
acctcgggag gcaagcacac aacggtgcat ggtcaccatg gcaagaagtt ggtcgacaca 2820
gggctctatg gctggctcca ttga 2844

```
<210>  7
<211>  5140
<212>  DNA
<213>  Hordeum vulgare


<220>
<221>  misc_feature
<223>  Nishinohoshi HvCslF6 gene genomic DNA

<400>  7
atggcgccag cggtggccgg aggggccgc gtgcggagca atgagccggt tgctgctgct      60

gccgccgcgc cggcggccag cggcaagccc tgcgtgtgcg gcttccaggt ttgcgcctgc     120

acggggtcgg ccgcggtggc ctccgccgcc tcgtcgctgg acatggacat cgtggccatg     180

gggcagatcg gcgccgtcaa cgacgagagc tgggtgggcg tggagctcgg cgaagatggc     240

gagaccgacg aaagcggtgc cgccgttgac gaccgccccg tattccgcac cgagaagatc     300

aagggtgtcc tcctccaccc ctaccggtac gttctgctcc cacaactaaa cagaaactcc     360

ctatatctgc gtcacactca acaattaatc caactaagtc tctctactac tctagtattt     420

atttttactc tctatctgca caacaagcgc tactacaatt aacccaacaa gcaccacgcc     480

aggttgacag tcaggataat ttgatcttga ccggagtaag tactagtact aggtcggtgt     540

taatcagagt aattattgca ctagttaatt aaaatttgag taatccgaga caggtgcacg     600

ttagggccgg gccaatgatg gctcgaatcc acccaaaata gcgcgtcccg gtgtgggctg     660

tcggctcggt gcttcttcct tccattttac tagtcgcagt cactgcagct tgggcccacg     720

ggaggggacg ttagccgttg ggcctgcctg gcaggtgggc cccggtggcc accctggcgg     780

ctcataaatc cttgctactt tggagctgta atggacgctc tgcaatagca ataggaatcc     840

gaggtgaaac gacgacagtg ggcatggcat ggcttgcatg tgaatccaag ccacatcatt     900

aaaagcatcc tccctgggca cgtcgcggtg agaaagttgg ataaactttt gggggttcgg     960

acaagatgag aaaaagcaag taacatgtcc ctttttggc accgaagaaa tcctatgtac    1020

ggcgagtttt ttctgcatct agttatgggt agatgtacgt tagtttttgt tgagcgtttt    1080

atgtgctaca tatatggaga aaaagagaaa aatattatca tgtcatgtca tgccatgcca    1140

tgagaaggag gagaagaaaa agaagtgttg ccaccgctcg aatgcctttt ttttctttcg    1200

gaaggatgcg tgagtcatgt tggcaccgag aaaagccata ttaaagtggc agagttacaa    1260

ctcagaataa atgcgggtgt tacaaaaaca ctaggatatg tgaagggcac tcggcacaac    1320

actttaagac tacacaattg aaaaagtgta ctctctgtat ctaaataatt ataattgaaa    1380

agaattaatc tatatatgaa agtagtaatg attagcggta gaaggttcca acgacttttt    1440

tggcgccaat agcaagaaga aagaaaaaga aaaaaatctt tactgtacag tataacgaga    1500

aaagaggccc attaatagag caacgaatcg agcggcacca cctctggcgg tcacgtccat    1560

gccctcgcac gacggatgag gcccgggggg tcctactgac agccgaagca tgtcggtgct    1620
```

caaacacggc gccgtttgct gccaagtgtg ccagctcgca ctcattgact tgccagctct 1680
ctccttggtt gtcaatgaga acatgatgcc ttttggcatt tgcaaactta ttaaaactag 1740
ctgtcgtccg atagggaaaa gaaaagaaaa gaaaagaata agaaaaaaag gacaaagaga 1800
aaagatgaac atggcgcatg ttccctccaa taattgcagg caccaacact gggtcgatta 1860
atccaacaac aatattttac tataccagac gagagtacag tagtcgggtg atgatggact 1920
gtaactgact gagtatgaat gactgtaatg cagggtgctg attttcgttc gtctgatcgc 1980
cttcacgctg ttcgtgatct ggcgtatctc ccacaagaac ccagacgcga tgtggctgtg 2040
ggtgacatcc atctgcggcg agttctggtt cggtttctcg tggctgctgg atcagctgcc 2100
caagctgaac cccatcaacc gcgtgccgga cctggcggtg ctgcggcagc gcttcgaccg 2160
ccccgacggc acctccacgc tcccggggct ggacatcttc gtcaccacgg ccgaccccat 2220
caaggagccc atcctctcca ccgccaactc ggtgctctcc atcctggccg ccgactaccc 2280
cgtggaccgc aacacatgct acgtctccga cgacagtggc atgctgctca cctacgaggc 2340
cctggcagag tcctccaagt tcgccacgct ctgggtgccc ttctgccgca agcacgggat 2400
cgagcccagg ggtccggaga gctacttcga gctcaagtca caccccttaca tggggagagc 2460
ccaggacgag ttcgtcaacg accgccgccg cgttcgcaag gagtacgacg agttcaaggc 2520
caggatcaac agcctggagc atgacatcaa gcagcgcaac gacgggtaca acgccgccat 2580
tgcccacagc caaggcgtgc cccggcccac ctggatggcg gacggcaccc agtgggaggg 2640
cacatgggtc gacgcctccg agaaccaccg caggggcgac cacgccggca tcgtactggt 2700
cagtatccat ccatctttct gctgcttata ttactcttag gttactctta tcgtctcttt 2760
cctatactgt acatgcatgc atgctgctat tcttggaatc gtggttggtt actactccac 2820
catgcaaaaa taacaagaag aggaatcttg gttagttagg gcctcgttgt tatattagtg 2880
gccatctgat gtgatgcctg ccggctgtgc ccatccatat ccatggaaga tttcgacaga 2940
atcgacgtgg tgatagtcga gagtgcaacc accacccaga gccagccaag cacatgcatg 3000
cttctcttct cgtctcgtcg tgtggccagc agcgcattca tgctattgct gtgacgaggg 3060
aggaatggta gttggggtgg tcctttcccc ccgacagcac tacagcctcc actttatgac 3120
ccatttaatt caccggccct gctttgttgt aaccgccttc tcacctcaat caatcattca 3180
ttcattcata agtttactca ctctttgtta ctactcgaac cactaatcag gaaggagtag 3240
gagtaatgca gatttactat taacagttaa aggagtaaaa agaaggaagc acaattacag 3300
aaccttgttt ttttttacta ctgtacgtaa ggtgtaagaa tggagcgctg acagagaatg 3360
gatgcaggtg ctgctgaacc acccgagcca ccgccggcag acgggcccgc cggcgagcgc 3420
tgacaaccca ctggacttga gcggcgtgga tgtgcgtctc cccatgctgg tgtacgtgtc 3480
ccgtgagaag cgcccccgggc acgaccacca gaagaaggcc ggtgccatga acgcgcttac 3540
ccgcgcctcg gcgctgctct ccaactcccc cttcatcctc aacctcgact gcgatcatta 3600

catcaacaac tcccaggccc ttcgcgccgg catctgcttc atggtgggac gggacagcga 3660

cacggttgcc ttcgtccagt tcccgcagcg cttcgagggc gtcgacccca ccgacctcta 3720

cgccaaccac aaccgcatct tcttcgacgg caccctccgt gccctggacg gcatgcaggg 3780

ccccatctac gtcggcactg ggtgtctctt ccgccgcatc accgtctacg gcttcgaccc 3840

gccgaggatc aacgtcggcg gtccctgctt ccccaggctc gccgggctct tcgccaagac 3900

caagtacgag aagcccgggc tcgagatgac cacggccaag gccaaggccg cgcccgtgcc 3960

cgccaagggt aagcacggct tcttgccact gcccaagaag acgtacggca agtcggacgc 4020

cttcgtggac accatcccgc gcgcgtcgca cccgtcgccc tacgccgcgg cggctgaggg 4080

gatcgtggcc gacgaggcga ccatcgtcga ggcggtgaac gtgacggccg ccgcgttcga 4140

gaagaagacc ggctggggca aagagatcgg ctgggtgtac gacaccgtca cggaggacgt 4200

ggtcaccggc taccggatgc atatcaaggg gtggcggtca cgctactgct ccatctaccc 4260

acacgccttc atcggcaccg cccccatcaa cctcacggag aggctcttcc aggtgctccg 4320

ctggtccacg ggatccctcg agatcttctt ctccaagaac aacccgctct tcggcagcac 4380

atacctccac ccgctgcagc gcgtcgccta catcaacatc accacttacc ccttcaccgc 4440

catcttcctc atcttctaca ccaccgtgcc ggcgctatcc ttcgtcaccg gccacttcat 4500

cgtgcagcgc ccgaccacca tgttctacgt ctacctgggc atcgtgctat ccacgctgct 4560

cgtcatcgcc gtgctggagg tcaagtgggc cggggtcaca gtcttcgagt ggttcaggaa 4620

cggccagttc tggatgacag caagttgctc cgcctacctc gccgccgtct gccaggtgct 4680

gaccaaggtg atattccggc gggacatctc cttcaagctc acatccaagc taccctcggg 4740

agacgagaag aaggacccct acgccgacct ctacgtggtg cgctggacgc cgctcatgat 4800

tacacccatc atcatcatct tcgtcaacat catcggatcc gccgtggcct tcgccaaggt 4860

tctcgacggc gagtggacgc actggctcaa ggtcgccggc ggcgtcttct tcaacttctg 4920

ggtgctcttc cacctctacc ccttcgccaa gggcatcctg gggaagcacg gaaagacgcc 4980

agtcgtggtg ctcgtctggt gggcattcac cttcgtcatc accgccgtgc tctacatcaa 5040

catcccccac atgcatacct cgggaggcaa gcacacaacg gtgcatggtc accatggcaa 5100

gaagttggtc gacacagggc tctatggctg gctccattga 5140

<210> 8
<211> 947
<212> PRT
<213> Hordeum vulgare

<220>
<221> misc_feature
<223> Nishinohoshi HvCslF6 aa

<400> 8

```
Met Ala Pro Ala Val Ala Gly Gly Gly Arg Val Arg Ser Asn Glu Pro
1               5                   10                  15

Val Ala Ala Ala Ala Ala Ala Pro Ala Ala Ser Gly Lys Pro Cys Val
            20                  25                  30

Cys Gly Phe Gln Val Cys Ala Cys Thr Gly Ser Ala Ala Val Ala Ser
        35                  40                  45

Ala Ala Ser Ser Leu Asp Met Asp Ile Val Ala Met Gly Gln Ile Gly
    50                  55                  60

Ala Val Asn Asp Glu Ser Trp Val Gly Val Glu Leu Gly Glu Asp Gly
65                  70                  75                  80

Glu Thr Asp Glu Ser Gly Ala Ala Val Asp Asp Arg Pro Val Phe Arg
                85                  90                  95

Thr Glu Lys Ile Lys Gly Val Leu Leu His Pro Tyr Arg Val Leu Ile
            100                 105                 110

Phe Val Arg Leu Ile Ala Phe Thr Leu Phe Val Ile Trp Arg Ile Ser
        115                 120                 125

His Lys Asn Pro Asp Ala Met Trp Leu Trp Val Thr Ser Ile Cys Gly
    130                 135                 140

Glu Phe Trp Phe Gly Phe Ser Trp Leu Leu Asp Gln Leu Pro Lys Leu
145                 150                 155                 160

Asn Pro Ile Asn Arg Val Pro Asp Leu Ala Val Leu Arg Gln Arg Phe
                165                 170                 175

Asp Arg Pro Asp Gly Thr Ser Thr Leu Pro Gly Leu Asp Ile Phe Val
            180                 185                 190

Thr Thr Ala Asp Pro Ile Lys Glu Pro Ile Leu Ser Thr Ala Asn Ser
        195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240

Glu Ser Ser Lys Phe Ala Thr Leu Trp Val Pro Phe Cys Arg Lys His
                245                 250                 255
```

```
Gly Ile Glu Pro Arg Gly Pro Glu Ser Tyr Phe Glu Leu Lys Ser His
                260                 265                 270

Pro Tyr Met Gly Arg Ala Gln Asp Glu Phe Val Asn Asp Arg Arg Arg
            275                 280                 285

Val Arg Lys Glu Tyr Asp Glu Phe Lys Ala Arg Ile Asn Ser Leu Glu
    290                 295                 300

His Asp Ile Lys Gln Arg Asn Asp Gly Tyr Asn Ala Ala Ile Ala His
305                 310                 315                 320

Ser Gln Gly Val Pro Arg Pro Thr Trp Met Ala Asp Gly Thr Gln Trp
                    325                 330                 335

Glu Gly Thr Trp Val Asp Ala Ser Glu Asn His Arg Arg Gly Asp His
                340                 345                 350

Ala Gly Ile Val Leu Val Leu Leu Asn His Pro Ser His Arg Arg Gln
            355                 360                 365

Thr Gly Pro Pro Ala Ser Ala Asp Asn Pro Leu Asp Leu Ser Gly Val
    370                 375                 380

Asp Val Arg Leu Pro Met Leu Val Tyr Val Ser Arg Glu Lys Arg Pro
385                 390                 395                 400

Gly His Asp His Gln Lys Lys Ala Gly Ala Met Asn Ala Leu Thr Arg
                    405                 410                 415

Ala Ser Ala Leu Leu Ser Asn Ser Pro Phe Ile Leu Asn Leu Asp Cys
                420                 425                 430

Asp His Tyr Ile Asn Asn Ser Gln Ala Leu Arg Ala Gly Ile Cys Phe
            435                 440                 445

Met Val Gly Arg Asp Ser Asp Thr Val Ala Phe Val Gln Phe Pro Gln
    450                 455                 460

Arg Phe Glu Gly Val Asp Pro Thr Asp Leu Tyr Ala Asn His Asn Arg
465                 470                 475                 480

Ile Phe Phe Asp Gly Thr Leu Arg Ala Leu Asp Gly Met Gln Gly Pro
                    485                 490                 495

Ile Tyr Val Gly Thr Gly Cys Leu Phe Arg Arg Ile Thr Val Tyr Gly
                500                 505                 510

Phe Asp Pro Pro Arg Ile Asn Val Gly Gly Pro Cys Phe Pro Arg Leu
```

515 520 525

Ala Gly Leu Phe Ala Lys Thr Lys Tyr Glu Lys Pro Gly Leu Glu Met
530 535 540

Thr Thr Ala Lys Ala Lys Ala Ala Pro Val Pro Ala Lys Gly Lys His
545 550 555 560

Gly Phe Leu Pro Leu Pro Lys Lys Thr Tyr Gly Lys Ser Asp Ala Phe
565 570 575

Val Asp Thr Ile Pro Arg Ala Ser His Pro Ser Pro Tyr Ala Ala Ala
580 585 590

Ala Glu Gly Ile Val Ala Asp Glu Ala Thr Ile Val Glu Ala Val Asn
595 600 605

Val Thr Ala Ala Ala Phe Glu Lys Lys Thr Gly Trp Gly Lys Glu Ile
610 615 620

Gly Trp Val Tyr Asp Thr Val Thr Glu Asp Val Val Thr Gly Tyr Arg
625 630 635 640

Met His Ile Lys Gly Trp Arg Ser Arg Tyr Cys Ser Ile Tyr Pro His
645 650 655

Ala Phe Ile Gly Thr Ala Pro Ile Asn Leu Thr Glu Arg Leu Phe Gln
660 665 670

Val Leu Arg Trp Ser Thr Gly Ser Leu Glu Ile Phe Phe Ser Lys Asn
675 680 685

Asn Pro Leu Phe Gly Ser Thr Tyr Leu His Pro Leu Gln Arg Val Ala
690 695 700

Tyr Ile Asn Ile Thr Thr Tyr Pro Phe Thr Ala Ile Phe Leu Ile Phe
705 710 715 720

Tyr Thr Thr Val Pro Ala Leu Ser Phe Val Thr Gly His Phe Ile Val
725 730 735

Gln Arg Pro Thr Thr Met Phe Tyr Val Tyr Leu Gly Ile Val Leu Ser
740 745 750

Thr Leu Leu Val Ile Ala Val Leu Glu Val Lys Trp Ala Gly Val Thr
755 760 765

Val Phe Glu Trp Phe Arg Asn Gly Gln Phe Trp Met Thr Ala Ser Cys
770 775 780

Ser Ala Tyr Leu Ala Ala Val Cys Gln Val Leu Thr Lys Val Ile Phe
785 790 795 800

Arg Arg Asp Ile Ser Phe Lys Leu Thr Ser Lys Leu Pro Ser Gly Asp
805 810 815

Glu Lys Lys Asp Pro Tyr Ala Asp Leu Tyr Val Val Arg Trp Thr Pro
820 825 830

Leu Met Ile Thr Pro Ile Ile Ile Ile Phe Val Asn Ile Ile Gly Ser
835 840 845

Ala Val Ala Phe Ala Lys Val Leu Asp Gly Glu Trp Thr His Trp Leu
850 855 860

Lys Val Ala Gly Gly Val Phe Phe Asn Phe Trp Val Leu Phe His Leu
865 870 875 880

Tyr Pro Phe Ala Lys Gly Ile Leu Gly Lys His Gly Lys Thr Pro Val
885 890 895

Val Val Leu Val Trp Trp Ala Phe Thr Phe Val Ile Thr Ala Val Leu
900 905 910

Tyr Ile Asn Ile Pro His Met His Thr Ser Gly Gly Lys His Thr Thr
915 920 925

Val His Gly His His Gly Lys Lys Leu Val Asp Thr Gly Leu Tyr Gly
930 935 940

Trp Leu His
945

<210> 9
<211> 2844
<212> DNA
<213> Hordeum vulgare

<220>
<221> misc_feature
<223> EU267181 HvCslF6 mRNA to cDNA

<400> 9
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

```
gagaccgacg aaagcggtgc cgccgttgac gaccgccccg tattccgcac cgagaagatc      300
aagggtgtcc tcctccaccc ctaccgggtg ctgattttcg ttcgtctgat cgccttcacg      360
ctgttcgtga tctggcgtat ctcccacaag aacccagacg cgatgtggct gtgggtgaca      420
tccatctgcg gcgagttctg gttcggtttc tcgtggctgc tagatcagct gcccaagctg      480
aaccccatca accgcgtgcc ggacctggcg gtgctgcggc agcgcttcga ccgccccgac      540
ggcacctcca cgctcccggg gctggacatc ttcgtcacca cggccgaccc catcaaggag      600
cccatcctct ccaccgccaa ctcggtgctc tccatcctgg ccgccgacta ccccgtggac      660
cgcaacacat gctacgtctc cgacgacagt ggcatgctgc tcacctacga ggccctggca      720
gagtcctcca agttcgccac gctctgggtg cccttctgcc gcaagcacgg gatcgagccc      780
aggggtccgg agagctactt cgagctcaag tcacaccctt acatggggag agcccaggac      840
gagttcgtca acgaccgccg ccgcgttcgc aaggagtacg acgagttcaa ggccaggatc      900
aacagcctgg agcatgacat caagcagcgc aacgacgggt acaacgccgc cattgcccac      960
agccaaggcg tgccccggcc cacctggatg gcggacggca cccagtggga gggcacatgg     1020
gtcgacgcct ccgagaacca ccgcaggggc gaccacgccg gcatcgtact ggtgctgctg     1080
aaccacccga gccaccgccg gcagacgggc ccgccggcga gcgctgacaa ccccactggac    1140
ttgagcggcg tggatgtgcg tctccccatg ctggtgtacg tgtcccgtga gaagcgcccc     1200
gggcacgacc accagaagaa ggccggtgcc atgaacgcgc ttacccgcgc ctcggcgctg     1260
ctctccaact cccccttcat cctcaacctc gactgcgatc attacatcaa caactcccag     1320
gcccttcgcg ccggcatctg cttcatggtg ggacgggaca gcgacacggt tgccttcgtc     1380
cagttcccgc agcgcttcga gggcgtcgac ccccaccgacc tctacgccaa ccacaaccgc    1440
atcttcttcg acggcaccct ccgtgccctg gacggcatgc agggccccat ctacgtcggc     1500
actgggtgtc tcttccgccg catcaccgtc tacggcttcg acccgccgag gatcaacgtc     1560
ggcggtccct gcttccccag gctcgccggg ctcttcgcca agaccaagta cgagaagccc     1620
gggctcgaga tgaccacggc caaggccaag gccgcgcccg tgcccgccaa gggtaagcac     1680
ggcttcttgc cactgcccaa gaagacgtac ggcaagtcgg acgccttcgt ggacaccatc     1740
ccgcgcgcgt cgcacccgtc gccctacgcc gcggcggctg aggggatcgt ggccgacgag     1800
gcgaccatcg tcgaggcggt gaacgtgacg gccgccgcgt tcgagaagaa gaccggctgg     1860
ggcaaagaga tcggctgggt gtacgacacc gtcacggagg acgtggtcac cggctaccgg     1920
atgcatatca aggggtggcg gtcacgctac tgctccatct acccacacgc cttcatcggc     1980
accgccccca tcaacctcac ggagaggctc ttccaggtgc tccgctggtc cacgggatcc     2040
ctcgagatct tcttctccaa gaacaacccg ctcttcggca gcacatacct ccacccgctg     2100
cagcgcgtcg cctacatcaa catcaccact taccccttca ccgccatctt cctcatcttc     2160
tacaccaccg tgccggcgct atccttcgtc accggccact tcatcgtgca gcgcccgacc     2220
```

accatgttct acgtctacct gggcatcgtg ctatccacgc tgctcgtcat cgccgtgctg 2280

gaggtcaagt gggccggggt cacagtcttc gagtggttca ggaacggcca gttctggatg 2340

acagcaagtt gctccgccta cctcgccgcc gtctgccagg tgctgaccaa ggtgatattc 2400

cggcgggaca tctccttcaa gctcacatcc aagctaccct cgggagacga gaagaaggac 2460

ccctacgccg acctctacgt ggtgcgctgg acgccgctca tgattacacc catcatcatc 2520

atcttcgtca acatcatcgg atccgccgtg gccttcgcca aggttctcga cggcgagtgg 2580

acgcactggc tcaaggtcgc cggcggcgtc ttcttcaact tctgggtgct cttccacctc 2640

tacccettcg ccaagggcat cctggggaag cacggaaaga cgccagtcgt ggtgctcgtc 2700

tggtgggcat tcaccttcgt catcaccgcc gtgctctaca tcaacatccc ccacatgcat 2760

acctcgggag gcaagcacac aacggtgcat ggtcaccatg gcaagaagtt ggtcgacaca 2820

gggctctatg gctggctcca ttga 2844

<210> 10
<211> 947
<212> PRT
<213> Hordeum vulgare

<220>
<221> misc_feature
<223> EU267181 HvCslF6 aa

<400> 10

Met Ala Pro Ala Val Ala Gly Gly Gly Arg Val Arg Ser Asn Glu Pro
1 5 10 15

Val Ala Ala Ala Ala Ala Ala Pro Ala Ala Ser Gly Lys Pro Cys Val
20 25 30

Cys Gly Phe Gln Val Cys Ala Cys Thr Gly Ser Ala Ala Val Ala Ser
35 40 45

Ala Ala Ser Ser Leu Asp Met Asp Ile Val Ala Met Gly Gln Ile Gly
50 55 60

Ala Val Asn Asp Glu Ser Trp Val Gly Val Glu Leu Gly Glu Asp Gly
65 70 75 80

Glu Thr Asp Glu Ser Gly Ala Ala Val Asp Asp Arg Pro Val Phe Arg
85 90 95

Thr Glu Lys Ile Lys Gly Val Leu Leu His Pro Tyr Arg Val Leu Ile
100 105 110

Phe Val Arg Leu Ile Ala Phe Thr Leu Phe Val Ile Trp Arg Ile Ser

```
            115                 120                 125

His Lys Asn Pro Asp Ala Met Trp Leu Trp Val Thr Ser Ile Cys Gly
    130                 135                 140

Glu Phe Trp Phe Gly Phe Ser Trp Leu Leu Asp Gln Leu Pro Lys Leu
145                 150                 155                 160

Asn Pro Ile Asn Arg Val Pro Asp Leu Ala Val Leu Arg Gln Arg Phe
                165                 170                 175

Asp Arg Pro Asp Gly Thr Ser Thr Leu Pro Gly Leu Asp Ile Phe Val
            180                 185                 190

Thr Thr Ala Asp Pro Ile Lys Glu Pro Ile Leu Ser Thr Ala Asn Ser
        195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240

Glu Ser Ser Lys Phe Ala Thr Leu Trp Val Pro Phe Cys Arg Lys His
                245                 250                 255

Gly Ile Glu Pro Arg Gly Pro Glu Ser Tyr Phe Glu Leu Lys Ser His
            260                 265                 270

Pro Tyr Met Gly Arg Ala Gln Asp Glu Phe Val Asn Asp Arg Arg Arg
        275                 280                 285

Val Arg Lys Glu Tyr Asp Glu Phe Lys Ala Arg Ile Asn Ser Leu Glu
    290                 295                 300

His Asp Ile Lys Gln Arg Asn Asp Gly Tyr Asn Ala Ala Ile Ala His
305                 310                 315                 320

Ser Gln Gly Val Pro Arg Pro Thr Trp Met Ala Asp Gly Thr Gln Trp
                325                 330                 335

Glu Gly Thr Trp Val Asp Ala Ser Glu Asn His Arg Arg Gly Asp His
            340                 345                 350

Ala Gly Ile Val Leu Val Leu Leu Asn His Pro Ser His Arg Arg Gln
        355                 360                 365

Thr Gly Pro Pro Ala Ser Ala Asp Asn Pro Leu Asp Leu Ser Gly Val
    370                 375                 380
```

```
Asp Val Arg Leu Pro Met Leu Val Tyr Val Ser Arg Glu Lys Arg Pro
385                 390                 395                 400

Gly His Asp His Gln Lys Lys Ala Gly Ala Met Asn Ala Leu Thr Arg
                405                 410                 415

Ala Ser Ala Leu Leu Ser Asn Ser Pro Phe Ile Leu Asn Leu Asp Cys
            420                 425                 430

Asp His Tyr Ile Asn Asn Ser Gln Ala Leu Arg Ala Gly Ile Cys Phe
        435                 440                 445

Met Val Gly Arg Asp Ser Asp Thr Val Ala Phe Val Gln Phe Pro Gln
    450                 455                 460

Arg Phe Glu Gly Val Asp Pro Thr Asp Leu Tyr Ala Asn His Asn Arg
465                 470                 475                 480

Ile Phe Phe Asp Gly Thr Leu Arg Ala Leu Asp Gly Met Gln Gly Pro
                485                 490                 495

Ile Tyr Val Gly Thr Gly Cys Leu Phe Arg Arg Ile Thr Val Tyr Gly
            500                 505                 510

Phe Asp Pro Pro Arg Ile Asn Val Gly Gly Pro Cys Phe Pro Arg Leu
        515                 520                 525

Ala Gly Leu Phe Ala Lys Thr Lys Tyr Glu Lys Pro Gly Leu Glu Met
    530                 535                 540

Thr Thr Ala Lys Ala Lys Ala Ala Pro Val Pro Ala Lys Gly Lys His
545                 550                 555                 560

Gly Phe Leu Pro Leu Pro Lys Lys Thr Tyr Gly Lys Ser Asp Ala Phe
                565                 570                 575

Val Asp Thr Ile Pro Arg Ala Ser His Pro Ser Pro Tyr Ala Ala Ala
            580                 585                 590

Ala Glu Gly Ile Val Ala Asp Glu Ala Thr Ile Val Glu Ala Val Asn
        595                 600                 605

Val Thr Ala Ala Ala Phe Glu Lys Lys Thr Gly Trp Gly Lys Glu Ile
    610                 615                 620

Gly Trp Val Tyr Asp Thr Val Thr Glu Asp Val Val Thr Gly Tyr Arg
625                 630                 635                 640
```

```
Met His Ile Lys Gly Trp Arg Ser Arg Tyr Cys Ser Ile Tyr Pro His
                645                 650                 655

Ala Phe Ile Gly Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685

Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
705                 710                 715                 720

Tyr Thr Thr Val Pro Ala Leu Ser Phe Val Thr Gly His Phe Ile Val
                725                 730                 735

Gln Arg Pro Thr Thr Met Phe Tyr Val Tyr Leu Gly Ile Val Leu Ser
            740                 745                 750

Thr Leu Leu Val Ile Ala Val Leu Glu Val Lys Trp Ala Gly Val Thr
        755                 760                 765

Val Phe Glu Trp Phe Arg Asn Gly Gln Phe Trp Met Thr Ala Ser Cys
    770                 775                 780

Ser Ala Tyr Leu Ala Ala Val Cys Gln Val Leu Thr Lys Val Ile Phe
785                 790                 795                 800

Arg Arg Asp Ile Ser Phe Lys Leu Thr Ser Lys Leu Pro Ser Gly Asp
                805                 810                 815

Glu Lys Lys Asp Pro Tyr Ala Asp Leu Tyr Val Val Arg Trp Thr Pro
            820                 825                 830

Leu Met Ile Thr Pro Ile Ile Ile Ile Phe Val Asn Ile Ile Gly Ser
        835                 840                 845

Ala Val Ala Phe Ala Lys Val Leu Asp Gly Glu Trp Thr His Trp Leu
    850                 855                 860

Lys Val Ala Gly Gly Val Phe Phe Asn Phe Trp Val Leu Phe His Leu
865                 870                 875                 880

Tyr Pro Phe Ala Lys Gly Ile Leu Gly Lys His Gly Lys Thr Pro Val
                885                 890                 895
```

Val Val Leu Val Trp Trp Ala Phe Thr Phe Val Ile Thr Ala Val Leu
900 905 910

Tyr Ile Asn Ile Pro His Met His Thr Ser Gly Gly Lys His Thr Thr
915 920 925

Val His Gly His His Gly Lys Lys Leu Val Asp Thr Gly Leu Tyr Gly
930 935 940

Trp Leu His
945

<210> 11
<211> 22
<212> DNA
<213> Artificial Sequence

<220>
<223> Forward primer

<400> 11
gccaagacca agtacgagaa gc 22

<210> 12
<211> 23
<212> DNA
<213> Artificial Sequence

<220>
<223> Reverse primer

<400> 12
tgttcttgga gaagaagatc tcg 23

<210> 13
<211> 2279
<212> DNA
<213> Hordeum vulgare

<220>
<221> misc_feature
<223> TR251 HvCslF6 promoter

<400> 13
atcttgtttt tgcgggacag cttacgaatt actttttatt ttgcggtaca gcttacgaat 60

tacttaactt gatggagcag ttcagcaaag aaattgagca aaaatcattt gcagagtcaa 120

ggggtgagct tgtgggcgtc aatcaggtga ttcgtctcct gtccacacat agacgcgcgc 180

gcacacataa gcaagtgagt agtaggtgat tacattaccg tcggcgtgaa gcgttaaaat 240

tgcgtccgtt ctctcccgcc tcctcgtaaa tgctttggga ctcgttgatt gtagcagtgg 300

tagtttatca tgaatgctgg ggctagcgtg cccgcatatg tagttggatc gtcgatgcaa 360

cagcaaggcc ggttaattac tcctccggcc tctccatacc agtgcacggc tcgccacgat 420

cgttttcttg tggtatgttg tccttttgtg tgttgtgtgg cggtgtggtt atagttggag 480
gtcgtgacag aacccattag ttgtccggcg cgcgcgcgtc attgtcctaa caactggcct 540
aatttgcaaa ttgctctaga cctcatgctt gattcctccc gttagttcag ggagctccat 600
cagcacggat tacatgctcc tccccgtccc cgccgtgaat ctctcatcgg atacgcgcgc 660
gacgttcccc gccgtgactc tctcaatctc tcatcggata cgcgcgggac gttcctatag 720
ggacgtcgta acgggcagaa gggcaagcct ccttccggcc aggggaccaa tgcattcctt 780
ctcgtgaagc atttgctcgc caaagccaaa gccaaagcca aagccacaag gggaaaaaga 840
gagagaagga ggcggtagaa ccggaaaggg ctctgcatgt ctacctttcg ctgtgaccaa 900
aattactctt cccttccgtg gccctgctgc atgagcatgg ccctccgctt ttcaaccagc 960
aagacaagaa tatgtcgcaa ttgctcctac tagtgttaaa ccgatggatc aatcctccaa 1020
aatgcatccc ccaaccagac ccaacttgcg cataaacatc aatgtgctaa aaattccatg 1080
gacagaagag agcgccgcat cgcatttgcc ggtgggctgc aaggccacag cggagcaaag 1140
aggcttgtac ggtactcaaa ccagctcact gatgcatggc gatgctacag ggactcacgg 1200
gccacgctgg ctgggagaga gcagcagaaa aggcgcaaga aaagaatctt tctctcaatc 1260
aagggcaaca accaagcaca agctcacaaa ccccccacca agttaagaaa aagaaaaaaa 1320
aatacaggca cttcgcataa caaacaaact aatcatcatc accactcatc gcccccggca 1380
cctttattct acgcccagct cctctctcat tacacttcta acatatataa caatcaatcg 1440
gtggaggagg agggaccaat taattaatgg attagtaata acaattctta tgcgagcaaa 1500
aatatacgta ctcctgtact aataagaatc ggaaatgcaa gctaatcaga gctactgcgt 1560
acgtactagt tttcttgcgc ggggagggca tcacaaatca catgggacac gcaggggcag 1620
gaatatgagt ggaagcaacc tggtaggtag gtgaggtgcg gtgcggtgcg ggcatatgat 1680
aagcatatgg gctgggccga gcatacacgc gtacatgcat tgcatttgca tacaccgaag 1740
gaagtgcttg gcaccaccta catataaacg cacccgcaca ccgctcaaac acaatttaca 1800
acccgcacac agctccaacc cgttggttgg atagctcagc aagacaagcg agctgagcag 1860
agctttgctt tgcttgaacc ccctaccgac acttcttgtg catcccagcc cataaatccc 1920
ccacgtactt tactcgtcat ttctcgcacc tcctcctccc cctccccctc gcctacattc 1980
attcattgct tccttttctc tctccctgca catgctaaag cctgcctcgg ccattgcctg 2040
agcctgccat tgttggacct tggacctgtt ctccttgtcg taaaggcaaa ggagagcgcg 2100
tgcattgagg acgacggcca tggcgccagc ggtggccgga gggggccgcg tgcggagcaa 2160
tgagccggtt gctgctgctg ccgccgcgcc ggcggccagc ggcaagccct gcgtgtgcgg 2220
cttccaggtt tgcgcctgca cggggtcggc cgcggtggcc tccgccgcct cgtcgctgg 2279

<210> 14
<211> 2271

<212> DNA
<213> Hordeum vulgare

<220>
<221> misc_feature
<223> CDC-Bold HvCslF6 promoter

<400> 14
atcttgttta tgcgggacag cttacgaatt actttttatt ttgcagtaca gcttacgaat 60

tacttaactt gatggagcag ttcagcaaag aaattgagca aaaatcattt gcagagtcaa 120

ggggtgagct tgtgggcgtc aatcaggtga ttcgtctcct gtccacacat agacgcgcgc 180

gcacacataa gcaagtgagt agtaggtgat tacattaccg tcggcgtgaa gcgttaaaat 240

tgcgtccgtt ctctcccgcc tcctcgtaaa tgctttggga ctcgttgatt gtagcagtgg 300

tagtttatca tgaatgctgg ggctagcgtg cccgcatatg tagttggatc gtcgatgcaa 360

cagcaaggcc ggttaattac tcctccggcc tctccatacc agtgcacggc tcgccacgat 420

cgttttcttg tggtatgttg tccttttgtg tgttgtgtgg cggtgtggtt atagttggag 480

gtcgtgacag aacccattag ttgtccggcg cgcgcgcgtc attgtcctaa caactggcct 540

aatttgcaaa ttgctctaga cctcatgctt gattcctccc gttagttcag ggagctccat 600

cagcacggat tacgtgctcc tccccgtccc cgccgtgaat ctctcatcgg atacgcgcgc 660

gacgttcccc gccgtgactc tctcatcgga tacgcgcggg acgttcctat agggacgtcg 720

taacgggcag aagggcaagc ctccttccgg ccaggggacc aatgcattcc ttctcgtgaa 780

gcatttgctc gccaaagcca aagccaaagc caaagccaca aggggaaaaa gagagagaag 840

gaggcggtag aaccggaaag ggctctgcat gtctaccttt cgctgtgacc aaaattactc 900

ttcccttccg tggccctgct gcatgagcat ggccctccgc ttttcaacca gcaagacaag 960

aatatgtcgc aattgctcct actagtgtta aaccgatgga tcaatcctcc aaaatgcatc 1020

ccccaaccag acccaacttg cgcataaaca tcaatgtgct aaaaattcca tggacagaag 1080

agagcgccgc atcgcatttg ccggtgggct gcaaggccac agcggagcaa agaggcttgt 1140

acggtactca aaccagctca ctgatgcatg gcgatgctac agggactcac gggccacgct 1200

ggctgggaga gagcagcaga aaaggcgcaa gaaaagaatc tttctctcaa tcaagggcaa 1260

caaccaagca caagctcaca aaccccccac caagttaaga aaaagaaaaa aaaatacagg 1320

cacttcgcat aacaaacaaa ctaatcatca tcaccactca tcgcccccgg cacctttatt 1380

ctacgcccag ctcctctctc attacacttc taacatatat aacaatcaat cggtggagga 1440

ggagggacca attaattaat ggattagtaa taacaattct tatgcgagca aaaatatacg 1500

tactcctgta ctaataagaa tcggaaatgc aagctaatca gagctactgc gtacgtacta 1560

gttttcttgc gcggggaggg catcacaaat cacatgggac acgcaggggc aggaatatga 1620

gtggaagcaa cctggtaggt aggtgaggtg cggtgcggtg cgggcatatg ataagcatat 1680

gggctgggcc gagcatacac gcgtacatgc attgcatttg catacaccga aggaagtgct 1740

tggcaccacc tacatataaa cgcacccgca caccgctcaa acacaattta caacccgcac 1800

acagctccaa cccgttggtt ggatagctca gcaagacaag cgagctgagc agagctttgc 1860

tttgcttgaa ccccctaccg acacttcttg tgcatcccag cccataaatc ccccacgtac 1920

tttactcgtc atttctcgca cctcctcctc cccctccccc tcgcctacat tcattcattg 1980

cttccttttc tctctccctg cccatgctaa agcctgcctc ggccattgcc tgagcctgcc 2040

attgttggac cttggacctg ttctccttgt cgtaaaggca aaggagagcg cgtgcattga 2100

ggacgacggc catggcgcca gcggtggccg gaggggccg cgtgcggagc aatgagccgg 2160

ttgctgctgc tgccgccgcg ccggcggcca gcggcaagcc ctgcgtgtgc ggcttccagg 2220

tttgcgcctg cacggggtcg gccgcggtgg cctccgccgc ctcgtcgctg g 2271

<210> 15
<211> 5140
<212> DNA
<213> Hordeum vulgare

<220>
<221> misc_feature
<223> Sachiho Golden HvCslF6 gene genomic DNA

<400> 15
atggcgccag cggtggccgg aggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccggtac gtcctgctcc cacaactaaa cagaaactcc 360

ctatatctgc gtcacactca acaattaatc caactaagtc tctctactac tctagtattt 420

atttttactc tctatctgca caacaagcgc tactacaatt aacccaacaa gcaccacgcc 480

aggttgacag tcaggataat ttgatcttga ccggagtaag tactagtact aggtcggtgt 540

taatcagagt aattattaca ctagttaatt aaaatttgag taatccgaga caggtgcacg 600

ttagggccgg gccaatgatg gctcgaatcc acccaaaata gcgcgtcccg gtgtgggctg 660

tcggctcggt gcttcttcct tccattttac tagtcgcagt cactgcagct tgggcccacg 720

ggaggggacg ttagccgttg ggcctgcctg gcaggtgggc cccggtggcc accctggcgg 780

ctcataaatc cttgctactt tggagctgta atggacgctc tgcaatagca ataggaatcc 840

gaggtgaaac gacgacagtg ggcatggcat ggcttgcatg tgaatccaag ccacatcatt 900

aaaagcatcc tccctgggca cgtcgcggtg agaaagttgg ataaactttt gggggttcgg 960

acaagatgag aaaaagcaag taacatgtcc ctttttggc accgaagaaa tcctatgtac 1020

ggcgagtttt ttctgcatct agttatgggt agatgtacgt tagcttttgt tgagcgtttt 1080

atgtgctaca tatatggaga aaaagagaaa aatattatca tgtcatgtca tgccatgcca 1140

tgagaaggag gagaagaaaa ataagtgttg ccaccgctcg aatgcctttt ttttctttcg 1200

gaaggatgcg taagtcatgt tggcaccgag aaaagccata ttaaagtggc agagttacaa 1260

ctcagaataa atgcgggtgt tacaaaaaca ctaggatatg tgaagggcac tcggcacaac 1320

actttaagac tacacaattg aaaaagtgta ctctctgtat ctaaataatt ataattgaaa 1380

agaattaatc tatatatgaa agtagtaatg attagcggta gaaggttcca acgacttttt 1440

tggcgccaat agcaagaaga aagaaaaaga aaaaaatctt tactgtacag tataacgaga 1500

aaagaggccc attaatagag caacgaatcc agcggcacca cctctggcgg tcacgtccat 1560

gccctcgcac gacggatgag gcccgggggg tcctactgac agccgaagca tgtcggtgct 1620

caaacacggc gccgtttgct gccaagtgtg ccagctcgca ctcattgact tgccagctct 1680

ctccttggtt gtcaatgaga acatgatgcc ttttggcatt tgcaaactta ttaaaactag 1740

ctgtcgtccg atagggaaaa gaaaagaaaa gaaaagaata agaaaaaaag gacaaagaga 1800

aaagatgaac atggcgcatg ttccctccaa taattgcagg caccaacact gggtcgatta 1860

atccaacaac aatattttac tataccagac gagagtacag tagtcgggtg atgatggact 1920

gtaactgact gagtatgaat gactgtaatg cagggtgctg attttcgttc gtctgatcgc 1980

cttcacgctg ttcgtgatct ggcgtatctc ccacaagaac ccagacgcga tgtggctgtg 2040

ggtgacatcc atctgcggcg agttctggtt cggtttctcg tggctgctgg atcagctgcc 2100

caagctgaac cccatcaacc gcgtgccgga cctggcggtg ctgcggcagc gcttcgaccg 2160

ccccgacggc acctccacgc tcccggggct ggacatcttc gtcaccacgg ccgaccccat 2220

caaggagccc atcctctcca ccgccaactc ggtgctctcc atcctggccg ccgactaccc 2280

cgtggaccgc aacacatgct acgtctccga cgacagtggc atgctgctca cctacgaggc 2340

cctggcagag tcctccaagt tcgccacgct ctgggtgccc ttctgccgca agcacgggat 2400

cgagcccagg ggtccggaga gctacttcga gctcaagtca caccccttaca tggggagagc 2460

ccaggacgag ttcgtcaacg accgccgccg cgttcgcaag gagtacgacg agttcaaggc 2520

caggatcaac agcctggagc atgacatcaa gcagcgcaac gacgggtaca acgccgccat 2580

tgcccacagc caaggcgtgc cccggcccac ctggatggcg gacggcaccc agtgggaggg 2640

cacatgggtc gacgcctccg agaaccaccg caggggcgac cacgccggca tcgtactggt 2700

cagtatccat ccatctttct gctgcttata ttactcttag gttactctta tcgtctcttt 2760

cctatactgt acatgcatgc atgctgctat tcttggaatc gtggttggtt actactccac 2820

catgcaaaaa taacaagaag aggaatcttg gttagttagg gcctcgttgt tatattagtg 2880

gccatctgat gtgatgcctg ccggctgtgc ccatccatat ccatggaaga tttcgacaga 2940

atcgacgtgg tgatagtcga gagtgcaacc accacccaga gccagccaag cacatgcatg 3000
cttctcttct cgtctcgtcg tgtggccagc agcgcattca tgctattgct gtgacgaggg 3060
aggaatggtg gttggggtgg tcctttcccc ccgacagcac tacagcctcc actttatgac 3120
ccatttaatt caccggccct gctttgttgt aaccgccttc tcacctcaat caatcattca 3180
ttcattcata agtttactca ctctttgtta ctactcgaac cactaatcag gaaggagtag 3240
gagtaatgca gatttactat taacagttaa aggagtaaaa agaaggaagc acaattacag 3300
aaccttgttt ttttttacta ctgtacgtaa ggtgtaagaa tggagtgctg acagagaatg 3360
gatgcaggtg ctgctgaacc acccgagcca ccgccggcag acgggcccgc cggcgagcgc 3420
tgacaaccca ctggacttga gcggcgtgga tgtgcgtctc cccatgctgg tgtacgtgtc 3480
ccgtgagaag cgccccgggc acgaccacca gaagaaggcc ggtgccatga acgcgcttac 3540
ccgcgcctcg gcgctgctct ccaactcccc cttcatcctc aacctcgact gcgatcatta 3600
catcaacaac tcccaggccc ttcgcgccgg catctgcttc atggtgggac gggacagcga 3660
cacggttgcc ttcgtccagt tcccgcagcg cttcgagggc gtcgacccca ccgacctcta 3720
cgccaaccac aaccgcatct tcttcgacgg caccctccgt gccctggacg gcatgcaggg 3780
ccccatctac gtcggcactg ggtgtctctt ccgccgcatc accgtctacg gcttcgaccc 3840
gccgaggatc aacgtcggcg gtccctgctt ccccaggctc gccgggctct tcgccaagac 3900
caagtacgag aagcccgggc tcgagatgac cacggccaag gccaaggccg cgcccgtgcc 3960
cgccaagggt aagcacggct tcttgccact gcccaagaag acgtacggca agtcggacgc 4020
cttcgtggac accatcccgc gcgcgtcgca cccgtcgccc tacgccgcgg cggctgaggg 4080
gatcgtggcc gacgaggcga ccatcgtcga ggcggtgaac gtgacggccg ccgcgttcga 4140
gaagaagacc ggctggggca aagagatcgg ctgggtgtac gacaccgtca cggaggacgt 4200
ggtcaccggc taccggatgc atatcaaggg gtggcggtca cgctactgct ccatctaccc 4260
acacgccttc atcggcaccg cccccatcaa cctcacggag aggctcttcc aggtgctccg 4320
ctggtccacg ggatccctcg agatcttctt ctccaagaac aacccgctct tcggcagcac 4380
atacctccac ccgctgcagc gcgtcgccta catcaacatc accacttacc ccttcaccgc 4440
catcttcctc atcttctaca ccaccgtgcc ggcgctatcc ttcgtcaccg gccacttcat 4500
cgtgcagcgc ccgaccacca tgttctacgt ctacctgggc atcgtgctat ccacgctgct 4560
cgtcatcgcc gtgctggagg tcaagtgggc cggggtcaca gtcttcgagt ggttcaggaa 4620
cggccagttc tggatgacag caagttgctc cgcctacctc gccgccgtct gccaggtgct 4680
gaccaaggtg atattccggc gggacatctc cttcaagctc acatccaagc taccctcggg 4740
agacgagaag aaggacccct acgccgacct ctacgtggtg cgctggacgc cgctcatgat 4800
tacacccatc atcatcatct tcgtcaacat catcggatcc gccgtggcct tcgccaaggt 4860
tctcgacggc gagtggacgc actggctcaa ggtcgccggc ggcgtcttct tcaacttctg 4920

```
ggtgctcttc cacctctacc ccttcgccaa gggcatcctg gggaagcacg gaaagacgcc     4980

agtcgtggtg ctcgtctggt gggcattcac cttcgtcatc accgccgtgc tctacatcaa     5040

catcccccac atgcatacct cgggaggcaa gcacacaacg gtgcatggtc accatggcaa     5100

gaagttggtc gacacagggc tctatggctg gctccattga                           5140


<210>  16
<211>  2844
<212>  DNA
<213>  Hordeum vulgare


<220>
<221>  misc_feature
<223>  Sachiho Golden HvCslF6 gene cDNA

<400>  16
atggcgccag cggtggccgg agggggccgc gtgcggagca atgagccggt tgctgctgct       60

gccgccgcgc cggcggccag cggcaagccc tgcgtgtgcg gcttccaggt ttgcgcctgc      120

acggggtcgg ccgcggtggc ctccgccgcc tcgtcgctgg acatggacat cgtggccatg      180

gggcagatcg gcgccgtcaa cgacgagagc tgggtgggcg tggagctcgg cgaagatggc      240

gagaccgacg aaagcggtgc cgccgttgac gaccgccccg tattccgcac cgagaagatc      300

aagggtgtcc tcctccaccc ctaccgggtg ctgattttcg ttcgtctgat cgccttcacg      360

ctgttcgtga tctggcgtat ctcccacaag aacccagacg cgatgtggct gtgggtgaca      420

tccatctgcg gcgagttctg gttcggtttc tcgtggctgc tggatcagct gcccaagctg      480

aaccccatca accgcgtgcc ggacctggcg gtgctgcggc agcgcttcga ccgccccgac      540

ggcacctcca cgctcccggg gctggacatc ttcgtcacca cggccgaccc catcaaggag      600

cccatcctct ccaccgccaa ctcggtgctc tccatcctgg ccgccgacta ccccgtggac      660

cgcaacacat gctacgtctc cgacgacagt ggcatgctgc tcacctacga ggccctggca      720

gagtcctcca agttcgccac gctctgggtg cccttctgcc gcaagcacgg gatcgagccc      780

aggggtccgg agagctactt cgagctcaag tcacaccctt acatggggag agcccaggac      840

gagttcgtca acgaccgccg ccgcgttcgc aaggagtacg acgagttcaa ggccaggatc      900

aacagcctgg agcatgacat caagcagcgc aacgacgggt acaacgccgc cattgcccac      960

agccaaggcg tgccccggcc cacctggatg gcggacggca cccagtggga gggcacatgg     1020

gtcgacgcct ccgagaacca ccgcaggggc gaccacgccg gcatcgtact ggtgctgctg     1080

aaccacccga gccaccgccg gcagacgggc ccgccggcga gcgctgacaa ccccactggac     1140

ttgagcggcg tggatgtgcg tctccccatg ctggtgtacg tgtcccgtga gaagcgcccc     1200

gggcacgacc accagaagaa ggccggtgcc atgaacgcgc ttacccgcgc ctcggcgctg     1260

ctctccaact ccccccttcat cctcaacctc gactgcgatc attacatcaa caactcccag     1320
```

gcccttcgcg ccggcatctg cttcatggtg ggacgggaca gcgacacggt tgccttcgtc 1380

cagttcccgc agcgcttcga gggcgtcgac cccaccgacc tctacgccaa ccacaaccgc 1440

atcttcttcg acggcaccct ccgtgccctg gacggcatgc agggccccat ctacgtcggc 1500

actgggtgtc tcttccgccg catcaccgtc tacggcttcg acccgccgag gatcaacgtc 1560

ggcggtccct gcttccccag gctcgccggg ctcttcgcca agaccaagta cgagaagccc 1620

gggctcgaga tgaccacggc caaggccaag gccgcgcccg tgcccgccaa gggtaagcac 1680

ggcttcttgc cactgcccaa gaagacgtac ggcaagtcgg acgccttcgt ggacaccatc 1740

ccgcgcgcgt cgcacccgtc gccctacgcc gcggcggctg aggggatcgt ggccgacgag 1800

gcgaccatcg tcgaggcggt gaacgtgacg gccgccgcgt tcgagaagaa gaccggctgg 1860

ggcaaagaga tcggctgggt gtacgacacc gtcacggagg acgtggtcac cggctaccgg 1920

atgcatatca aggggtggcg gtcacgctac tgctccatct acccacacgc cttcatcggc 1980

accgccccca tcaacctcac ggagaggctc ttccaggtgc tccgctggtc cacgggatcc 2040

ctcgagatct tcttctccaa gaacaacccg ctcttcggca gcacatacct ccacccgctg 2100

cagcgcgtcg cctacatcaa catcaccact taccccttca ccgccatctt cctcatcttc 2160

tacaccaccg tgccggcgct atccttcgtc accggccact tcatcgtgca gcgcccgacc 2220

accatgttct acgtctacct gggcatcgtg ctatccacgc tgctcgtcat cgccgtgctg 2280

gaggtcaagt gggccggggt cacagtcttc gagtggttca ggaacggcca gttctggatg 2340

acagcaagtt gctccgccta cctcgccgcc gtctgccagg tgctgaccaa ggtgatattc 2400

cggcgggaca tctccttcaa gctcacatcc aagctaccct cgggagacga gaagaaggac 2460

ccctacgccg acctctacgt ggtgcgctgg acgccgctca tgattacacc catcatcatc 2520

atcttcgtca acatcatcgg atccgccgtg gccttcgcca aggttctcga cggcgagtgg 2580

acgcactggc tcaaggtcgc cggcggcgtc ttcttcaact tctgggtgct cttccacctc 2640

taccccttcg ccaagggcat cctggggaag cacggaaaga cgccagtcgt ggtgctcgtc 2700

tggtgggcat tcaccttcgt catcaccgcc gtgctctaca tcaacatccc ccacatgcat 2760

acctcgggag gcaagcacac aacggtgcat ggtcaccatg gcaagaagtt ggtcgacaca 2820

gggctctatg gctggctcca ttga 2844

<210> 17
<211> 947
<212> PRT
<213> Hordeum vulgare

<220>
<221> misc_feature
<223> Sachiho Golden HvCslF6 aa

<400> 17

Met Ala Pro Ala Val Ala Gly Gly Gly Arg Val Arg Ser Asn Glu Pro
1               5                   10                  15

Val Ala Ala Ala Ala Ala Ala Pro Ala Ala Ser Gly Lys Pro Cys Val
            20                  25                  30

Cys Gly Phe Gln Val Cys Ala Cys Thr Gly Ser Ala Ala Val Ala Ser
        35                  40                  45

Ala Ala Ser Ser Leu Asp Met Asp Ile Val Ala Met Gly Gln Ile Gly
    50                  55                  60

Ala Val Asn Asp Glu Ser Trp Val Gly Val Glu Leu Gly Glu Asp Gly
65                  70                  75                  80

Glu Thr Asp Glu Ser Gly Ala Ala Val Asp Asp Arg Pro Val Phe Arg
                85                  90                  95

Thr Glu Lys Ile Lys Gly Val Leu Leu His Pro Tyr Arg Val Leu Ile
            100                 105                 110

Phe Val Arg Leu Ile Ala Phe Thr Leu Phe Val Ile Trp Arg Ile Ser
        115                 120                 125

His Lys Asn Pro Asp Ala Met Trp Leu Trp Val Thr Ser Ile Cys Gly
    130                 135                 140

Glu Phe Trp Phe Gly Phe Ser Trp Leu Leu Asp Gln Leu Pro Lys Leu
145                 150                 155                 160

Asn Pro Ile Asn Arg Val Pro Asp Leu Ala Val Leu Arg Gln Arg Phe
                165                 170                 175

Asp Arg Pro Asp Gly Thr Ser Thr Leu Pro Gly Leu Asp Ile Phe Val
            180                 185                 190

Thr Thr Ala Asp Pro Ile Lys Glu Pro Ile Leu Ser Thr Ala Asn Ser
        195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240

Glu Ser Ser Lys Phe Ala Thr Leu Trp Val Pro Phe Cys Arg Lys His
                245                 250                 255

Gly Ile Glu Pro Arg Gly Pro Glu Ser Tyr Phe Glu Leu Lys Ser His

```
            260                 265                 270

Pro Tyr Met Gly Arg Ala Gln Asp Glu Phe Val Asn Asp Arg Arg Arg
        275                 280                 285

Val Arg Lys Glu Tyr Asp Glu Phe Lys Ala Arg Ile Asn Ser Leu Glu
    290                 295                 300

His Asp Ile Lys Gln Arg Asn Asp Gly Tyr Asn Ala Ala Ile Ala His
305                 310                 315                 320

Ser Gln Gly Val Pro Arg Pro Thr Trp Met Ala Asp Gly Thr Gln Trp
                325                 330                 335

Glu Gly Thr Trp Val Asp Ala Ser Glu Asn His Arg Arg Gly Asp His
            340                 345                 350

Ala Gly Ile Val Leu Val Leu Leu Asn His Pro Ser His Arg Arg Gln
        355                 360                 365

Thr Gly Pro Pro Ala Ser Ala Asp Asn Pro Leu Asp Leu Ser Gly Val
    370                 375                 380

Asp Val Arg Leu Pro Met Leu Val Tyr Val Ser Arg Glu Lys Arg Pro
385                 390                 395                 400

Gly His Asp His Gln Lys Lys Ala Gly Ala Met Asn Ala Leu Thr Arg
                405                 410                 415

Ala Ser Ala Leu Leu Ser Asn Ser Pro Phe Ile Leu Asn Leu Asp Cys
            420                 425                 430

Asp His Tyr Ile Asn Asn Ser Gln Ala Leu Arg Ala Gly Ile Cys Phe
        435                 440                 445

Met Val Gly Arg Asp Ser Asp Thr Val Ala Phe Val Gln Phe Pro Gln
    450                 455                 460

Arg Phe Glu Gly Val Asp Pro Thr Asp Leu Tyr Ala Asn His Asn Arg
465                 470                 475                 480

Ile Phe Phe Asp Gly Thr Leu Arg Ala Leu Asp Gly Met Gln Gly Pro
                485                 490                 495

Ile Tyr Val Gly Thr Gly Cys Leu Phe Arg Arg Ile Thr Val Tyr Gly
            500                 505                 510

Phe Asp Pro Pro Arg Ile Asn Val Gly Gly Pro Cys Phe Pro Arg Leu
        515                 520                 525
```

```
Ala Gly Leu Phe Ala Lys Thr Lys Tyr Glu Lys Pro Gly Leu Glu Met
    530                 535                 540

Thr Thr Ala Lys Ala Lys Ala Ala Pro Val Pro Ala Lys Gly Lys His
545                 550                 555                 560

Gly Phe Leu Pro Leu Pro Lys Lys Thr Tyr Gly Lys Ser Asp Ala Phe
                565                 570                 575

Val Asp Thr Ile Pro Arg Ala Ser His Pro Ser Pro Tyr Ala Ala Ala
            580                 585                 590

Ala Glu Gly Ile Val Ala Asp Glu Ala Thr Ile Val Glu Ala Val Asn
        595                 600                 605

Val Thr Ala Ala Ala Phe Glu Lys Lys Thr Gly Trp Gly Lys Glu Ile
    610                 615                 620

Gly Trp Val Tyr Asp Thr Val Thr Glu Asp Val Val Thr Gly Tyr Arg
625                 630                 635                 640

Met His Ile Lys Gly Trp Arg Ser Arg Tyr Cys Ser Ile Tyr Pro His
                645                 650                 655

Ala Phe Ile Gly Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685

Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
705                 710                 715                 720

Tyr Thr Thr Val Pro Ala Leu Ser Phe Val Thr Gly His Phe Ile Val
                725                 730                 735

Gln Arg Pro Thr Thr Met Phe Tyr Val Tyr Leu Gly Ile Val Leu Ser
            740                 745                 750

Thr Leu Leu Val Ile Ala Val Leu Glu Val Lys Trp Ala Gly Val Thr
        755                 760                 765

Val Phe Glu Trp Phe Arg Asn Gly Gln Phe Trp Met Thr Ala Ser Cys
    770                 775                 780
```

Ser Ala Tyr Leu Ala Ala Val Cys Gln Val Leu Thr Lys Val Ile Phe
785 790 795 800

Arg Arg Asp Ile Ser Phe Lys Leu Thr Ser Lys Leu Pro Ser Gly Asp
805 810 815

Glu Lys Lys Asp Pro Tyr Ala Asp Leu Tyr Val Val Arg Trp Thr Pro
820 825 830

Leu Met Ile Thr Pro Ile Ile Ile Ile Phe Val Asn Ile Ile Gly Ser
835 840 845

Ala Val Ala Phe Ala Lys Val Leu Asp Gly Glu Trp Thr His Trp Leu
850 855 860

Lys Val Ala Gly Gly Val Phe Phe Asn Phe Trp Val Leu Phe His Leu
865 870 875 880

Tyr Pro Phe Ala Lys Gly Ile Leu Gly Lys His Gly Lys Thr Pro Val
885 890 895

Val Val Leu Val Trp Trp Ala Phe Thr Phe Val Ile Thr Ala Val Leu
900 905 910

Tyr Ile Asn Ile Pro His Met His Thr Ser Gly Gly Lys His Thr Thr
915 920 925

Val His Gly His His Gly Lys Lys Leu Val Asp Thr Gly Leu Tyr Gly
930 935 940

Trp Leu His
945

<210> 18
<211> 5140
<212> DNA
<213> Hordeum vulgare

<220>
<221> misc_feature
<223> KM27 bgl gene genomic DNA

<400> 18
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccggtac gtcctgctcc cacaactaaa cagaaactcc 360

ctatatctgc gtcacactca acaattaatc caactaagtc tctctactac tctagtattt 420

atttttactc tctatctgca caacaagcgc tactacaatt aacccaacaa gcaccacgcc 480

aggttgacag tcaggataat ttgatcttga ccggagtaag tactagtact aggtcggtgt 540

taatcagagt aattattaca ctagttaatt aaaatttgag taatccgaga caggtgcacg 600

ttagggccgg gccaatgatg gctcgaatcc acccaaaata gcgcgtcccg gtgtgggctg 660

tcggctcggt gcttcttcct tccattttac tagtcgcagt cactgcagct tgggcccacg 720

ggaggggacg ttagccgttg ggcctgcctg gcaggtgggc cccggtggcc accctggcgg 780

ctcataaatc cttgctactt tggagctgta atggacgctc tgcaatagca ataggaatcc 840

gaggtgaaac gacgacagtg ggcatggcat ggcttgcatg tgaatccaag ccacatcatt 900

aaaagcatcc tccctgggca cgtcgcggtg agaaagttgg ataaactttt gggggttcgg 960

acaagatgag aaaaagcaag taacatgtcc ctttttggc accgaagaaa tcctatgtac 1020

ggcgagtttt ttctgcatct agttatgggt agatgtacgt tagcttttgt tgagcgtttt 1080

atgtgctaca tatatggaga aaaagagaaa aatattatca tgtcatgtca tgccatgcca 1140

tgagaaggag gagaagaaaa ataagtgttg ccaccgctcg aatgcctttt ttttctttcg 1200

gaaggatgcg taagtcatgt tggcaccgag aaaagccata ttaaagtggc agagttacaa 1260

ctcagaataa atgcgggtgt tacaaaaaca ctaggatatg tgaagggcac tcggcacaac 1320

actttaagac tacacaattg aaaaagtgta ctctctgtat ctaaataatt ataattgaaa 1380

agaattaatc tatatatgaa agtagtaatg attagcggta gaaggttcca acgacttttt 1440

tggcgccaat agcaagaaga aagaaaaaga aaaaaatctt tactgtacag tataacgaga 1500

aaagaggccc attaatagag caacgaatcc agcggcacca cctctggcgg tcacgtccat 1560

gccctcgcac gacggatgag gcccgggggg tcctactgac agccgaagca tgtcggtgct 1620

caaacacggc gccgtttgct gccaagtgtg ccagctcgca ctcattgact tgccagctct 1680

ctccttggtt gtcaatgaga acatgatgcc ttttggcatt tgcaaactta ttaaaactag 1740

ctgtcgtccg atagggaaaa gaaaagaaaa gaaaagaata agaaaaaaag gacaaagaga 1800

aaagatgaac atggcgcatg ttccctccaa taattgcagg caccaacact gggtcgatta 1860

atccaacaac aatattttac tataccagac gagagtacag tagtcgggtg atgatggact 1920

gtaactgact gagtatgaat gactgtaatg cagggtgctg attttcgttc gtctgatcgc 1980

cttcacgctg ttcgtgatct ggcgtatctc ccacaagaac ccagacgcga tgtggctgtg 2040

ggtgacatcc atctgcggcg agttctggtt cggtttctcg tggctgctgg atcagctgcc 2100

caagctgaac cccatcaacc gcgtgccgga cctggcggtg ctgcggcagc gcttcgaccg 2160

ccccgacggc acctccacgc tcccggggct ggacatcttc gtcaccacgg ccgaccccat 2220

caaggagccc atcctctcca ccgccaactc ggtgctctcc atcctggccg ccgactaccc 2280

cgtggaccgc aacacatgct acgtctccga cgacagtggc atgctgctca cctacgaggc 2340

cctggcagag tcctccaagt tcgccacgct ctgggtgccc ttctaccgca agcacgggat 2400

cgagcccagg ggtccggaga gctacttcga gctcaagtca caccccttaca tggggagagc 2460

ccaggacgag ttcgtcaacg accgccgccg cgttcgcaag gagtacgacg agttcaaggc 2520

caggatcaac agcctggagc atgacatcaa gcagcgcaac gacgggtaca acgccgccat 2580

tgcccacagc caaggcgtgc cccggcccac ctggatggcg gacggcaccc agtgggaggg 2640

cacatgggtc gacgcctccg agaaccaccg caggggcgac cacgccggca tcgtactggt 2700

cagtatccat ccatctttct gctgcttata ttactcttag gttactctta tcgtctcttt 2760

cctatactgt acatgcatgc atgctgctat tcttggaatc gtggttggtt actactccac 2820

catgcaaaaa taacaagaag aggaatcttg gttagttagg gcctcgttgt tatattagtg 2880

gccatctgat gtgatgcctg ccggctgtgc ccatccatat ccatggaaga tttcgacaga 2940

atcgacgtgg tgatagtcga gagtgcaacc accacccaga gccagccaag cacatgcatg 3000

cttctcttct cgtctcgtcg tgtggccagc agcgcattca tgctattgct gtgacgaggg 3060

aggaatggtg gttggggtgg tcctttcccc ccgacagcac tacagcctcc actttatgac 3120

ccatttaatt caccggccct gctttgttgt aaccgccttc tcacctcaat caatcattca 3180

ttcattcata agtttactca ctctttgtta ctactcgaac cactaatcag gaaggagtag 3240

gagtaatgca gatttactat taacagttaa aggagtaaaa agaaggaagc acaattacag 3300

aaccttgttt ttttttacta ctgtacgtaa ggtgtaagaa tggagtgctg acagagaatg 3360

gatgcaggtg ctgctgaacc acccgagcca ccgccggcag acgggcccgc cggcgagcgc 3420

tgacaaccca ctggacttga gcggcgtgga tgtgcgtctc cccatgctgg tgtacgtgtc 3480

ccgtgagaag cgcccccgggc acgaccacca gaagaaggcc ggtgccatga acgcgcttac 3540

ccgcgcctcg gcgctgctct ccaactcccc cttcatcctc aacctcgact gcgatcatta 3600

catcaacaac tcccaggccc ttcgcgccgg catctgcttc atggtgggac gggacagcga 3660

cacggttgcc ttcgtccagt tcccgcagcg cttcgagggc gtcgacccca ccgacctcta 3720

cgccaaccac aaccgcatct tcttcgacgg caccctccgt gccctggacg gcatgcaggg 3780

ccccatctac gtcggcactg ggtgtctctt ccgccgcatc accgtctacg gcttcgaccc 3840

gccgaggatc aacgtcggcg gtccctgctt ccccaggctc gccgggctct tcgccaagac 3900

caagtacgag aagcccgggc tcgagatgac cacggccaag gccaaggccg cgcccgtgcc 3960

cgccaagggt aagcacggct tcttgccact gcccaagaag acgtacggca agtcggacgc 4020

cttcgtggac accatcccgc gcgcgtcgca cccgtcgccc tacgccgcgg cggctgaggg 4080

gatcgtggcc gacgaggcga ccatcgtcga ggcggtgaac gtgacggccg ccgcgttcga 4140

gaagaagacc ggctggggca aagagatcgg ctgggtgtac gacaccgtca cggaggacgt 4200

ggtcaccggc taccggatgc atatcaaggg gtggcggtca cgctactgct ccatctaccc 4260

acacgccttc atcggcaccg cccccatcaa cctcacggag aggctcttcc aggtgctccg 4320

ctggtccacg ggatccctcg agatcttctt ctccaagaac aacccgctct tcggcagcac 4380

atacctccac ccgctgcagc gcgtcgccta catcaacatc accacttacc ccttcaccgc 4440

catcttcctc atcttctaca ccaccgtgcc ggcgctatcc ttcgtcaccg gccacttcat 4500

cgtgcagcgc ccgaccacca tgttctacgt ctacctgggc atcgtgctat ccacgctgct 4560

cgtcatcgcc gtgctggagg tcaagtgggc cggggtcaca gtcttcgagt ggttcaggaa 4620

cggccagttc tggatgacag caagttgctc cgcctacctc gccgccgtct gccaggtgct 4680

gaccaaggtg atattccggc gggacatctc cttcaagctc acatccaagc taccctcggg 4740

agacgagaag aaggacccct acgccgacct ctacgtggtg cgctggacgc cgctcatgat 4800

tacacccatc atcatcatct tcgtcaacat catcggatcc gccgtggcct tcgccaaggt 4860

tctcgacggc gagtggacgc actggctcaa ggtcgccggc ggcgtcttct tcaacttctg 4920

ggtgctcttc cacctctacc ccttcgccaa gggcatcctg gggaagcacg gaaagacgcc 4980

agtcgtggtg ctcgtctggt gggcattcac cttcgtcatc accgccgtgc tctacatcaa 5040

catcccccac atgcatacct cgggaggcaa gcacacaacg gtgcatggtc accatggcaa 5100

gaagttggtc gacacagggc tctatggctg gctccattga 5140

<210> 19
<211> 2844
<212> DNA
<213> Hordeum vulgare

<220>
<221> misc_feature
<223> KM27 bgl gene cDNA

<400> 19
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccgggtg ctgattttcg ttcgtctgat cgccttcacg 360

ctgttcgtga tctggcgtat ctcccacaag aacccagacg cgatgtggct gtgggtgaca 420

tccatctgcg gcgagttctg gttcggtttc tcgtggctgc tggatcagct gcccaagctg 480

aaccccatca accgcgtgcc ggacctggcg gtgctgcggc agcgcttcga ccgccccgac 540

ggcacctcca cgctcccggg gctggacatc ttcgtcacca cggccgaccc catcaaggag 600

cccatcctct ccaccgccaa ctcggtgctc tccatcctgg ccgccgacta ccccgtggac 660
cgcaacacat gctacgtctc cgacgacagt ggcatgctgc tcacctacga ggccctggca 720
gagtcctcca agttcgccac gctctgggtg cccttctacc gcaagcacgg gatcgagccc 780
aggggtccgg agagctactt cgagctcaag tcacaccctt acatggggag agcccaggac 840
gagttcgtca acgaccgccg ccgcgttcgc aaggagtacg acgagttcaa ggccaggatc 900
aacagcctgg agcatgacat caagcagcgc aacgacgggt acaacgccgc cattgcccac 960
agccaaggcg tgccccggcc cacctggatg gcggacggca cccagtggga gggcacatgg 1020
gtcgacgcct ccgagaacca ccgcaggggc gaccacgccg gcatcgtact ggtgctgctg 1080
aaccacccga gccaccgccg gcagacgggc ccgccggcga gcgctgacaa ccccactggac 1140
ttgagcggcg tggatgtgcg tctccccatg ctggtgtacg tgtcccgtga gaagcgcccc 1200
gggcacgacc accagaagaa ggccggtgcc atgaacgcgc ttacccgcgc ctcggcgctg 1260
ctctccaact ccccccttcat cctcaacctc gactgcgatc attacatcaa caactcccag 1320
gcccttcgcg ccggcatctg cttcatggtg ggacgggaca gcgacacggt tgccttcgtc 1380
cagttcccgc agcgcttcga gggcgtcgac cccaccgacc tctacgccaa ccacaaccgc 1440
atcttcttcg acggcaccct ccgtgccctg gacggcatgc agggccccat ctacgtcggc 1500
actgggtgtc tcttccgccg catcaccgtc tacggcttcg acccgccgag gatcaacgtc 1560
ggcggtccct gcttccccag gctcgccggg ctcttcgcca agaccaagta cgagaagccc 1620
gggctcgaga tgaccacggc caaggccaag gccgcgcccg tgcccgccaa gggtaagcac 1680
ggcttcttgc cactgcccaa gaagacgtac ggcaagtcgg acgccttcgt ggacaccatc 1740
ccgcgcgcgt cgcacccgtc gccctacgcc gcggcggctg aggggatcgt ggccgacgag 1800
gcgaccatcg tcgaggcggt gaacgtgacg gccgccgcgt tcgagaagaa gaccggctgg 1860
ggcaaagaga tcggctgggt gtacgacacc gtcacggagg acgtggtcac cggctaccgg 1920
atgcatatca aggggtggcg gtcacgctac tgctccatct acccacacgc cttcatcggc 1980
accgccccca tcaacctcac ggagaggctc ttccaggtgc tccgctggtc cacgggatcc 2040
ctcgagatct tcttctccaa gaacaacccg ctcttcggca gcacatacct ccacccgctg 2100
cagcgcgtcg cctacatcaa catcaccact tacccccttca ccgccatctt cctcatcttc 2160
tacaccaccg tgccggcgct atccttcgtc accggccact tcatcgtgca gcgcccgacc 2220
accatgttct acgtctacct gggcatcgtg ctatccacgc tgctcgtcat cgccgtgctg 2280
gaggtcaagt gggccggggt cacagtcttc gagtggttca ggaacggcca gttctggatg 2340
acagcaagtt gctccgccta cctcgccgcc gtctgccagg tgctgaccaa ggtgatattc 2400
cggcgggaca tctccttcaa gctcacatcc aagctaccct cgggagacga gaagaaggac 2460
ccctacgccg acctctacgt ggtgcgctgg acgccgctca tgattacacc catcatcatc 2520
atcttcgtca acatcatcgg atccgccgtg gccttcgcca aggttctcga cggcgagtgg 2580

acgcactggc tcaaggtcgc cggcggcgtc ttcttcaact tctgggtgct cttccacctc 2640

taccccttcg ccaagggcat cctggggaag cacggaaaga cgccagtcgt ggtgctcgtc 2700

tggtgggcat tcaccttcgt catcaccgcc gtgctctaca tcaacatccc ccacatgcat 2760

acctcgggag gcaagcacac aacggtgcat ggtcaccatg gcaagaagtt ggtcgacaca 2820

gggctctatg gctggctcca ttga 2844

<210> 20
<211> 947
<212> PRT
<213> Hordeum vulgare

<220>
<221> misc_feature
<223> KM27 bgl gene aa

<400> 20

Met Ala Pro Ala Val Ala Gly Gly Gly Arg Val Arg Ser Asn Glu Pro
1 5 10 15

Val Ala Ala Ala Ala Ala Ala Pro Ala Ala Ser Gly Lys Pro Cys Val
20 25 30

Cys Gly Phe Gln Val Cys Ala Cys Thr Gly Ser Ala Ala Val Ala Ser
35 40 45

Ala Ala Ser Ser Leu Asp Met Asp Ile Val Ala Met Gly Gln Ile Gly
50 55 60

Ala Val Asn Asp Glu Ser Trp Val Gly Val Glu Leu Gly Glu Asp Gly
65 70 75 80

Glu Thr Asp Glu Ser Gly Ala Ala Val Asp Asp Arg Pro Val Phe Arg
85 90 95

Thr Glu Lys Ile Lys Gly Val Leu Leu His Pro Tyr Arg Val Leu Ile
100 105 110

Phe Val Arg Leu Ile Ala Phe Thr Leu Phe Val Ile Trp Arg Ile Ser
115 120 125

His Lys Asn Pro Asp Ala Met Trp Leu Trp Val Thr Ser Ile Cys Gly
130 135 140

Glu Phe Trp Phe Gly Phe Ser Trp Leu Leu Asp Gln Leu Pro Lys Leu
145 150 155 160

Asn Pro Ile Asn Arg Val Pro Asp Leu Ala Val Leu Arg Gln Arg Phe

165 170 175

Asp Arg Pro Asp Gly Thr Ser Thr Leu Pro Gly Leu Asp Ile Phe Val
180 185 190

Thr Thr Ala Asp Pro Ile Lys Glu Pro Ile Leu Ser Thr Ala Asn Ser
195 200 205

Val Leu Ser Ile Leu Ala Ala Asp Tyr Pro Val Asp Arg Asn Thr Cys
210 215 220

Tyr Val Ser Asp Asp Ser Gly Met Leu Leu Thr Tyr Glu Ala Leu Ala
225 230 235 240

Glu Ser Ser Lys Phe Ala Thr Leu Trp Val Pro Phe Tyr Arg Lys His
245 250 255

Gly Ile Glu Pro Arg Gly Pro Glu Ser Tyr Phe Glu Leu Lys Ser His
260 265 270

Pro Tyr Met Gly Arg Ala Gln Asp Glu Phe Val Asn Asp Arg Arg Arg
275 280 285

Val Arg Lys Glu Tyr Asp Glu Phe Lys Ala Arg Ile Asn Ser Leu Glu
290 295 300

His Asp Ile Lys Gln Arg Asn Asp Gly Tyr Asn Ala Ala Ile Ala His
305 310 315 320

Ser Gln Gly Val Pro Arg Pro Thr Trp Met Ala Asp Gly Thr Gln Trp
325 330 335

Glu Gly Thr Trp Val Asp Ala Ser Glu Asn His Arg Arg Gly Asp His
340 345 350

Ala Gly Ile Val Leu Val Leu Leu Asn His Pro Ser His Arg Arg Gln
355 360 365

Thr Gly Pro Pro Ala Ser Ala Asp Asn Pro Leu Asp Leu Ser Gly Val
370 375 380

Asp Val Arg Leu Pro Met Leu Val Tyr Val Ser Arg Glu Lys Arg Pro
385 390 395 400

Gly His Asp His Gln Lys Lys Ala Gly Ala Met Asn Ala Leu Thr Arg
405 410 415

Ala Ser Ala Leu Leu Ser Asn Ser Pro Phe Ile Leu Asn Leu Asp Cys
420 425 430

```
Asp His Tyr Ile Asn Asn Ser Gln Ala Leu Arg Ala Gly Ile Cys Phe
        435                 440                 445

Met Val Gly Arg Asp Ser Asp Thr Val Ala Phe Val Gln Phe Pro Gln
    450                 455                 460

Arg Phe Glu Gly Val Asp Pro Thr Asp Leu Tyr Ala Asn His Asn Arg
465                 470                 475                 480

Ile Phe Phe Asp Gly Thr Leu Arg Ala Leu Asp Gly Met Gln Gly Pro
                485                 490                 495

Ile Tyr Val Gly Thr Gly Cys Leu Phe Arg Arg Ile Thr Val Tyr Gly
            500                 505                 510

Phe Asp Pro Pro Arg Ile Asn Val Gly Gly Pro Cys Phe Pro Arg Leu
        515                 520                 525

Ala Gly Leu Phe Ala Lys Thr Lys Tyr Glu Lys Pro Gly Leu Glu Met
    530                 535                 540

Thr Thr Ala Lys Ala Lys Ala Ala Pro Val Pro Ala Lys Gly Lys His
545                 550                 555                 560

Gly Phe Leu Pro Leu Pro Lys Lys Thr Tyr Gly Lys Ser Asp Ala Phe
                565                 570                 575

Val Asp Thr Ile Pro Arg Ala Ser His Pro Ser Pro Tyr Ala Ala Ala
            580                 585                 590

Ala Glu Gly Ile Val Ala Asp Glu Ala Thr Ile Val Glu Ala Val Asn
        595                 600                 605

Val Thr Ala Ala Ala Phe Glu Lys Lys Thr Gly Trp Gly Lys Glu Ile
    610                 615                 620

Gly Trp Val Tyr Asp Thr Val Thr Glu Asp Val Val Thr Gly Tyr Arg
625                 630                 635                 640

Met His Ile Lys Gly Trp Arg Ser Arg Tyr Cys Ser Ile Tyr Pro His
                645                 650                 655

Ala Phe Ile Gly Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685
```

```
Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
705                 710                 715                 720

Tyr Thr Thr Val Pro Ala Leu Ser Phe Val Thr Gly His Phe Ile Val
                725                 730                 735

Gln Arg Pro Thr Thr Met Phe Tyr Val Tyr Leu Gly Ile Val Leu Ser
            740                 745                 750

Thr Leu Leu Val Ile Ala Val Leu Glu Val Lys Trp Ala Gly Val Thr
        755                 760                 765

Val Phe Glu Trp Phe Arg Asn Gly Gln Phe Trp Met Thr Ala Ser Cys
    770                 775                 780

Ser Ala Tyr Leu Ala Ala Val Cys Gln Val Leu Thr Lys Val Ile Phe
785                 790                 795                 800

Arg Arg Asp Ile Ser Phe Lys Leu Thr Ser Lys Leu Pro Ser Gly Asp
                805                 810                 815

Glu Lys Lys Asp Pro Tyr Ala Asp Leu Tyr Val Val Arg Trp Thr Pro
            820                 825                 830

Leu Met Ile Thr Pro Ile Ile Ile Ile Phe Val Asn Ile Ile Gly Ser
        835                 840                 845

Ala Val Ala Phe Ala Lys Val Leu Asp Gly Glu Trp Thr His Trp Leu
    850                 855                 860

Lys Val Ala Gly Gly Val Phe Phe Asn Phe Trp Val Leu Phe His Leu
865                 870                 875                 880

Tyr Pro Phe Ala Lys Gly Ile Leu Gly Lys His Gly Lys Thr Pro Val
                885                 890                 895

Val Val Leu Val Trp Trp Ala Phe Thr Phe Val Ile Thr Ala Val Leu
            900                 905                 910

Tyr Ile Asn Ile Pro His Met His Thr Ser Gly Gly Lys His Thr Thr
        915                 920                 925

Val His Gly His His Gly Lys Lys Leu Val Asp Thr Gly Leu Tyr Gly
    930                 935                 940
```

Trp Leu His
945

<210> 21
<211> 20
<212> DNA
<213> Artificial Sequence

<220>
<223> HvCSLF6_MPP2-1F

<400> 21
atcaaggagc ccatcctctc 20

<210> 22
<211> 20
<212> DNA
<213> Artificial Sequence

<220>
<223> HvCSLF6_MPP2-1R

<400> 22
ttgatcctgg ccttgaactc 20

<210> 23
<211> 5124
<212> DNA
<213> Hordeum vulgare

<220>
<221> misc_feature
<223> TR251 HvCslF6 gene genomic DNA

<400> 23
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtagccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccggtac gtcctgctcc cacaactaaa cagaaactcc 360

ctatatctgc gtcacactca acaattaatc caactaagtc tctctactac tatagtattt 420

atttttactc tctatctgca caacaagcgc tactacaatt aacccaacaa gcaccacgcc 480

aggttgacag tcaggataat ttgatcttga ccggagtaag tactagtact aggtcggtgt 540

taatcagagt aattattgca ctagttaatt aaaatttgag taatccgaga caggtgcacg 600

ttagggccgg gccaatgatc gctcgaatcc acccaaaata gcgcgtcccg gtgtgggctg 660

tcggctcggt gcttcttcct tccattttac tagtcgcagt cactgcagct tgggcccacg 720

ggaggggacg ttagccgttg ggcctgcctg gcaggtgggc cccggtggcc accctggcgg 780

ctcataaatc cttgctactt tggagctgta atggacgctc tgcaatagca ataggaatcc 840
gaggtgaaac gacgacagtg ggcatggcat ggcttgcatg tgaatccaag ccacatcatt 900
aaaagcatcc tccctgggca cgtcgcggtg agaaagttgg ataaactttt gggggttcgg 960
acaagatgag aaaaagcaag taacatggcc ctttttggc accgaagaaa tcctatgtac 1020
ggcgagtttt ttctgcatct agttatgggt agatgtacgt tagtttttgt tgagcgtttt 1080
atgtgctaca tatatggaga aaaagagaaa aatattatca tgtcatgtca tgccatgaga 1140
agaaaaagaa gtgttgccac cgctcgaatg cctcttttt ctttcggaag gatgcgtgag 1200
tcatgttggc accgagaaaa gccatattaa agtggcagag ttacaactca gaataaatgc 1260
gggtgttaca aaaacactag gatatgtgaa gggcactcgg cacaacactt taagactaca 1320
caattgaaaa agtgtactct ctgtatctaa ataattataa ttgaaaagaa ttaatctata 1380
tatgaaagta gtaatgatta gcggtagaag gttccaacga ctttttggc gccaatagca 1440
agaagaaaga aaaagaaaaa aatctttact gtacagtaac gagaaaagag gcccattaat 1500
agagcaacga atccagcggc accacctctg gcggtcacgt ccatgccctc gcacgacgga 1560
tgaggcccgg ggggtcctac tgacagccga agcatgtcgg tgctcaaaca cggcgccgtt 1620
tgctgccaag tgtgccagct cgcactcatt gacttgccag ctctctcctt ggttgtcaat 1680
gagaacatga tgccttttgg catttgcaaa cttattaaaa ctagctgtcg tccgataggg 1740
aaaagaaaag aaaagaaaag aataagaaaa aaaggacaaa gagaaaagat gaacatggcg 1800
catgttccct ccaataattg caggcaccaa cactgggtcg attaatccaa caacaatatt 1860
ttactatacc agacgagagt acagtagtcg ggtgatgatg gactgtaact gactgagtat 1920
gaatgactgt aatgcagggt gctgattttc gttcgtctga tcgccttcac gctgttcgtg 1980
atctggcgta tctcccacaa gaacccagac gcgatgtggc tgtgggtgac atccatctgc 2040
ggcgagttct ggttcggttt ctcgtggctg ctggatcagc tgcccaagct gaaccccatc 2100
aaccgcgtgc cggacctggc ggtgctgcgg cagcgcttcg accgccccga cggcacctcc 2160
acgctcccgg ggctggacat cttcgtcacc acggccgacc ccatcaagga gcccatcctc 2220
tccaccgcca actcggtgct ctccatcctg gccgccgact accccgtgga ccgcaacaca 2280
tgctacgtct ccgacgacag tggcatgctg ctcacctacg aggccctggc agagtcctcc 2340
aagttcgcca cgctctgggt gcccttctgc cgcaagcacg ggatcgagcc caggggtccg 2400
gagagctact tcgagctcaa gtcacaccct tacatgggga gagcccagga cgagttcgtc 2460
aacgaccgcc gccgcgttcg caaggagtac gacgagttca aggccaggat caacagcctg 2520
gagcatgaca tcaagcagcg caacgacggg tacaacgccg ccattgccca cagccaaggc 2580
gtgccccggc ccacctggat ggcggacggc acccagtggg agggcacatg ggtcgacgcc 2640
tccgagaacc accgcagggg cgaccacgcc ggcatcgtac tggtcagtat ccatccatct 2700
ttctgctgct tatattactc ttaggttact cttatcgtct ctttcctata ccgtacatgc 2760

atgcatgctg ctattcttgg aatcgtggtt ggttactact ccaccatgca aaaataacaa 2820
gaagaggaat cttggttagt tagggcctcg ttgttatatt agtggccatc tgatgtgatg 2880
cctgccggct gtgcccatcc atatccatgg aagatttcga cagaatcgac gtggtgatag 2940
ttgagagtgc aaccaccacc cagagccagc caagcacatg catgcttctc ttctcgtctc 3000
gtcgtgtggc cagcagcgca ttcatgctat tgctgtgacg agggaggaat ggtggttggg 3060
gtggtccttt ccccccgaca gcactacagc ctccacttta tgacccattt aattcatcgg 3120
ccctgctttg ttgtaaccgc cttctcacct caatcaatca ttcattcatt cataagttta 3180
ctcactcttt gttactactc gaaccactaa tcaggaagga gtaggagtaa tgcagattta 3240
ctattaacag ttaaaggagt aaaaagaagg aagcacaatt acagaacctt gttttttttt 3300
actactgtac gtaaggtgta agaatggagt gctgacagag aatggatgca ggtgctgctg 3360
aaccacccga gccaccgccg gcagacgggc ccgccggcga gcgctgacaa cccactggac 3420
ttgagcggcg tggatgtgcg tctccccatg ctggtgtacg tgtcccgtga gaagcgcccc 3480
gggcacgacc accagaagaa ggccggtgcc atgaacgcgc ttacccgcgc ctcggcgctg 3540
ctctccaact ccccttcat cctcaacctc gactgcgatc attacatcaa caactcccag 3600
gcccttcgcg ccggcatctg cttcatggtg ggacgggaca gcgacacggt tgccttcgtc 3660
cagttcccgc agcgcttcga gggcgtcgac cccaccgacc tctacgccaa ccacaaccgc 3720
atcttcttcg acggcaccct ccgtgccctg gacggcatgc agggccccat ctacgtcggc 3780
actgggtgtc tcttccgccg catcaccgtc tacggcttcg acccgccgag gatcaacgtc 3840
ggcggtccct gcttccccag gctcgccggg ctcttcgcca agaccaagta cgagaagccc 3900
gggctcgaga tgaccacggc caaggccaag gccgcgcccg tgcccgccaa gggtaagcac 3960
ggcttcttgc cactgcccaa gaagacgtac ggcaagtcgg acgccttcgt ggacaccatc 4020
ccgcgcgcgt cgcacccgtc gccctacacc gcggcggctg aggggatcgt ggccgacgag 4080
gcgaccatcg tcgaggcggt gaacgtgacg gccgccgcgt tcgagaagaa gaccggctgg 4140
ggcaaagaga tcggctgggt gtacgacacc gtcacggagg acgtggtcac cggctaccgg 4200
atgcatatca aggggtggcg gtcacgctac tgctccatct acccacacgc cttcatcggc 4260
accgccccca tcaacctcac ggagaggctc ttccaggtgc tccgctggtc cacgggatcc 4320
ctcgagatct tcttctccaa gaacaacccg ctcttcggca gcacatacct ccacccgctg 4380
cagcgcgtcg cctacatcaa catcaccact taccccttca ccgccatctt cctcatcttc 4440
tacaccaccg tgccggcgct atccttcgtc accggccact tcatcgtgca gcgcccgacc 4500
accatgttct acgtctacct gggcatcgtg ctatccacgc tgctcgtcat cgccgtgctg 4560
gaggtcaagt gggccggggt cacagtcttc gagtggttca ggaacggcca gttctggatg 4620
acagcaagtt gctccgccta cctcgccgcc gtctgccagg tgctgaccaa ggtgatattc 4680

cggcgggaca tctccttcaa gctcacatcc aagctaccct cgggagacga gaagaaggac 4740

ccctacgccg acctctacgt ggtgcgctgg acgccgctca tgatcacacc catcatcatc 4800

atcttcgtca acatcatcgg atccgccgtg gccttcgcca aggttctcga cggcgagtgg 4860

acgcactggc tcaaggtcgc cggcggcgtc ttcttcaact tctgggtgct cttccacctc 4920

taccccttcg ccaagggcat cctggggaag cacggaaaga cgccagtcgt ggtgctcgtc 4980

tggtgggcat tcaccttcgt catcaccgcc gtgctctaca tcaacatccc ccacatgcat 5040

acctcgggag gcaagcacac aacggtgcat ggtcaccatg gcaagaagtt ggtcgacaca 5100

gggctctatg gctggctcca ttga 5124

<210> 24
<211> 2844
<212> DNA
<213> Hordeum vulgare

<220>
<221> misc_feature
<223> TR251 HvCslF6 gene cDNA

<400> 24
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtagccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccgggtg ctgattttcg ttcgtctgat cgccttcacg 360

ctgttcgtga tctggcgtat ctcccacaag aacccagacg cgatgtggct gtgggtgaca 420

tccatctgcg gcgagttctg gttcggtttc tcgtggctgc tggatcagct gcccaagctg 480

aaccccatca accgcgtgcc ggacctggcg gtgctgcggc agcgcttcga ccgccccgac 540

ggcacctcca cgctcccggg gctggacatc ttcgtcacca cggccgaccc catcaaggag 600

cccatcctct ccaccgccaa ctcggtgctc tccatcctgg ccgccgacta ccccgtggac 660

cgcaacacat gctacgtctc cgacgacagt ggcatgctgc tcacctacga ggccctggca 720

gagtcctcca agttcgccac gctctgggtg cccttctgcc gcaagcacgg gatcgagccc 780

aggggtccgg agagctactt cgagctcaag tcacaccctt acatggggag agcccaggac 840

gagttcgtca acgaccgccg ccgcgttcgc aaggagtacg acgagttcaa ggccaggatc 900

aacagcctgg agcatgacat caagcagcgc aacgacgggt acaacgccgc cattgcccac 960

agccaaggcg tgccccggcc cacctggatg gcggacggca cccagtggga gggcacatgg 1020

gtcgacgcct ccgagaacca ccgcaggggc gaccacgccg gcatcgtact ggtgctgctg 1080

aaccacccga gccaccgccg gcagacgggc ccgccggcga gcgctgacaa cccactggac 1140

```
ttgagcggcg tggatgtgcg tctccccatg ctggtgtacg tgtcccgtga gaagcgcccc     1200
gggcacgacc accagaagaa ggccggtgcc atgaacgcgc ttacccgcgc ctcggcgctg     1260
ctctccaact ccccttcat cctcaacctc gactgcgatc attacatcaa caactcccag      1320
gcccttcgcg ccggcatctg cttcatggtg ggacgggaca gcgacacggt tgccttcgtc     1380
cagttcccgc agcgcttcga gggcgtcgac cccaccgacc tctacgccaa ccacaaccgc     1440
atcttcttcg acggcaccct ccgtgccctg gacggcatgc agggccccat ctacgtcggc     1500
actgggtgtc tcttccgccg catcaccgtc tacggcttcg acccgccgag gatcaacgtc     1560
ggcggtccct gcttccccag gctcgccggg ctcttcgcca agaccaagta cgagaagccc     1620
gggctcgaga tgaccacggc caaggccaag gccgcgcccg tgcccgccaa gggtaagcac     1680
ggcttcttgc cactgcccaa gaagacgtac ggcaagtcgg acgccttcgt ggacaccatc     1740
ccgcgcgcgt cgcacccgtc gccctacacc gcggcggctg aggggatcgt ggccgacgag     1800
gcgaccatcg tcgaggcggt gaacgtgacg gccgccgcgt tcgagaagaa gaccggctgg     1860
ggcaaagaga tcggctgggt gtacgacacc gtcacggagg acgtggtcac cggctaccgg     1920
atgcatatca aggggtggcg gtcacgctac tgctccatct acccacacgc cttcatcggc     1980
accgccccca tcaacctcac ggagaggctc ttccaggtgc tccgctggtc cacgggatcc     2040
ctcgagatct tcttctccaa gaacaacccg ctcttcggca gcacatacct ccacccgctg     2100
cagcgcgtcg cctacatcaa catcaccact taccccttca ccgccatctt cctcatcttc     2160
tacaccaccg tgccggcgct atccttcgtc accggccact tcatcgtgca gcgcccgacc     2220
accatgttct acgtctacct gggcatcgtg ctatccacgc tgctcgtcat cgccgtgctg     2280
gaggtcaagt gggccggggt cacagtcttc gagtggttca ggaacggcca gttctggatg     2340
acagcaagtt gctccgccta cctcgccgcc gtctgccagg tgctgaccaa ggtgatattc     2400
cggcgggaca tctccttcaa gctcacatcc aagctaccct cgggagacga gaagaaggac     2460
ccctacgccg acctctacgt ggtgcgctgg acgccgctca tgatcacacc catcatcatc     2520
atcttcgtca acatcatcgg atccgccgtg gccttcgcca aggttctcga cggcgagtgg     2580
acgcactggc tcaaggtcgc cggcggcgtc ttcttcaact tctgggtgct cttccacctc     2640
taccccttcg ccaagggcat cctggggaag cacggaaaga cgccagtcgt ggtgctcgtc     2700
tggtgggcat tcaccttcgt catcaccgcc gtgctctaca tcaacatccc ccacatgcat     2760
acctcgggag gcaagcacac aacggtgcat ggtcaccatg gcaagaagtt ggtcgacaca     2820
gggctctatg gctggctcca ttga                                            2844
```

<210> 25
<211> 947
<212> PRT
<213> Hordeum vulgare

<220>
<221> misc_feature
<223> TR251 HvCslF6 aa

<400> 25

```
Met Ala Pro Ala Val Ala Gly Gly Gly Arg Val Arg Ser Asn Glu Pro
1               5                   10                  15

Val Ala Ala Ala Ala Ala Ala Pro Ala Ala Ser Gly Lys Pro Cys Val
            20                  25                  30

Cys Gly Phe Gln Val Cys Ala Cys Thr Gly Ser Ala Ala Val Ala Ser
        35                  40                  45

Ala Ala Ser Ser Leu Asp Met Asp Ile Val Ala Met Gly Gln Ile Gly
    50                  55                  60

Ala Val Asn Asp Glu Ser Trp Val Gly Val Glu Leu Gly Glu Asp Gly
65                  70                  75                  80

Glu Thr Asp Glu Ser Gly Ala Ala Val Asp Asp Arg Pro Val Phe Arg
                85                  90                  95

Thr Glu Lys Ile Lys Gly Val Leu Leu His Pro Tyr Arg Val Leu Ile
            100                 105                 110

Phe Val Arg Leu Ile Ala Phe Thr Leu Phe Val Ile Trp Arg Ile Ser
        115                 120                 125

His Lys Asn Pro Asp Ala Met Trp Leu Trp Val Thr Ser Ile Cys Gly
    130                 135                 140

Glu Phe Trp Phe Gly Phe Ser Trp Leu Leu Asp Gln Leu Pro Lys Leu
145                 150                 155                 160

Asn Pro Ile Asn Arg Val Pro Asp Leu Ala Val Leu Arg Gln Arg Phe
                165                 170                 175

Asp Arg Pro Asp Gly Thr Ser Thr Leu Pro Gly Leu Asp Ile Phe Val
            180                 185                 190

Thr Thr Ala Asp Pro Ile Lys Glu Pro Ile Leu Ser Thr Ala Asn Ser
        195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240
```

Glu Ser Ser Lys Phe Ala Thr Leu Trp Val Pro Phe Cys Arg Lys His
245 250 255

Gly Ile Glu Pro Arg Gly Pro Glu Ser Tyr Phe Glu Leu Lys Ser His
260 265 270

Pro Tyr Met Gly Arg Ala Gln Asp Glu Phe Val Asn Asp Arg Arg Arg
275 280 285

Val Arg Lys Glu Tyr Asp Glu Phe Lys Ala Arg Ile Asn Ser Leu Glu
290 295 300

His Asp Ile Lys Gln Arg Asn Asp Gly Tyr Asn Ala Ala Ile Ala His
305 310 315 320

Ser Gln Gly Val Pro Arg Pro Thr Trp Met Ala Asp Gly Thr Gln Trp
325 330 335

Glu Gly Thr Trp Val Asp Ala Ser Glu Asn His Arg Arg Gly Asp His
340 345 350

Ala Gly Ile Val Leu Val Leu Leu Asn His Pro Ser His Arg Arg Gln
355 360 365

Thr Gly Pro Pro Ala Ser Ala Asp Asn Pro Leu Asp Leu Ser Gly Val
370 375 380

Asp Val Arg Leu Pro Met Leu Val Tyr Val Ser Arg Glu Lys Arg Pro
385 390 395 400

Gly His Asp His Gln Lys Lys Ala Gly Ala Met Asn Ala Leu Thr Arg
405 410 415

Ala Ser Ala Leu Leu Ser Asn Ser Pro Phe Ile Leu Asn Leu Asp Cys
420 425 430

Asp His Tyr Ile Asn Asn Ser Gln Ala Leu Arg Ala Gly Ile Cys Phe
435 440 445

Met Val Gly Arg Asp Ser Asp Thr Val Ala Phe Val Gln Phe Pro Gln
450 455 460

Arg Phe Glu Gly Val Asp Pro Thr Asp Leu Tyr Ala Asn His Asn Arg
465 470 475 480

Ile Phe Phe Asp Gly Thr Leu Arg Ala Leu Asp Gly Met Gln Gly Pro
485 490 495

```
Ile Tyr Val Gly Thr Gly Cys Leu Phe Arg Arg Ile Thr Val Tyr Gly
            500                 505                 510

Phe Asp Pro Pro Arg Ile Asn Val Gly Gly Pro Cys Phe Pro Arg Leu
        515                 520                 525

Ala Gly Leu Phe Ala Lys Thr Lys Tyr Glu Lys Pro Gly Leu Glu Met
    530                 535                 540

Thr Thr Ala Lys Ala Lys Ala Ala Pro Val Pro Ala Lys Gly Lys His
545                 550                 555                 560

Gly Phe Leu Pro Leu Pro Lys Lys Thr Tyr Gly Lys Ser Asp Ala Phe
                565                 570                 575

Val Asp Thr Ile Pro Arg Ala Ser His Pro Ser Pro Tyr Thr Ala Ala
            580                 585                 590

Ala Glu Gly Ile Val Ala Asp Glu Ala Thr Ile Val Glu Ala Val Asn
        595                 600                 605

Val Thr Ala Ala Ala Phe Glu Lys Lys Thr Gly Trp Gly Lys Glu Ile
    610                 615                 620

Gly Trp Val Tyr Asp Thr Val Thr Glu Asp Val Val Thr Gly Tyr Arg
625                 630                 635                 640

Met His Ile Lys Gly Trp Arg Ser Arg Tyr Cys Ser Ile Tyr Pro His
                645                 650                 655

Ala Phe Ile Gly Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685

Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
705                 710                 715                 720

Tyr Thr Thr Val Pro Ala Leu Ser Phe Val Thr Gly His Phe Ile Val
                725                 730                 735

Gln Arg Pro Thr Thr Met Phe Tyr Val Tyr Leu Gly Ile Val Leu Ser
            740                 745                 750
```

Thr Leu Leu Val Ile Ala Val Leu Glu Val Lys Trp Ala Gly Val Thr
755 760 765

Val Phe Glu Trp Phe Arg Asn Gly Gln Phe Trp Met Thr Ala Ser Cys
770 775 780

Ser Ala Tyr Leu Ala Ala Val Cys Gln Val Leu Thr Lys Val Ile Phe
785 790 795 800

Arg Arg Asp Ile Ser Phe Lys Leu Thr Ser Lys Leu Pro Ser Gly Asp
805 810 815

Glu Lys Lys Asp Pro Tyr Ala Asp Leu Tyr Val Val Arg Trp Thr Pro
820 825 830

Leu Met Ile Thr Pro Ile Ile Ile Ile Phe Val Asn Ile Ile Gly Ser
835 840 845

Ala Val Ala Phe Ala Lys Val Leu Asp Gly Glu Trp Thr His Trp Leu
850 855 860

Lys Val Ala Gly Gly Val Phe Phe Asn Phe Trp Val Leu Phe His Leu
865 870 875 880

Tyr Pro Phe Ala Lys Gly Ile Leu Gly Lys His Gly Lys Thr Pro Val
885 890 895

Val Val Leu Val Trp Trp Ala Phe Thr Phe Val Ile Thr Ala Val Leu
900 905 910

Tyr Ile Asn Ile Pro His Met His Thr Ser Gly Gly Lys His Thr Thr
915 920 925

Val His Gly His His Gly Lys Lys Leu Val Asp Thr Gly Leu Tyr Gly
930 935 940

Trp Leu His
945

<210> 26
<211> 5140
<212> DNA
<213> Hordeum vulgare

<220>
<221> misc_feature
<223> CDC-Bold HvCslF6 gene genomic DNA

<400> 26
atggcgccag cggtggccgg agggggccgc gtgcggagca atgagccggt tgctgctgct 60

```
gccgccgcgc cggcggccag cggcaagccc tgcgtgtgcg gcttccaggt ttgcgcctgc      120
acggggtcgg ccgcggtggc ctccgccgcc tcgtcgctgg acatggacat cgtggccatg      180
gggcagatcg gcgccgtcaa cgacgagagc tgggtgggcg tggagctcgg cgaagatggc      240
gagaccgacg aaagcggtgc cgccgttgac gaccgccccg tattccgcac cgagaagatc      300
aagggtgtcc tcctccaccc ctaccggtac gtcctgctcc cacaactaaa cagaaactcc      360
ctatatctgc gtcacactca acaattaatc caactaagtc tctctactac tctagtattt      420
atttttactc tctatctgca caacaagcgc tactacaatt aacccaacaa gcaccacgcc      480
aggttgacag tcaggataat ttgatcttga ccggagtaag tactagtact aggtcggtgt      540
taatcagagt aattattgca ctagttaatt aaaatttgag taatccgaga caggtgcacg      600
ttagggccgg gccaatgatg gctcgaatcc acccaaaata gcgcgtcccg gtgtgggctg      660
tcggctcggt gcttcttcct tccattttac tagtcgcagt cactgcagct tgggcccacg      720
ggaggggacg ttagccgttg ggcctgcctg gcaggtgggc cccggtggcc accctggcgg      780
ctcataaatc cttgctactt tggagctgta atggacgctc tgcaatagca ataggaatcc      840
gaggtgaaac gacgacagtg ggcatggcat ggcttgcatg tgaatccaag ccacatcatt      900
aaaagcatcc tccctgggca cgtcgcggtg agaaagttgg ataaactttt gggggttcgg      960
acaagatgag aaaaagcaag taacatgtcc ctttttggc accgaagaaa tcctatgtac      1020
ggcgagtttt ttctgcatct agttatgggt agatgtacgt tagtttttgt tgagcgtttt     1080
atgtgctaca tatatggaga aaaagagaaa aatattatca tgtcatgtca tgccatgcca     1140
tgagaaggag gagaagaaaa agaagtgttg ccaccgctcg aatgcctttt ttttctttcg     1200
gaaggatgcg tgagtcatgt tggcaccgag aaaagccata ttaaagtggc agagttacaa     1260
ctcagaataa atgcgggtgt tacaaaaaca ctaggatatg tgaagggcac tcggcacaac     1320
actttaagac tacacaattg aaaaagtgta ctctctgtat ctaaataatt ataattgaaa     1380
agaattaatc tatatatgaa agtagtaatg attagcggta gaaggttcca acgacttttt     1440
tggcgccaat agcaagaaga aagaaaaaga aaaaaatctt tactgtacag tataacgaga     1500
aaagaggccc attaatagag caacgaatcg agcggcacca cctctggcgg tcacgtccat     1560
gccctcgcac gacggatgag gcccgggggg tcctactgac agccgaagca tgtcggtgct     1620
caaacacggc gccgtttgct gccaagtgtg ccagctcgca ctcattgact tgccagctct     1680
ctccttggtt gtcaatgaga acatgatgcc ttttggcatt tgcaaactta ttaaaactag     1740
ctgtcgtccg atagggaaaa gaaaagaaaa gaaaagaata agaaaaaaag gacaaagaga     1800
aaagatgaac atggcgcatg ttccctccaa taattgcagg caccaacact gggtcgatta     1860
atccaacaac aatattttac tataccagac gagagtacag tagtcgggtg atgatggact     1920
gtaactgact gagtatgaat gactgtaatg cagggtgctg attttcgttc gtctgatcgc     1980
cttcacgctg ttcgtgatct ggcgtatctc ccacaagaac ccagacgcga tgtggctgtg     2040
```

ggtgacatcc atctgcggcg agttctggtt cggtttctcg tggctgctgg atcagctgcc 2100
caagctgaac cccatcaacc gcgtgccgga cctggcggtg ctgcggcagc gcttcgaccg 2160
ccccgacggc acctccacgc tcccggggct ggacatcttc gtcaccacgg ccgaccccat 2220
caaggagccc atcctctcca ccgccaactc ggtgctctcc atcctggccg ccgactaccc 2280
cgtggaccgc aacacatgct acgtctccga cgacagtggc atgctgctca cctacgaggc 2340
cctggcagag tcctccaagt tcgccacgct ctgggtgccc ttctgccgca agcacgggat 2400
cgagcccagg ggtccggaga gctacttcga gctcaagtca caccccttaca tggggagagc 2460
ccaggacgag ttcgtcaacg accgccgccg cgttcgcaag gagtacgacg agttcaaggc 2520
caggatcaac agcctggagc atgacatcaa gcagcgcaac gacgggtaca acgccgccat 2580
tgcccacagc caaggcgtgc cccggcccac ctggatggcg gacggcaccc agtgggaggg 2640
cacatgggtc gacgcctccg agaaccaccg caggggcgac cacgccggca tcgtactggt 2700
cagtatccat ccatctttct gctgcttata ttactcttag gttactctta tcgtctcttt 2760
cctatactgt acatgcatgc atgctgctat tcttggaatc gtggttggtt actactccac 2820
catgcaaaaa taacaagaag aggaatcttg gttagttagg gcctcgttgt tatattagtg 2880
gccatctgat gtgatgcctg ccggctgtgc ccatccatat ccatggaaga tttcgacaga 2940
atcgacgtgg tgatagtcga gagtgcaacc accacccaga gccagccaag cacatgcatg 3000
cttctcttct cgtctcgtcg tgtggccagc agcgcattca tgctattgct gtgacgaggg 3060
aggaatggta gttggggtgg tcctttcccc ccgacagcac tacagcctcc actttatgac 3120
ccatttaatt caccggccct gctttgttgt aaccgccttc tcatctcaat caatcattca 3180
ttcattcata agtttactca ctctttgtta ctactcgaac cactaatcag gaaggagtag 3240
gagtaatgca gatttactat tgacagttaa aggagtaaaa agaaggaagc acaattacag 3300
aaccttgttt ttttttacta ctgtacgtaa ggtgtaagaa tggagtgctg acagagaatg 3360
gatgcaggtg ctgctgaacc acccgagcca ccgccggcag acgggcccgc cggcgagcgc 3420
tgacaaccca ctggacttga gcggcgtgga tgtgcgtctc cccatgctgg tgtacgtgtc 3480
ccgtgagaag cgcccccggc acgaccacca gaagaaggcc ggtgccatga acgcgcttac 3540
ccgcgcctcg gcgctgctct ccaactcccc cttcatcctc aacctcgact gcgatcatta 3600
catcaacaac tcccaggccc ttcgcgccgg catctgcttc atggtgggac gggacagcga 3660
cacggttgcc ttcgtccagt tcccgcagcg cttcgagggc gtcgacccca ccgacctcta 3720
cgccaaccac aaccgcatct tcttcgacgg caccctccgt gccctggacg gcatgcaggg 3780
ccccatctac gtcggcactg ggtgtctctt ccgccgcatc accgtctacg gcttcgaccc 3840
gccgaggatc aacgtcggcg gtccctgctt ccccaggctc gccgggctct tcgccaagac 3900
caagtacgag aagcccgggc tcgagatgac cacggccaag gccaaggccg cgcccgtgcc 3960

cgccaagggt aagcacggct tcttgccact gcccaagaag acgtacggca agtcggacgc 4020

cttcgtggac accatcccgc gcgcgtcgca cccgtcgccc tacgccgcgg cggctgaggg 4080

gatcgtggcc gacgaggcga ccatcgtcga ggcggtgaac gtgacggccg ccgcgttcga 4140

gaagaagacc ggctggggca aagagatcgg ctgggtgtac gacaccgtca cggaggacgt 4200

ggtcaccggc taccggatgc atatcaaggg gtggcggtca cgctactgct ccatctaccc 4260

acacgccttc atcggcaccg cccccatcaa cctcacggag aggctcttcc aggtgctccg 4320

ctggtccacg ggatccctcg agatcttctt ctccaagaac aacccgctct tcggcagcac 4380

atacctccac ccgctgcagc gcgtcgccta catcaacatc accacttacc ccttcaccgc 4440

catcttcctc atcttctaca ccaccgtgcc ggcgctatcc ttcgtcaccg gccacttcat 4500

cgtgcagcgc ccgaccacca tgttctacgt ctacctgggc atcgtgctat ccacgctgct 4560

cgtcatcgcc gtgctggagg tcaagtgggc cggggtcaca gtcttcgagt ggttcaggaa 4620

cggccagttc tggatgacag caagttgctc cgcctacctc gccgccgtct gccaggtgct 4680

gaccaaggtg atattccggc gggacatctc cttcaagctc acatccaagc taccctcggg 4740

agacgagaag aaggacccct acgccgacct ctacgtggtg cgctggacgc cgctcatgat 4800

tacacccatc atcatcatct tcgtcaacat catcggatcc gccgtggcct tcgccaaggt 4860

tctcgacggc gagtggacgc actggctcaa ggtcgccggc ggcgtcttct tcaacttctg 4920

ggtgctcttc cacctctacc ccttcgccaa gggcatcctg ggaagcacg gaaagacgcc 4980

agtcgtggtg ctcgtctggt gggcattcac cttcgtcatc accgccgtgc tctacatcaa 5040

catcccccac atgcatacct cgggaggcaa gcacacaacg gtgcatggtc accatggcaa 5100

gaagttggtc gacacagggc tctatggctg gctccattga 5140

<210> 27
<211> 2844
<212> DNA
<213> Hordeum vulgare

<220>
<221> misc_feature
<223> CDC-Bold HvCslF6 gene cDNA

<400> 27
atggcgccag cggtggccgg agggggccgc gtgcggagca atgagccggt tgctgctgct 60

gccgccgcgc cggcggccag cggcaagccc tgcgtgtgcg gcttccaggt ttgcgcctgc 120

acggggtcgg ccgcggtggc ctccgccgcc tcgtcgctgg acatggacat cgtggccatg 180

gggcagatcg gcgccgtcaa cgacgagagc tgggtgggcg tggagctcgg cgaagatggc 240

gagaccgacg aaagcggtgc cgccgttgac gaccgccccg tattccgcac cgagaagatc 300

aagggtgtcc tcctccaccc ctaccgggtg ctgattttcg ttcgtctgat cgccttcacg 360

ctgttcgtga tctggcgtat ctcccacaag aacccagacg cgatgtggct gtgggtgaca 420

tccatctgcg gcgagttctg gttcggtttc tcgtggctgc tggatcagct gcccaagctg 480
aaccccatca accgcgtgcc ggacctggcg gtgctgcggc agcgcttcga ccgccccgac 540
ggcacctcca cgctcccggg gctggacatc ttcgtcacca cggccgaccc catcaaggag 600
cccatcctct ccaccgccaa ctcggtgctc tccatcctgg ccgccgacta ccccgtggac 660
cgcaacacat gctacgtctc cgacgacagt ggcatgctgc tcacctacga ggccctggca 720
gagtcctcca agttcgccac gctctgggtg cccttctgcc gcaagcacgg gatcgagccc 780
aggggtccgg agagctactt cgagctcaag tcacaccctt acatggggag agcccaggac 840
gagttcgtca acgaccgccg ccgcgttcgc aaggagtacg acgagttcaa ggccaggatc 900
aacagcctgg agcatgacat caagcagcgc aacgacgggt acaacgccgc cattgcccac 960
agccaaggcg tgccccggcc cacctggatg gcggacggca cccagtggga gggcacatgg 1020
gtcgacgcct ccgagaacca ccgcaggggc gaccacgccg gcatcgtact ggtgctgctg 1080
aaccacccga gccaccgccg gcagacgggc ccgccggcga gcgctgacaa ccccactggac 1140
ttgagcggcg tggatgtgcg tctccccatg ctggtgtacg tgtcccgtga gaagcgcccc 1200
gggcacgacc accagaagaa ggccggtgcc atgaacgcgc ttacccgcgc ctcggcgctg 1260
ctctccaact ccccettcat cctcaacctc gactgcgatc attacatcaa caactcccag 1320
gcccttcgcg ccggcatctg cttcatggtg ggacgggaca gcgacacggt tgccttcgtc 1380
cagttcccgc agcgcttcga gggcgtcgac ccccaccgacc tctacgccaa ccacaaccgc 1440
atcttcttcg acggcaccct ccgtgccctg gacggcatgc agggccccat ctacgtcggc 1500
actgggtgtc tcttccgccg catcaccgtc tacggcttcg acccgccgag gatcaacgtc 1560
ggcggtccct gcttccccag gctcgccggg ctcttcgcca agaccaagta cgagaagccc 1620
gggctcgaga tgaccacggc caaggccaag gccgcgcccg tgcccgccaa gggtaagcac 1680
ggcttcttgc cactgcccaa gaagacgtac ggcaagtcgg acgccttcgt ggacaccatc 1740
ccgcgcgcgt cgcacccgtc gccctacgcc gcggcggctg aggggatcgt ggccgacgag 1800
gcgaccatcg tcgaggcggt gaacgtgacg gccgccgcgt tcgagaagaa gaccggctgg 1860
ggcaaagaga tcggctgggt gtacgacacc gtcacggagg acgtggtcac cggctaccgg 1920
atgcatatca aggggtggcg gtcacgctac tgctccatct acccacacgc cttcatcggc 1980
accgccccca tcaacctcac ggagaggctc ttccaggtgc tccgctggtc cacgggatcc 2040
ctcgagatct tcttctccaa gaacaacccg ctcttcggca gcacatacct ccacccgctg 2100
cagcgcgtcg cctacatcaa catcaccact taccccttca ccgccatctt cctcatcttc 2160
tacaccaccg tgccggcgct atccttcgtc accggccact tcatcgtgca gcgcccgacc 2220
accatgttct acgtctacct gggcatcgtg ctatccacgc tgctcgtcat cgccgtgctg 2280
gaggtcaagt gggccggggt cacagtcttc gagtggttca ggaacggcca gttctggatg 2340

```
acagcaagtt gctccgccta cctcgccgcc gtctgccagg tgctgaccaa ggtgatattc     2400

cggcgggaca tctccttcaa gctcacatcc aagctaccct cgggagacga gaagaaggac     2460

ccctacgccg acctctacgt ggtgcgctgg acgccgctca tgattacacc catcatcatc     2520

atcttcgtca acatcatcgg atccgccgtg gccttcgcca aggttctcga cggcgagtgg     2580

acgcactggc tcaaggtcgc cggcggcgtc ttcttcaact tctgggtgct cttccacctc     2640

tacccettcg ccaagggcat cctggggaag cacggaaaga cgccagtcgt ggtgctcgtc     2700

tggtgggcat tcaccttcgt catcaccgcc gtgctctaca tcaacatccc ccacatgcat     2760

acctcgggag gcaagcacac aacggtgcat ggtcaccatg gcaagaagtt ggtcgacaca     2820

gggctctatg gctggctcca ttga                                            2844
```

<210> 28
<211> 947
<212> PRT
<213> Hordeum vulgare

<220>
<221> misc_feature
<223> CDC-Bold HvCslF6 aa

<400> 28

```
Met Ala Pro Ala Val Ala Gly Gly Gly Arg Val Arg Ser Asn Glu Pro
1               5                   10                  15

Val Ala Ala Ala Ala Ala Ala Pro Ala Ala Ser Gly Lys Pro Cys Val
            20                  25                  30

Cys Gly Phe Gln Val Cys Ala Cys Thr Gly Ser Ala Ala Val Ala Ser
        35                  40                  45

Ala Ala Ser Ser Leu Asp Met Asp Ile Val Ala Met Gly Gln Ile Gly
    50                  55                  60

Ala Val Asn Asp Glu Ser Trp Val Gly Val Glu Leu Gly Glu Asp Gly
65                  70                  75                  80

Glu Thr Asp Glu Ser Gly Ala Ala Val Asp Asp Arg Pro Val Phe Arg
                85                  90                  95

Thr Glu Lys Ile Lys Gly Val Leu Leu His Pro Tyr Arg Val Leu Ile
            100                 105                 110

Phe Val Arg Leu Ile Ala Phe Thr Leu Phe Val Ile Trp Arg Ile Ser
        115                 120                 125

His Lys Asn Pro Asp Ala Met Trp Leu Trp Val Thr Ser Ile Cys Gly
    130                 135                 140
```

```
Glu Phe Trp Phe Gly Phe Ser Trp Leu Leu Asp Gln Leu Pro Lys Leu
145                 150                 155                 160

Asn Pro Ile Asn Arg Val Pro Asp Leu Ala Val Leu Arg Gln Arg Phe
                165                 170                 175

Asp Arg Pro Asp Gly Thr Ser Thr Leu Pro Gly Leu Asp Ile Phe Val
            180                 185                 190

Thr Thr Ala Asp Pro Ile Lys Glu Pro Ile Leu Ser Thr Ala Asn Ser
        195                 200                 205

Val Leu Ser Ile Leu Ala Ala Asp Tyr Pro Val Asp Arg Asn Thr Cys
    210                 215                 220

Tyr Val Ser Asp Asp Ser Gly Met Leu Leu Thr Tyr Glu Ala Leu Ala
225                 230                 235                 240

Glu Ser Ser Lys Phe Ala Thr Leu Trp Val Pro Phe Cys Arg Lys His
                245                 250                 255

Gly Ile Glu Pro Arg Gly Pro Glu Ser Tyr Phe Glu Leu Lys Ser His
            260                 265                 270

Pro Tyr Met Gly Arg Ala Gln Asp Glu Phe Val Asn Asp Arg Arg Arg
        275                 280                 285

Val Arg Lys Glu Tyr Asp Glu Phe Lys Ala Arg Ile Asn Ser Leu Glu
    290                 295                 300

His Asp Ile Lys Gln Arg Asn Asp Gly Tyr Asn Ala Ala Ile Ala His
305                 310                 315                 320

Ser Gln Gly Val Pro Arg Pro Thr Trp Met Ala Asp Gly Thr Gln Trp
                325                 330                 335

Glu Gly Thr Trp Val Asp Ala Ser Glu Asn His Arg Arg Gly Asp His
            340                 345                 350

Ala Gly Ile Val Leu Val Leu Leu Asn His Pro Ser His Arg Arg Gln
        355                 360                 365

Thr Gly Pro Pro Ala Ser Ala Asp Asn Pro Leu Asp Leu Ser Gly Val
    370                 375                 380

Asp Val Arg Leu Pro Met Leu Val Tyr Val Ser Arg Glu Lys Arg Pro
385                 390                 395                 400
```

Gly His Asp His Gln Lys Lys Ala Gly Ala Met Asn Ala Leu Thr Arg
405 410 415

Ala Ser Ala Leu Leu Ser Asn Ser Pro Phe Ile Leu Asn Leu Asp Cys
420 425 430

Asp His Tyr Ile Asn Asn Ser Gln Ala Leu Arg Ala Gly Ile Cys Phe
435 440 445

Met Val Gly Arg Asp Ser Asp Thr Val Ala Phe Val Gln Phe Pro Gln
450 455 460

Arg Phe Glu Gly Val Asp Pro Thr Asp Leu Tyr Ala Asn His Asn Arg
465 470 475 480

Ile Phe Phe Asp Gly Thr Leu Arg Ala Leu Asp Gly Met Gln Gly Pro
485 490 495

Ile Tyr Val Gly Thr Gly Cys Leu Phe Arg Arg Ile Thr Val Tyr Gly
500 505 510

Phe Asp Pro Pro Arg Ile Asn Val Gly Gly Pro Cys Phe Pro Arg Leu
515 520 525

Ala Gly Leu Phe Ala Lys Thr Lys Tyr Glu Lys Pro Gly Leu Glu Met
530 535 540

Thr Thr Ala Lys Ala Lys Ala Ala Pro Val Pro Ala Lys Gly Lys His
545 550 555 560

Gly Phe Leu Pro Leu Pro Lys Lys Thr Tyr Gly Lys Ser Asp Ala Phe
565 570 575

Val Asp Thr Ile Pro Arg Ala Ser His Pro Ser Pro Tyr Ala Ala Ala
580 585 590

Ala Glu Gly Ile Val Ala Asp Glu Ala Thr Ile Val Glu Ala Val Asn
595 600 605

Val Thr Ala Ala Ala Phe Glu Lys Lys Thr Gly Trp Gly Lys Glu Ile
610 615 620

Gly Trp Val Tyr Asp Thr Val Thr Glu Asp Val Val Thr Gly Tyr Arg
625 630 635 640

Met His Ile Lys Gly Trp Arg Ser Arg Tyr Cys Ser Ile Tyr Pro His
645 650 655

```
Ala Phe Ile Gly Thr Ala Pro Ile Asn Leu Thr Glu Arg Leu Phe Gln
            660                 665                 670

Val Leu Arg Trp Ser Thr Gly Ser Leu Glu Ile Phe Phe Ser Lys Asn
        675                 680                 685

Asn Pro Leu Phe Gly Ser Thr Tyr Leu His Pro Leu Gln Arg Val Ala
    690                 695                 700

Tyr Ile Asn Ile Thr Thr Tyr Pro Phe Thr Ala Ile Phe Leu Ile Phe
705                 710                 715                 720

Tyr Thr Thr Val Pro Ala Leu Ser Phe Val Thr Gly His Phe Ile Val
                725                 730                 735

Gln Arg Pro Thr Thr Met Phe Tyr Val Tyr Leu Gly Ile Val Leu Ser
            740                 745                 750

Thr Leu Leu Val Ile Ala Val Leu Glu Val Lys Trp Ala Gly Val Thr
        755                 760                 765

Val Phe Glu Trp Phe Arg Asn Gly Gln Phe Trp Met Thr Ala Ser Cys
    770                 775                 780

Ser Ala Tyr Leu Ala Ala Val Cys Gln Val Leu Thr Lys Val Ile Phe
785                 790                 795                 800

Arg Arg Asp Ile Ser Phe Lys Leu Thr Ser Lys Leu Pro Ser Gly Asp
                805                 810                 815

Glu Lys Lys Asp Pro Tyr Ala Asp Leu Tyr Val Val Arg Trp Thr Pro
            820                 825                 830

Leu Met Ile Thr Pro Ile Ile Ile Ile Phe Val Asn Ile Ile Gly Ser
        835                 840                 845

Ala Val Ala Phe Ala Lys Val Leu Asp Gly Glu Trp Thr His Trp Leu
    850                 855                 860

Lys Val Ala Gly Gly Val Phe Phe Asn Phe Trp Val Leu Phe His Leu
865                 870                 875                 880

Tyr Pro Phe Ala Lys Gly Ile Leu Gly Lys His Gly Lys Thr Pro Val
                885                 890                 895

Val Val Leu Val Trp Trp Ala Phe Thr Phe Val Ile Thr Ala Val Leu
            900                 905                 910

Tyr Ile Asn Ile Pro His Met His Thr Ser Gly Gly Lys His Thr Thr
```

```
            915                 920                 925
Val His Gly His His Gly Lys Lys Leu Val Asp Thr Gly Leu Tyr Gly
    930                 935                 940

Trp Leu His
945
```